(19) Europäisches Patentamt
European Patent Office
Office européen des brevets

(11) Veröffentlichungsnummer: **0 364 797 B1**

(12) EUROPÄISCHE PATENTSCHRIFT

(45) Veröffentlichungstag der Patentschrift: **16.02.94**

(51) Int. Cl.[5]: **C07D 209/48**, C07D 471/02, C07D 487/02, C07D 207/452, A01N 43/36, A01N 43/38, A01N 43/90

(21) Anmeldenummer: **89118149.7**

(22) Anmeldetag: **30.09.89**

Die Akte enthält technische Angaben, die nach dem Eingang der Anmeldung eingereicht wurden und die nicht in dieser Patentschrift enthalten sind.

# (54) N-Aryl-Stickstoffheterocyclen, mehrere Verfahren zu ihrer Herstellung und ihre Verwendung als Herbizide.

(30) Priorität: **15.10.88 DE 3835168**

(43) Veröffentlichungstag der Anmeldung:
**25.04.90 Patentblatt 90/17**

(45) Bekanntmachung des Hinweises auf die Patenterteilung:
**16.02.94 Patentblatt 94/07**

(84) Benannte Vertragsstaaten:
**BE CH DE FR GB IT LI NL**

(56) Entgegenhaltungen:
**EP-A- 0 190 755**
**EP-A- 0 211 805**
**EP-A- 0 216 243**
**EP-A- 0 259 265**
**EP-A- 0 303 573**
**DE-A- 3 642 372**
**GB-A- 2 046 754**
**US-A- 3 878 224**

(73) Patentinhaber: **BAYER AG**
**D-51368 Leverkusen(DE)**

(72) Erfinder: **Fischer, Reiner, Dr.**
**Nelly-Sachs-Strasse 23**
**D-4019 Monheim(DE)**
Erfinder: **Jensen-Korte, Uta, Dr.**
**Geibelstrasse 9**
**D-4000 Düsseldorf 1(DE)**
Erfinder: **Kunisch, Franz, Dr.**
**Zum Hahnenberg 20**
**D-5068 Odenthal(DE)**
Erfinder: **Marhold, Albrecht, Dr.**
**Carl-Duisberg-Strasse 329**
**D-5090 Leverkusen 1(DE)**
Erfinder: **Ooms, Pieter, Dr.**
**Doerperhofstrasse 16**
**D-4150 Krefeld 1(DE)**
Erfinder: **Schallner, Otto, Dr.**
**Noldeweg 22**
**D-4019 Monheim(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Strang, Harry, Dr.**
**Heiderweg 53**
**D-4000 Düsseldorf 31(DE)**

**Beschreibung**

Die Erfindung betrifft neue N-Aryl-Stickstoffheterocyclen, mehrere Verfahren zu ihrer Herstellung und ihre Verwendung als Herbizide.

Es ist bereits bekannt, daß bestimmte N-Aryl-Stickstoffheterocyclen, wie beispielsweise das N-(2-Fluor-4-chlor-5-propargyloxy-phenyl)-$\Delta^1$-tetrahydrophthalimid herbizide Eigenschaften besitzen(vgl. EP 83 055 oder EP 61 741).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Weiterhin sind in der nicht vorveröffentlichten EP-A 303 573 Pyrrolidin-2,5-dione und 4,5,6,7-Tetrahydroisoindol-1,3-dione mit herbizider Wirkung beschrieben.

In der EP-A-211 805 werden N-(2-Fluorphenyl)-azolidine mit guten pre- und postemergenten selektiven, herbiziden Eigenschaften beschrieben, die jedoch in 4-Position des Phenylrings Halogen substituiert sind.

In der GB-20 46 754 werden N-substituierte Tetrahydrophthalimide und herbizid wirksame Zusammensetzungen mit diesen offenbart.

Die EP 216 243 beschreibt ebenfalls herbizid wirksame N-substituierte 3,4,5,6-Tetrahydrophthalimide, deren Phenylrest jedoch in 4-Position nicht CN-substituiert ist, und der in 5-Position eine Carbonylgruppe trägt.

Die US-Patentschrift US 3.878.224 offenbart ebenfalls N-substituierte 3,4,5,6-Tetrahydrophthalimide mit herbiziden Eigenschaften.

Schließlich sei noch auf die DE-OS 36 42 372 verwiesen die herbizid wirksame Bernsteinsäurederivate offenbart.

Es wurden neue N-Aryl-Stickstoffheterocyclen der allgemeinen Formel (I),

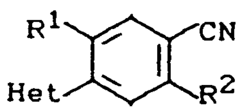

(I)

in welcher

Het. für einen Heterocyclus der Formel

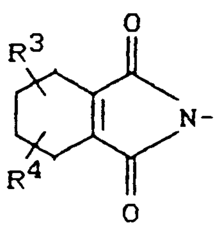 ; 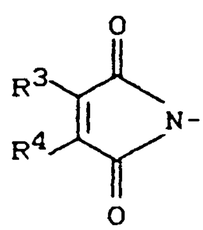 ; 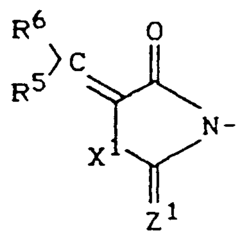 ;

oder 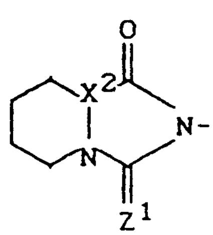

steht,

$R^1$ für Wasserstoff oder Halogen steht und

$R^2$ für Halogen, Hydroxy oder für einen Rest $-Z^2-R^8$ steht, wobei

$X^1$ für eine $-CH_2$-Gruppe, für eine

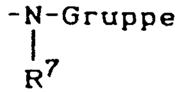

oder für eine

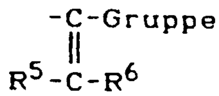

steht,

$X^2$ für Stickstoff oder eine CH-Gruppe steht,

$Z^1$ für Sauerstoff oder Schwefel steht,

$Z^2$ für Sauerstoff oder Schwefel steht,

$R^3$ und $R^4$ unabhängig voneinander jeweils für Wasserstoff oder Alkyl stehen,

$R^5$ und $R^6$ entweder unabhängig voneinander jeweils für Wasserstoff oder Alkyl stehen oder gemeinsam für einen zweifach verknüpften Alkandiylrest stehen,

$R^7$ für Wasserstoff, Alkyl oder für gegebenenfalls substituiertes Aryl steht und

$R^8$ für jeweils gegebenenfalls substituiertes Alkyl, Alkenyl, Alkinyl oder Cycloalkyl steht, ausgenommen die Verbindungen in denen Het für einen Heterocyclus der Formel

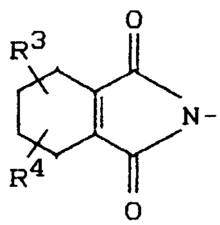

oder

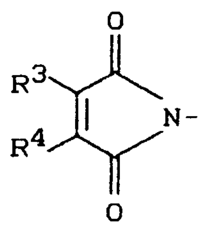

steht

$R^1$ für Wasserstoff oder Halogen steht,

$R^2$ für einen Rest $-Z^2-R^8$ steht, wobei

$Z^2$ für Sauerstoff steht und

$R^8$ für Wasserstoff oder für jeweils gegebenenfalls substituiertes Alkyl, Alkenyl, Alkinyl oder Cycloalkyl steht und

$R^3$ und $R^4$ jeweils für Alkyl stehen,

gefunden, wobei die folgenen Verbindungen vom Disclaimer ausgenommen sind:

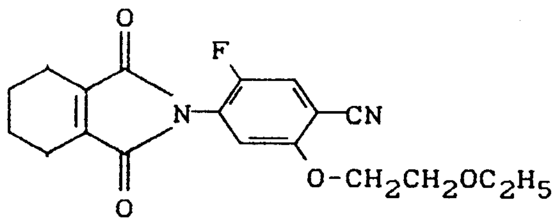

,

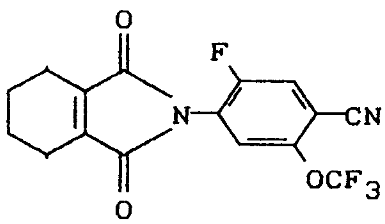

,

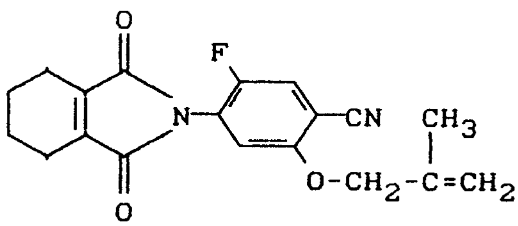

,

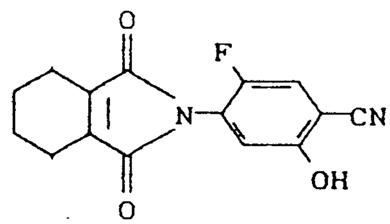

,

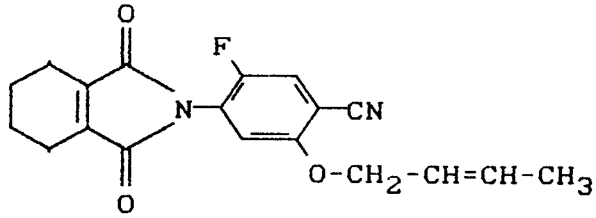,

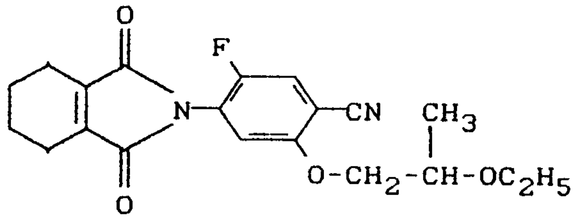,

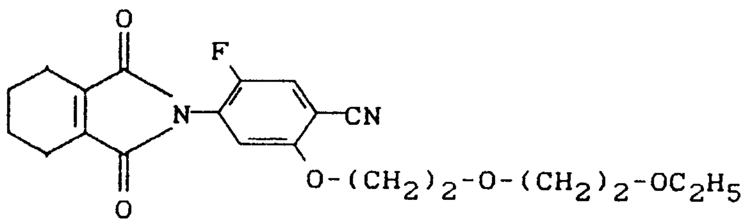

und

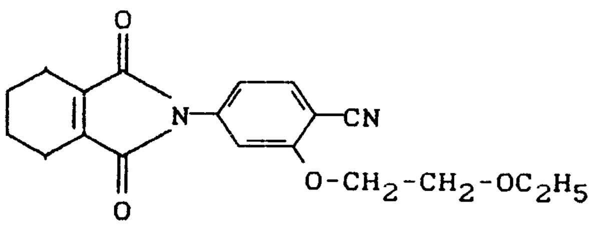.

Weiterhin wurde gefunden, daß man die neuen N-Aryl-Stickstoffheterocyclen der allgemeinen Formel (I),

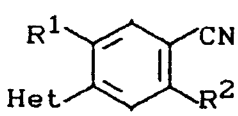

(I)

in welcher

Het für einen Heterocyclus der Formel

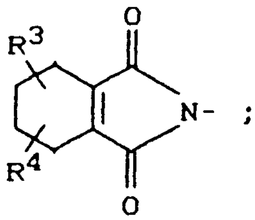 ; 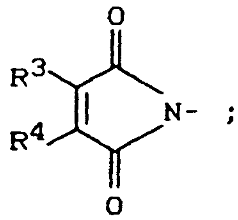 ; 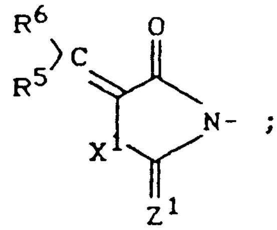 ;

oder 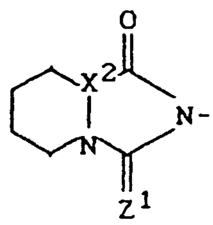

steht,

R¹ für Wasserstoff oder Halogen steht und

R² für Halogen, Hydroxy oder für einen Rest -Z²-R⁸ steht, wobei

X¹ für eine -CH₂-Gruppe, für eine

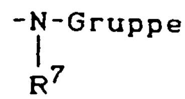

oder für eine

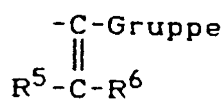

steht,

X² für Stickstoff oder eine CH-Gruppe steht,

Z¹ für Sauerstoff oder Schwefel steht,

Z² für Sauerstoff oder Schwefel steht,

R³ und R⁴ unabhängig voneinander jeweils für Wasserstoff oder Alkyl stehen,

R⁵ und R⁶ entweder unabhängig voneinander jeweils für Wasserstoff oder Alkyl stehen oder gemeinsam für einen zweifach verknüpften Alkandiylrest stehen,

R⁷ für Wasserstoff, Alkyl oder für gegebenenfalls substituiertes Aryl steht und

R⁸ für jeweils gegebenenfalls substituiertes Alkyl, Alkenyl, Alkinyl oder Cycloalkyl steht, ausgenommen die Verbindungen in denen Het für einen Heterocyclus der Formel

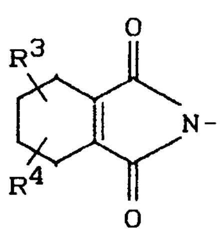 oder 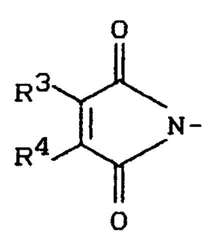

steht

$R^1$ für Wasserstoff oder Halogen steht,

$R^2$ für einen Rest $-Z^2-R^8$ steht, wobei

$Z^2$ für Sauerstoff steht und

$R^8$ für Wasserstoff oder für jeweils gegebenenfalls substituiertes Alkyl, Alkenyl, Alkinyl oder Cycloalkyl steht und

$R^3$ und $R^4$ jeweils für Alkyl stehen, wobei die folgenden Verbindungen vom Disclaimer ausgenommen sind:

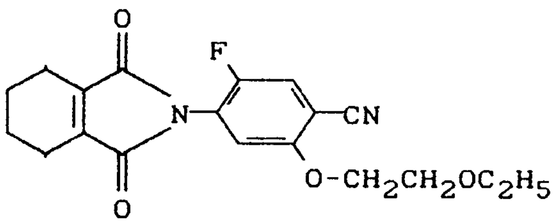

,

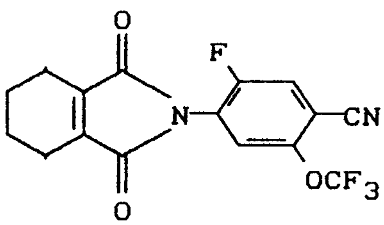

,

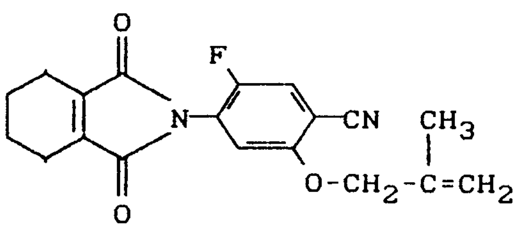

,

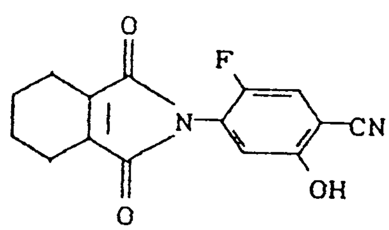

,

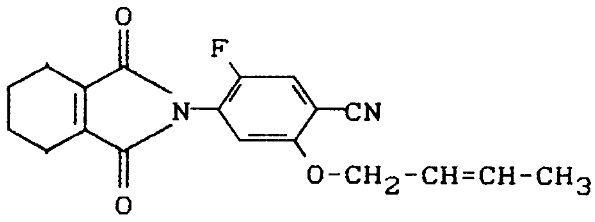
,

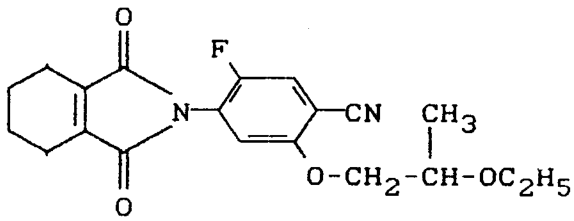
,

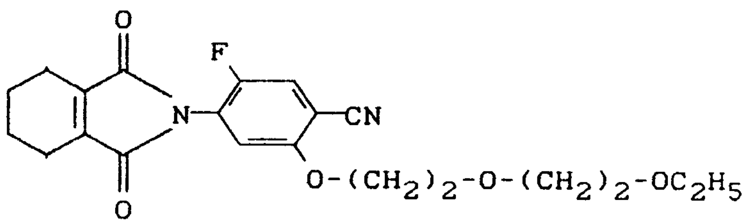

und

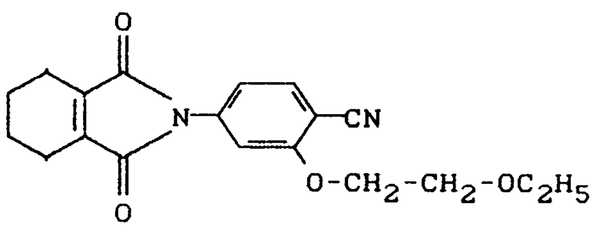
,

nach einem der im folgenden beschriebenen Verfahren erhält:

(a) Man erhält N-Aryl-Stickstoffheterocyclen der Formel (Ia),

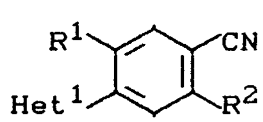
(Ia)

in welcher

Het[1] für einen Heterocyclus der Formel

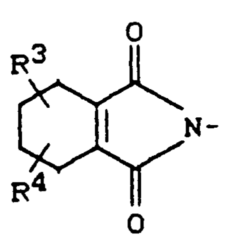
;
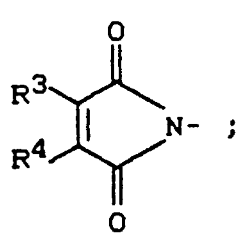
;
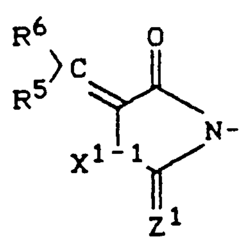
;

steht, wobei

$X^{1-1}$ für eine $-CH_2$-Gruppe oder für eine

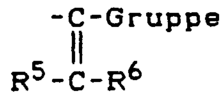

steht und

$R^1$, $R^2$, $R^3$, $R^4$, $R^5$ und $R^6$ die oben angegebene Bedeutung haben,

wenn man Anhydride der Formel (II),

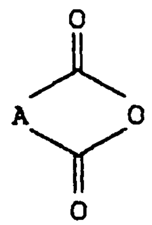 (II)

in welcher

A für einen Rest der Formel

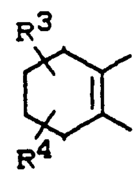 ; 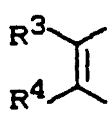 oder 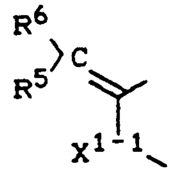

steht,

wobei

$X^{1-1}$, $R^3$, $R^4$, $R^5$ und $R^6$ die oben angegebene Bedeutung haben,

mit Aminen der Formel (III),

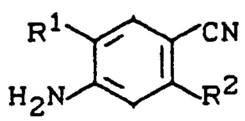 (III)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt;

(b) man erhält N-Aryl-Stickstoffheterocyclen der Formel (Ib),

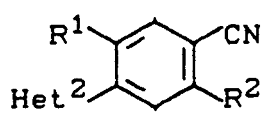 (Ib)

in welcher

$Het^2$ für einen Heterocyclus der Formel

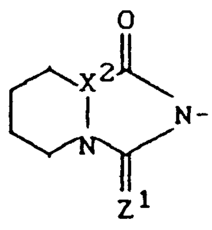

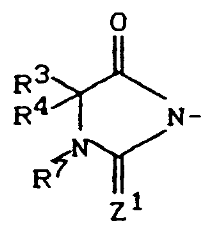

oder

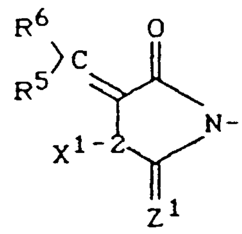

steht, wobei

$X^{1-2}$ für eine

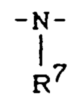 Gruppe

steht und

$R^1$, $R^2$, $R^3$, $R^4$, $R^5$, $R^6$, $R^7$, $X^2$ und $Z^1$ die oben angegebene Bedeutung haben,

wenn man Carbonsäureester der Formel (IV),

$R^9$-$COOR^{10}$ (IV)

in welcher

$R^9$ für einen Rest der Formel

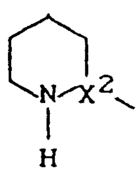

;

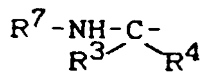

oder

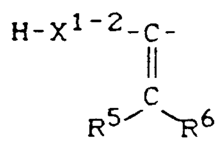

steht,

und

$R^{10}$ für Alkyl steht, wobei

$X^{1-2}$, $X^2$, $R^3$, $R^4$, $R^5$, $R^6$ und $R^7$ die oben angegebene Bedeutung haben,

oder deren Säureadditionssalze mit Iso(thio)cyanaten der Formel (V),

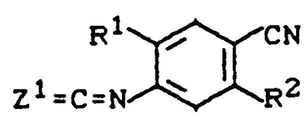

(V)

in welcher

$R^1$, $R^2$ und $Z^1$ die oben angegebene Bedeutung haben,

gegebenenfalls in Gegenwart eines Verdünnungsmittels und gegebenenfalls in Gegenwart eines Reaktionshilfsmittels umsetzt;

(c) man erhält N-Aryl-Stickstoffheterocyclen der Formel (Ic),

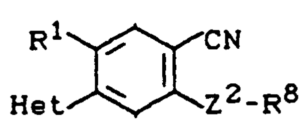

(Ic)

in welcher
$R^1$, $R^8$, $Z^2$ und Het die oben angegebene Bedeutung haben,
wenn man die nach Verfahren (a) und (b) erhältlichen N-Arylstickstoffheterocyclen der Formel (If),

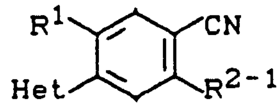

(If)

in welcher
$R^{2-1}$ für Halogen steht und
Het und $R^1$ die oben angegebene Bedeutung haben,
mit Alkoholen oder Thiolen der Formel (VI),

$R^8$-$Z^2$H (VI)

in welcher
$R^8$ und $Z^2$ die oben angegebene Bedeutung haben,
gegebenenfalls in Gegenwart eines Verdünnungsmittels sowie in Gegenwart eines Reaktionshilfsmittels umsetzt;
(d) man erhält N-Aryl-Stickstoffheterocyclen der Formel (Id),

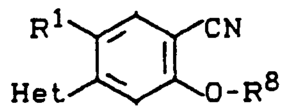

(Id)

in welcher
$R^1$, $R^8$ und Het die oben angegebene Bedeutung haben,
wenn man N-Aryl-Stickstoffheterocyclen der Formel (Ie),

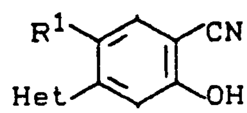

(Ie)

in welcher
$R^1$ und Het die oben angegebene Bedeutung haben,
mit Alkylierungsmitteln der Formel (VII),

$R^8$-E (VII)

in welcher
E für eine elektronenanziehende Abgangsgruppe steht und
$R^8$ die oben angegebene Bedeutung hat,
gegebenenfalls in Gegenwart eines Verdünnungsmittels und gegebenenfalls in Gegenwart eines Reaktionshilfsmittels umsetzt;
e) man erhält N-Aryl-Stickstoffheterocyclen der Formel (Ie)

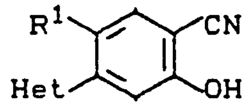

(Ie)

in welcher

$R^1$ und Het die oben angegebene Bedeutung haben,

wenn man

e-α) N-Aryl-Stickstoffheterocyclen der Formel (Ig)

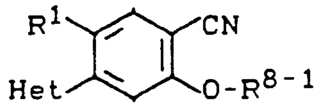

(Ig)

in welcher

$R^{8-1}$ für Allyl oder Benzyl steht und

$R^1$ und Het die oben angegebene Bedeutung haben,

im Fall, daß $R^{8-1}$ für Benzyl steht, mit Reduktionsmitteln gegebenenfalls in Gegenwart eines Katalysators und gegebenenfalls in Gegenwart eines Verdünnungsmittels und gegebenenfalls in Gegenwart eines Reaktionshilfsmittels umsetzt, und im Fall, daß $R^{8-1}$ für Allyl steht, mit Edelmetallkatalysatoren mit anschließender saurer Abspaltung gegebenenfalls in Gegenwart eines Verdünnungsmittels umsetzt, oder wenn man

e-β) Amine der Formel (IIIa)

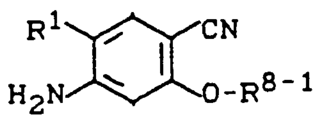

(IIIa)

in welcher

$R^1$ und $R^{8-1}$ die oben angegebene Bedeutung haben,

im Fall, daß $R^{8-1}$ für Benzyl steht, mit Reduktionsmitteln gegebenenfalls in Gegenwart eines Katalysators und gegebenenfalls in Gegenwart eines Verdünnungsmittels und gegebenenfalls in Gegenwart eines Reaktionsmittels umsetzt und im Fall, daß $R^{8-1}$ für Allyl steht, mit Edelmetallkatalysatoren mit anschließender saurer Abspaltung gegebenenfalls in Gegenwart eines Verdünnungsmittels umsetzt, und die so erhältlichen Aminophenole der Formel (VIII)

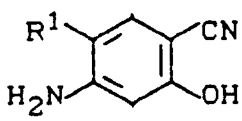

(VIII)

in welcher

$R^1$ die oben angegebene Bedeutung hat,

mit Anhydriden der Formel (II),

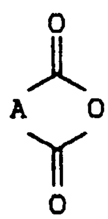

(II)

in welcher

A für einen Rest der Formel

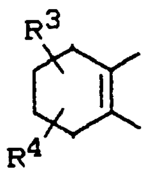 ; 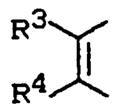 oder 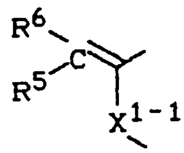

steht, wobei

$X^{1-1}$ für eine $-CH_2$-Gruppe oder für eine

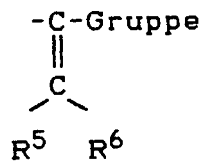

steht und

$R^3$, $R^4$, $R^5$ und $R^6$ die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen N-Aryl-Stickstoffheterocyclen der allgemeinen Formel (I) herbizide Eigenschaften besitzen. Die Verbindungen der Formel (I), in denen $R^2$ für Hydroxy steht, sind darüberhinaus auch als wertvolle Zwischenprodukte für erfindungsgemäße N-Aryl-Stickstoffheterocyclen der Formel (I) geeignet.

Überraschenderweise zeigen die erfindungsgemäßen N-Aryl-Stickstoffheterocyclender allgemeinen Formel (I) bei einer vergleichbaren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Technik bekannten N-Aryl-Stickstoffheterocyclen, wie beispielsweise das N-(2-Fluor-4-chlor-5-propargyloxy-phenyl)-$\Delta^1$-tetrahydrophthalimid, welche chemisch und wirkungsmäßig naheliegende Verbindungen sind.

Die erfindungsgemäßen N-Aryl-Stickstoffheterocyclen sind durch die Formel (I) allgemein definiert.

Bevorzugt sind Verbindungen der Formel (I), bei welchen

Het für einen Heterocyclus der Formel

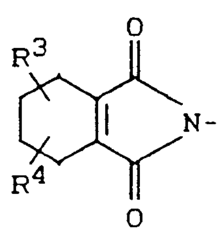 ; 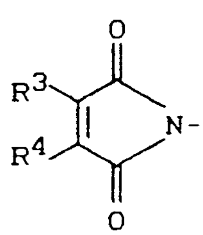 ; 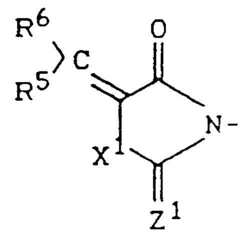 ;

oder 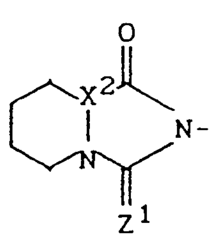

steht,

$R^1$ für Wasserstoff, Fluor, Chlor oder Brom steht und

$R^2$ für Fluor, Chlor, Brom, Hydroxy oder für einen Rest $-Z^2-R^8$ steht, wobei

$X^1$ für eine $-CH_2$-Gruppe, für eine

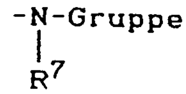

oder für eine

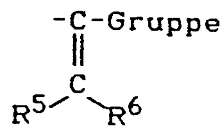

steht,

$X^2$ für Stickstoff oder eine CH-Gruppe steht,

$Z^1$ für Sauerstoff oder Schwefel steht,

$Z^2$ für Sauerstoff oder Schwefel steht,

$R^3$ und $R^4$ unabhängig voneinander jeweils für Wasserstoff oder für geradkettiges oder verzweigtes Alkyl mit 1 bis 4 Kohlenstoffatomen stehen,

$R^5$ und $R^6$ entweder unabhängig voneinander jeweils für Wasserstoff oder für geradkettiges oder verzweigtes Alkyl mit 1 bis 4 Kohlenstoffatomen stehen oder gemeinsam für einen zweifachverknüpften Alkandiylrest mit 2 bis 7 Kohlenstoffatomen stehen,

$R^7$ für Wasserstoff, für geradkettiges oder verzweigtes Alkyl mit 1 bis 4 Kohlenstoffatomen oder für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

$R^8$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils infrage kommen: Halogen sowie jeweils geradkettiges oder verzweigtes Alkyl oder Alkoxy mit jeweils 1 bis 4 Kohlenstoffatomen, $R^8$ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen Alkylteilen steht und schließlich für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

ausgenommen die Verbindungen in denen Het für einen Heterocyclus der Formel

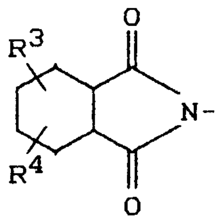

oder

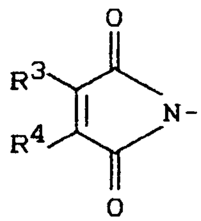

steht

- $R^1$ für Wasserstoff oder Halogen steht,
- $R^2$ für einen Reste $-Z^2-R^8$ steht, wobei
- $Z^2$ für Sauerstoff steht und
- $R^8$ für Wasserstoff oder jeweils gegebenenfalls substituiertes Alkyl, Alkenyl, Alkinyl oder Cycloalkyl steht und
- $R^3$ und $R^4$ jeweils für Alkyl stehen, wobei die folgenden Verbindungen vom Disclaimer ausgenommen sind:

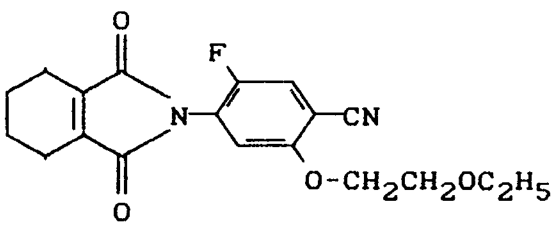

,

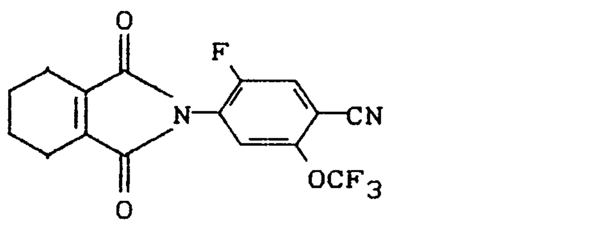
,

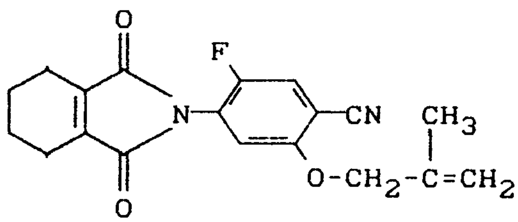
,

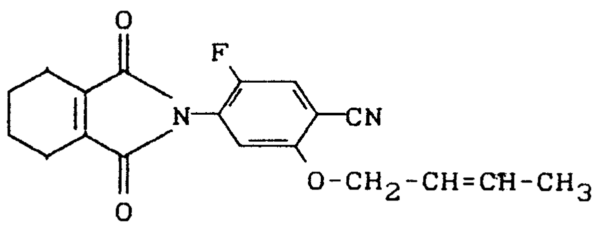
,

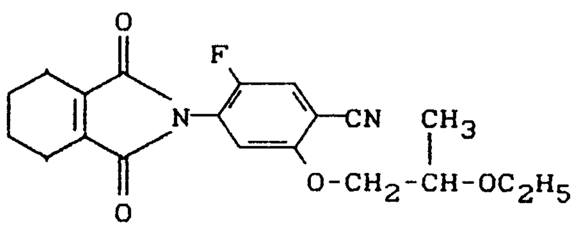
,

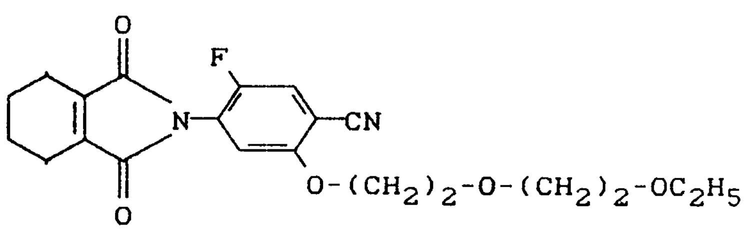

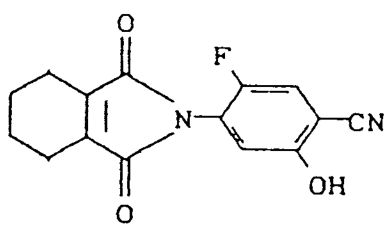

und 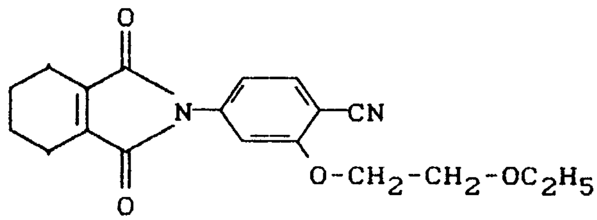 .

Besonders bevorzugt ist die Gruppe von Verbindungen der Formel (I), bei welchen

Het für einen Heterocyclus der Formel

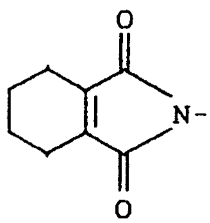 ; 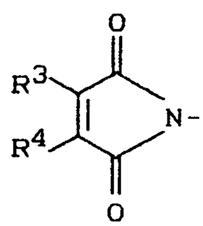 ; 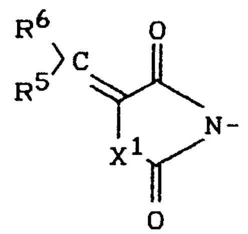 ;

oder 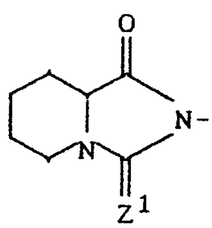

steht,

$R^1$ für Wasserstoff, Fluor oder Chlor steht und

$R^2$ für Fluor, Chlor oder für einen Rest $-Z^2-R^8$ steht,

$X^1$ für eine $-CH_2$-Gruppe, für eine

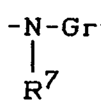 Gruppe

oder für eine

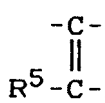 Gruppe

steht,

$Z^1$ für Sauerstoff oder Schwefel steht,

$Z^2$ für Sauerstoff oder Schwefel steht,

$R^3$ und $R^4$ unabhängig voneinander jeweils für Wasserstoff, Methyl, Ethyl, n- oder i-Propyl stehen,

$R^5$ und $R^6$ entweder unabhängig voneinander jeweils für Wasserstoff, Methyl, Ethyl, n- oder i-Propyl stehen oder gemeinsam für einen zweifach verknüpfen Alkandiylrest mit 2 bis 5 Kohlenstoffatomen stehen,

$R^7$ für Wasserstoff, Methyl, Ethyl, n- oder i-Propyl oder für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy

oder Trifluormethylthio und

$R^8$ für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich und verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

ausgenommen die Verbindungen in denen Het für einen Heterocyclus der Formel

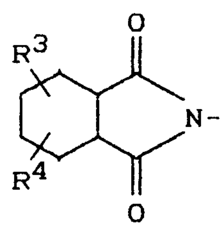

oder

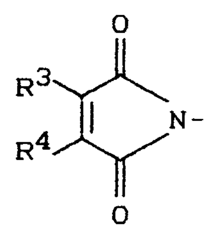

steht

$R^1$ für Wasserstoff oder Halogen steht,

$R^2$ für einen Reste $-Z^2-R^8$ steht, wobei

$Z^2$ für Sauerstoff steht und

$R^8$ für Wasserstoff oder für jeweils gegebenenfalls substituiertes Alkyl, Alkenyl, Alkinyl oder Cycloalkyl steht und

$R^3$ und $R^4$ jeweils für Alkyl stehen.

wobei die folgenden Verbindungen vom Disclaimer ausgenommen sind:

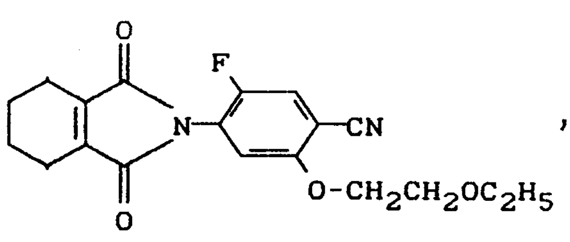

,

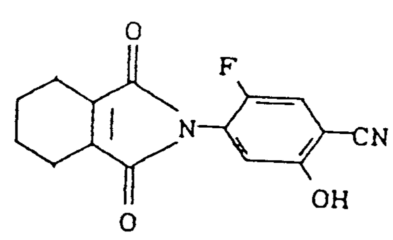

,

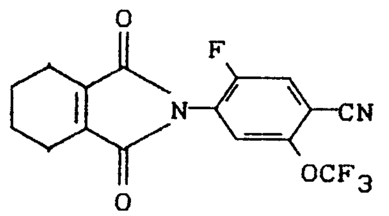

,

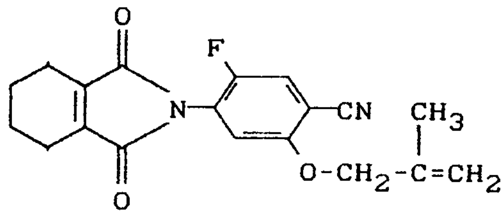

,

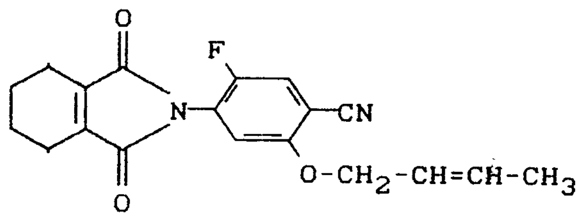

,

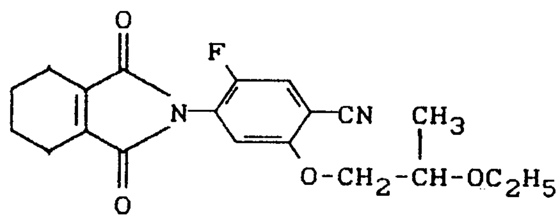

,

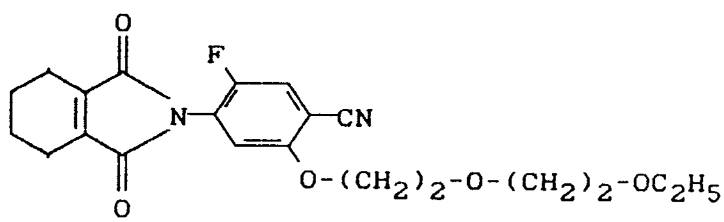

und

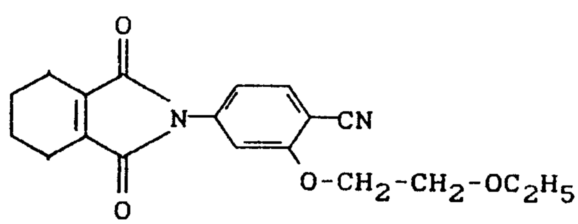

.

Besonders bevorzugt ist auch die Gruppe von Verbindungen der Formel (I), bei welchen $R^2$ für Hydroxy steht und die übrigen Reste die oben als besonders bevorzugt angegebene Bedeutung haben.

Ganz besonders bevorzugt ist die Gruppe von Verbindungen der Formel (I), bei welchen

Het für einen Heterocyclus der Formel

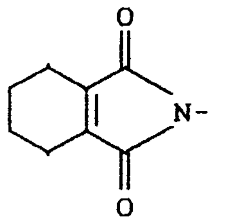 ; 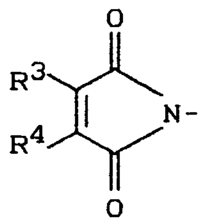 ; 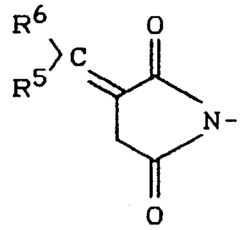 ;

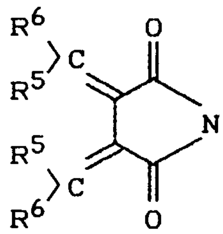 oder 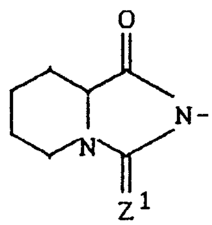

steht,

$R^1$ für Fluor steht und

$R^2$ für Fluor oder für einen Rest $-Z^2-R^8$ steht, wobei

$Z^2$ für Sauerstoff oder Schwefel steht,

$R^3$ und $R^4$ unabhängig voneinander jeweils für Wasserstoff oder Methyl stehen,

$R^5$ und $R^6$ entweder unabhängig voneinander jeweils für Wasserstoff oder Methyl oder gemeinsam für einen Ethan-1,2-diylrest, einen Butan-1,4-diylrest oder einen Pentan-1,5-diylrest stehen und

$R^8$ für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht oder für jeweils gegebenenfalls ein- bis dreifach gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

ausgenommen die Verbindungen in denen Het für einen Heterocyclus der Formel

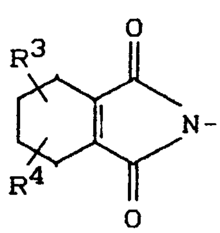 oder 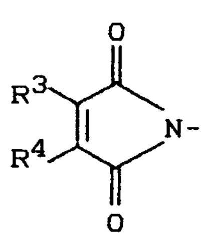

steht

$R^1$ für Wasserstoff oder Halogen steht,

| | |
|---|---|
| $R^2$ | für einen Rest $-Z^2-R^8$ steht, wobei |
| $Z^2$ | für Sauerstoff steht und |
| $R^8$ | für Wasserstoff oder für jeweils gegebenenfalls substituiertes Alkyl, Alkenyl, Alkinyl oder Cycloalkvl steht und |
| $R^3$ und $R^4$ | jeweils für Alkyl stehen, wobei die folgenden Verbindungen vom Disclaimer ausgenommen sind: |

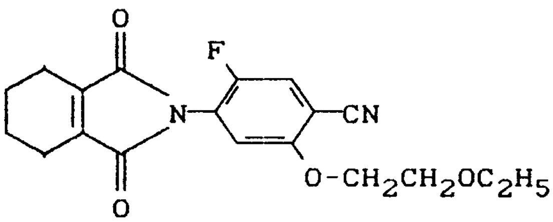

,

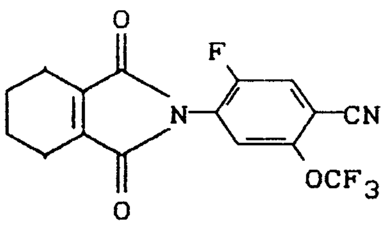

,

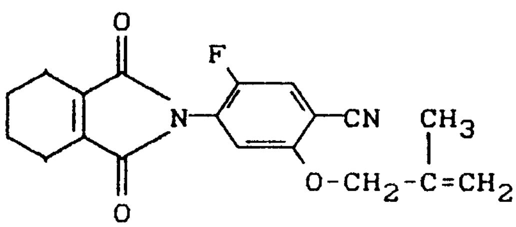

,

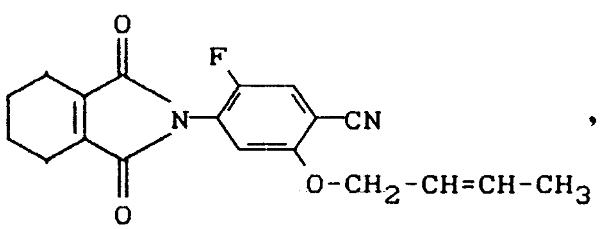

,

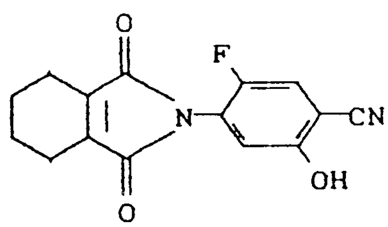

,

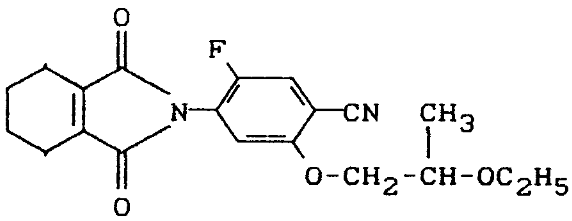

,

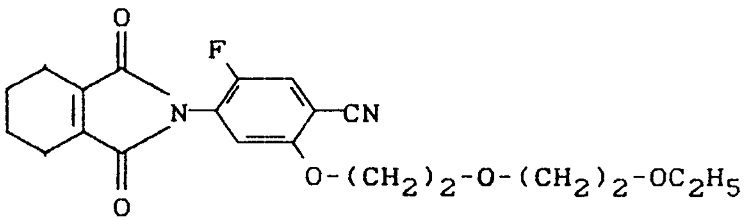

und

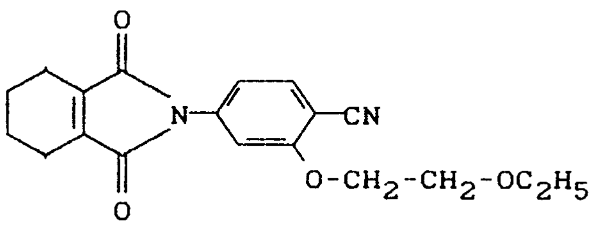

.

Im einzelnen seien außer den bei den Herstellungsbeispielen genannten Verbindungen die folgenden N-Aryl-Stickstoffheterocyclen der allgemeinen Formel (I) genannt:

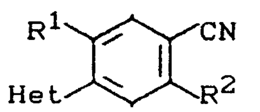

(I)

| Het | $R^1$ | $R^2$ |
| --- | --- | --- |
| 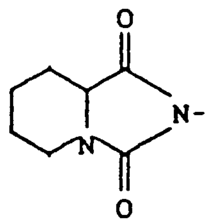 | F | $-O-CH_2-C\equiv CH$ |
| 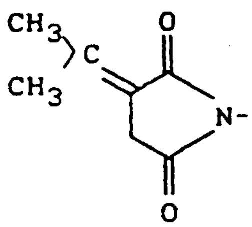 | F | $-O-CH_2-C\equiv CH$ |
| 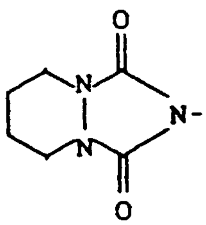 | F | $-O-CH_2-C\equiv CH$ |

| Het | $R^1$ | $R^2$ |
|---|---|---|
| 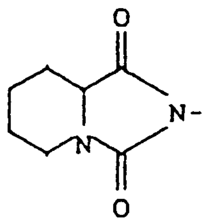 | H | $-O-CH_2-C{\equiv}CH$ |
| 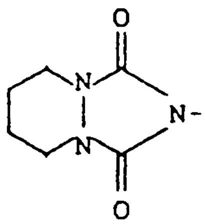 | H | $-O-CH_2-C{\equiv}CH$ |
| 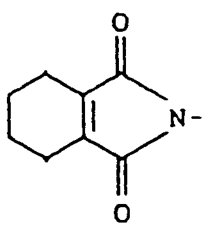 | F | $-S-CH_2-COOC_2H_5$ |

| Het | $R^1$ | $R^2$ |
|---|---|---|
| 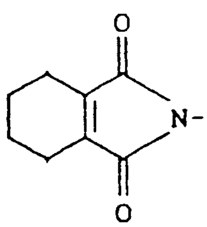 | F | $-S-CH(CH_3)_2$ |
| 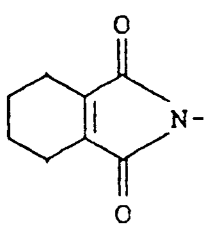 | F | $-O-CH_2-\overset{O}{\overset{\|}{C}}-CH_3$ |

| Het | $R^1$ | $R^2$ |
|---|---|---|
| 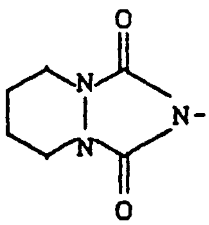 | F | 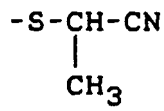 |
| 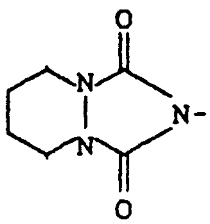 | F | 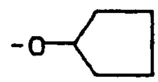 |
| 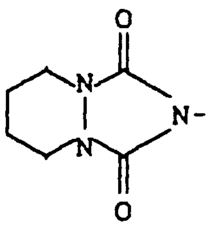 | F | 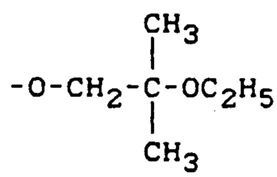 |
| 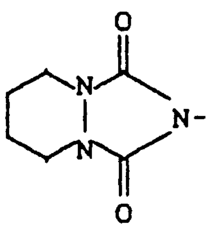 | F | 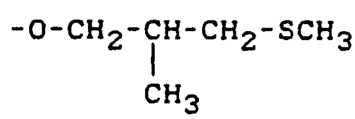 |
| 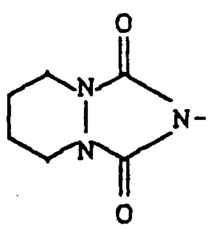 | F | 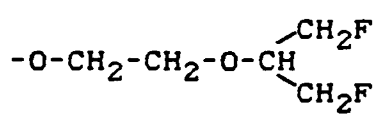 |

| Het | $R^1$ | $R^2$ |
|---|---|---|
| 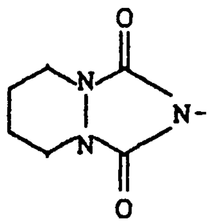 | F | 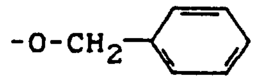 |
| 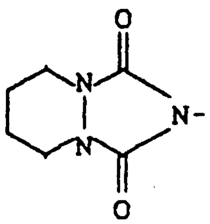 | F | 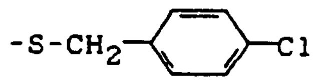 |
| 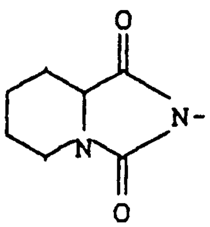 | F | 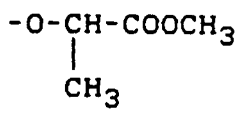 |
| 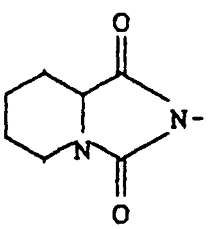 | F | 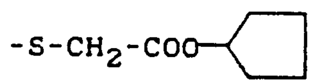 |
| 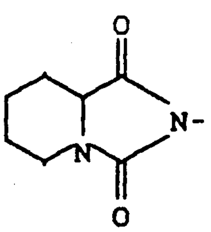 | F | $-S-CH_2-COO-(CH_2)_4-CH_3$ |

| Het | $R^1$ | $R^2$ |
|---|---|---|
| 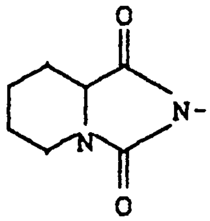 | F | $-O-CH_2-CN$ |
| 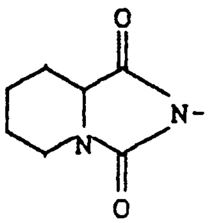 | F | $-O-CH-C-CH_3$<br>$CH_3$ $O$ |
| 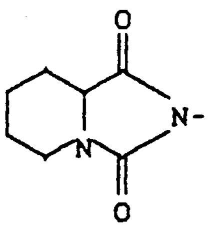 | F | $-S-CH_2-CH=CH_2$ |
| 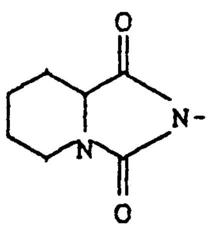 | F | $-S-CH_2-C\equiv CH$ |
| 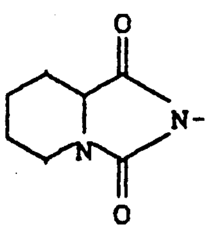 | F | $-O-CH-COOC_2H_5$<br>$C_2H_5$ |

| Het | $R^1$ | $R^2$ |
|---|---|---|
| 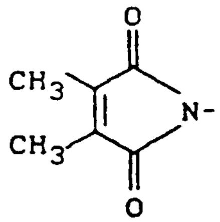 | F | $-S-CH_2-CH=CH_2$ |
| 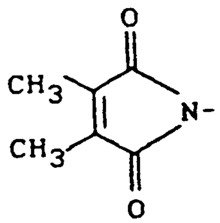 | F | $-S-CH_2-C\equiv CH$ |

| Het | $R^1$ | $R^2$ |
|---|---|---|
| 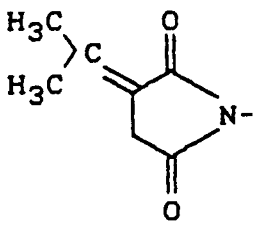 | F | 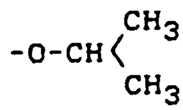 |
| 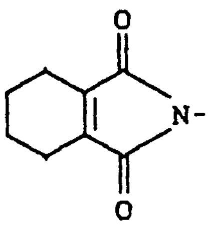 | F | 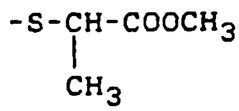 |
| 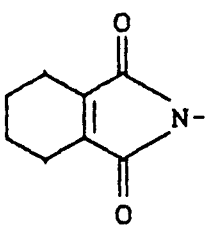 | F | 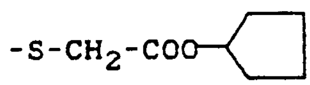 |
| 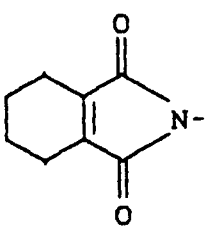 | F | $-S-CH_2-COO-(CH_2)_4-CH_3$ |
| 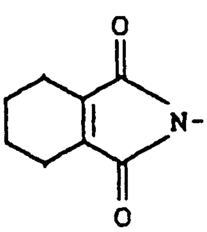 | F | 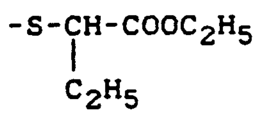 |

| Het | $R^1$ | $R^2$ |
|---|---|---|
| 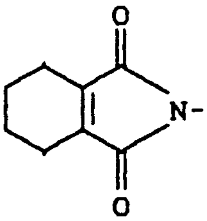 | H | $-S-CH-COOCH_3$<br>$CH_3$ |
| 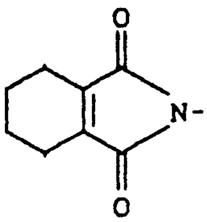 | H | $-S-CH_2-COO$ 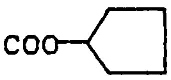 |
| 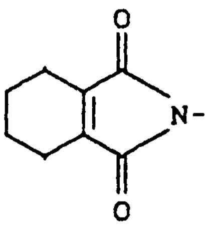 | H | $-S-CH_2-COO-(CH_2)_4-CH_3$ |
| 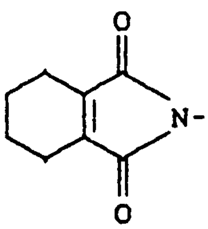 | H | $-S-CH-COOC_2H_5$<br>$C_2H_5$ |
| 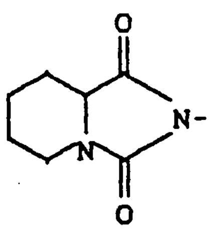 | H | $-S-CH-COOC_2H_5$<br>$CH_3$ |

| Het | $R^1$ | $R^2$ |
|---|---|---|
| 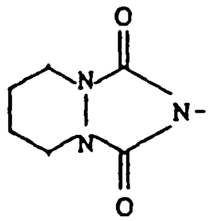 | H | $-S-CH_2-C \equiv CH$ |
| 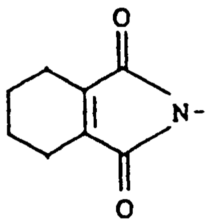 | H | $-S-CH_2-COOC_2H_5$ |
| 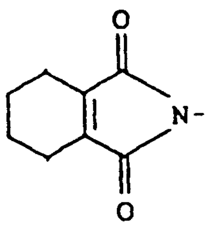 | H | $-S-CH(CH_3)_2$ |
| 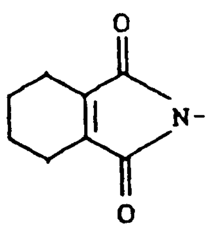 | H | $-O-CH_2-\underset{\underset{O}{\|}}{C}-CH_3$ |

| Het | $R^1$ | $R^2$ |
|---|---|---|
| 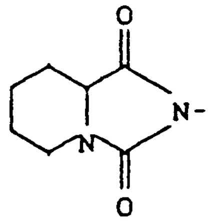 | H | $-O-CH-COOCH_3$<br>$CH_3$ |
| 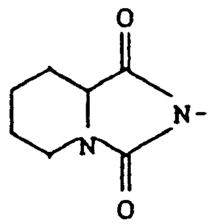 | H | $-S-CH_2-COO$ 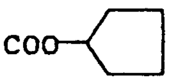 |

| Het | $R^1$ | $R^2$ |
|---|---|---|
| 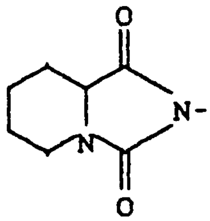 | H | $-S-CH_2-COO-(CH_2)_4-CH_3$ |
| 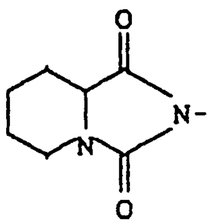 | H | $-O-CH_2-CN$ |
| 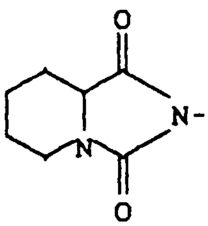 | H | 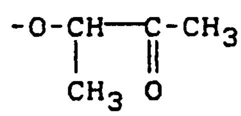 |
| 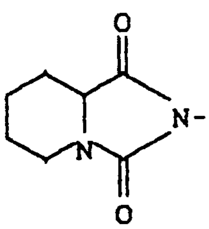 | H | $-S-CH_2-CH=CH_2$ |
| 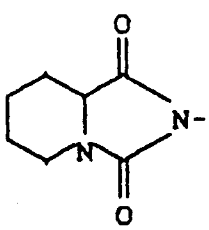 | H | $-S-CH_2-C{\equiv}CH$ |

| Het | $R^1$ | $R^2$ |
|---|---|---|
| 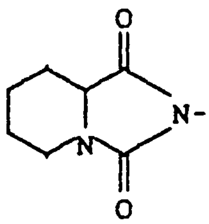 | H | 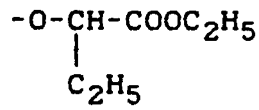 |
| 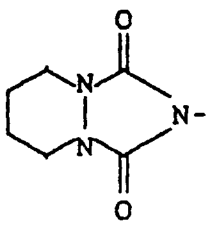 | H | 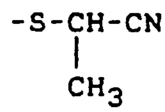 |
| 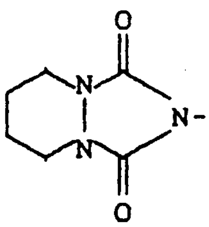 | H | 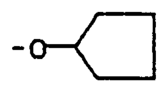 |
| 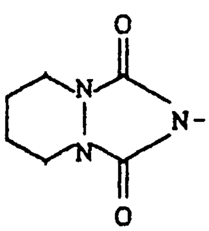 | H | 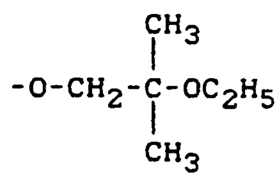 |
| 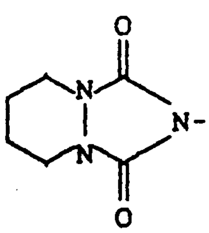 | H | 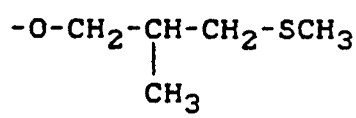 |

| Het | $R^1$ | $R^2$ |
|---|---|---|
| 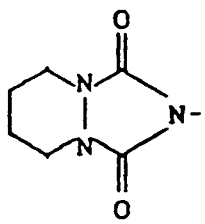 | H | 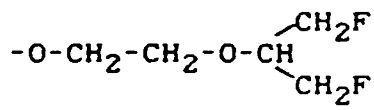 |
| 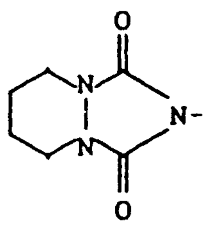 | H | 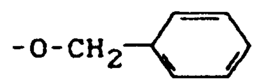 |
| 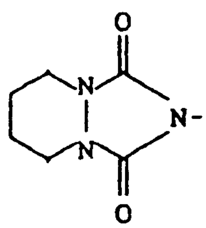 | H | 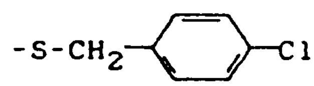 |
| 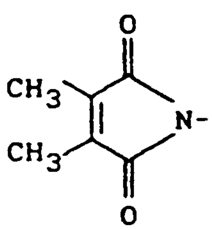 | H | 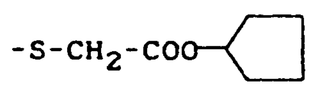 |

| Het | $R^1$ | $R^2$ |
|---|---|---|
| 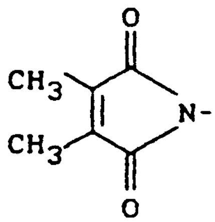 | H | $-S-CH_2-COO-(CH_2)_4-CH_3$ |
| 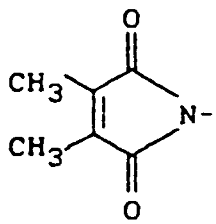 | H | $-S-CH_2-CH=CH_2$ |

| Het | $R^1$ | $R^2$ |
|---|---|---|
| 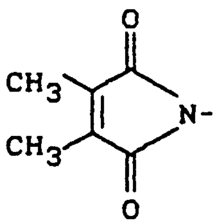 | | $-S-CH_2-C\equiv CH$ |
| 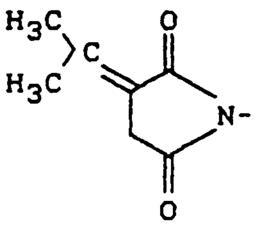 | H | 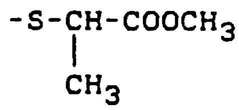 |
| 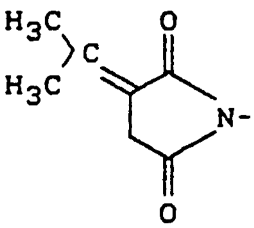 | H | 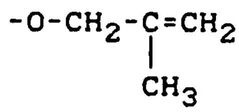 |
| 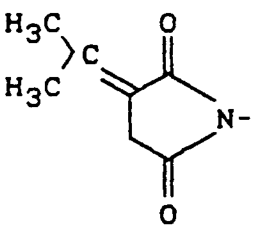 | H | 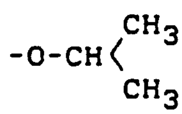 |
| 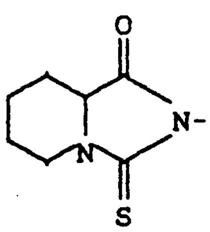 | F | $-O-CH_2-C\equiv CH$ |

| Het | $R^1$ | $R^2$ |
|---|---|---|
| 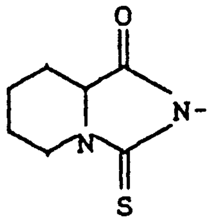 | F | $-O-CH_2-CH=CH_2$ |
| 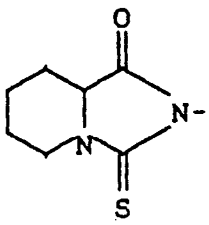 | F | $-O-CH_2-CH_2-OCH_3$ |
| 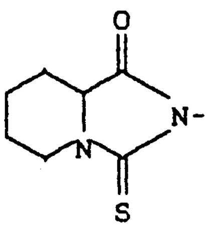 | F | $-S-CH_2-COO-$ 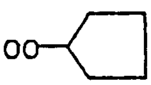 |
| 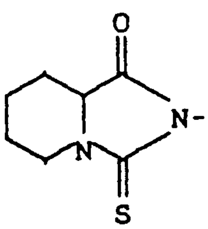 | F | $-O-CH(CH_3)-COOC_2H_5$ |
| 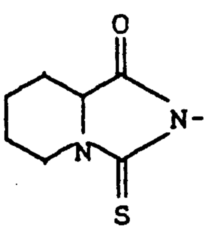 | F | $-O-CH_2-COOC_2H_5$ |

| Het | $R^1$ | $R^2$ |
|---|---|---|
| 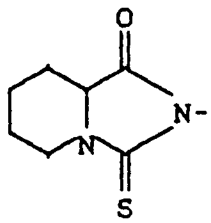 | F | $-O-CH_2-COO-CH_2CH_2-O-CH_3$ |
| 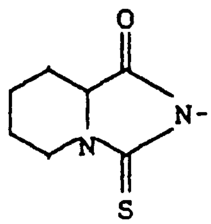 | F | $-O-CH_2-CH_2-O-CH_2-CH_2-OC_2H_5$ |
| 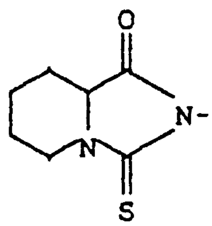 | F | $-S-CH-COOC_2H_5$<br>$CH_3$ |
| 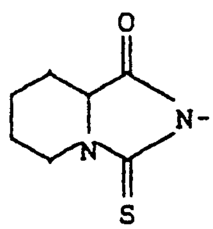 | F | $-O-CH(CH_2F)_2$ |
| 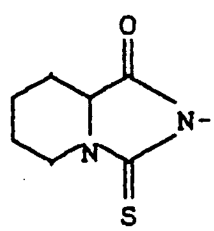 | F | $-O-CH_2-CN$ |

| Het | $R^1$ | $R^2$ |
|---|---|---|
| 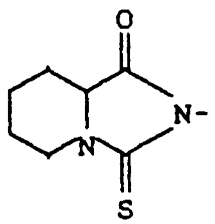 | F | $-S-CH_2-CH=CH_2$ |
| 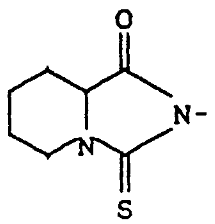 | F | $-S-CH_2-C\equiv CH$ |
| 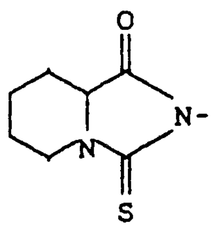 | F | $-O-CH_2-COO-(CH_2)_3-CH_3$ |
| 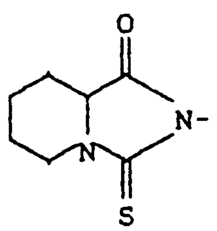 | H | $-O-CH_2-C\equiv CH$ |
| 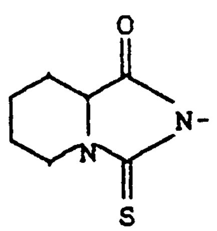 | H | $-O-CH_2-CH=CH_2$ |

| Het | $R^1$ | $R^2$ |
|---|---|---|
| 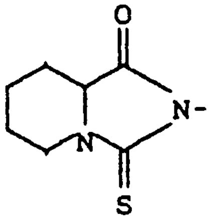 | H | $-O-CH_2-CH_2-OCH_3$ |
| 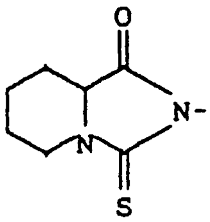 | H | $-S-CH_2-COO-$ 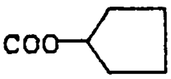 |
| 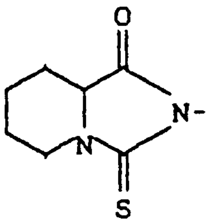 | H | $-O-CH-COOC_2H_5$<br>$CH_3$ |
| 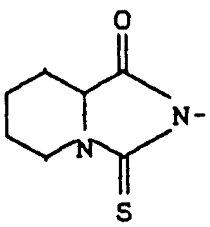 | H | $-O-CH_2-COOC_2H_5$ |
| 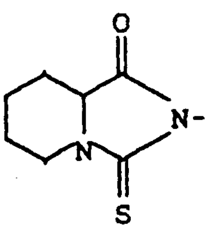 | H | $-O-CH_2-COO-CH_2-CH_2-OCH_3$ |

| Het | $R^1$ | $R^2$ |
|---|---|---|
| 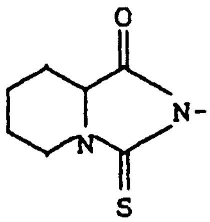 | H | $-O-CH_2-CH_2-O-CH_2-CH_2-OC_2H_5$ |
| 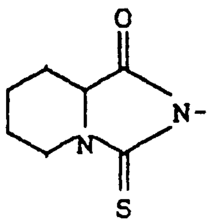 | H | $-S-CH-COOC_2H_5$<br>$\vert$<br>$CH_3$ |
| 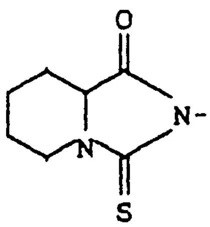 | H | $-O-CH(CH_2F)_2$ |
| 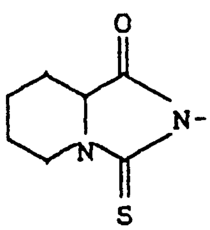 | H | $-O-CH_2-CN$ |
| 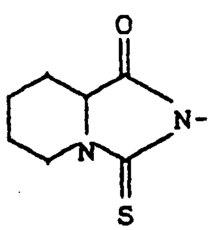 | H | $-S-CH_2-CH=CH_2$ |

| Het | $R^1$ | $R^2$ |
|---|---|---|
| 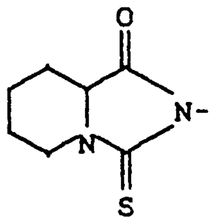 | H | $-S-CH_2-C{\equiv}CH$ |
| 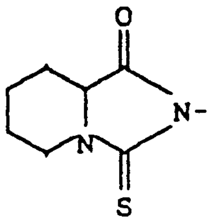 | H | $-O-CH_2-COO-(CH_2)_3-CH_3$ |
| 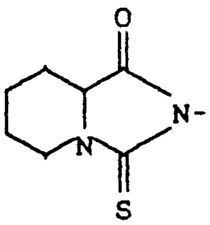 | Cl | $-O-CH_2-C{\equiv}CH$ |
| 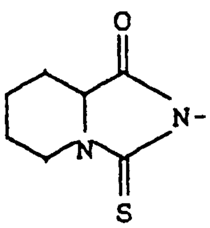 | Cl | $-O-CH_2-CH{=}CH$ |
| 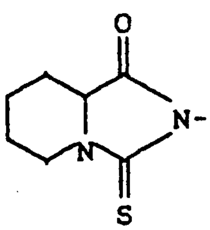 | Cl | $-O-CH_2-CH_2-OCH_3$ |

| Het | $R^1$ | $R^2$ |
|---|---|---|
| 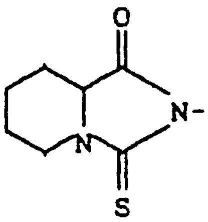 | Cl | $-S-CH_2-COO-$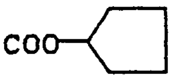 |
| 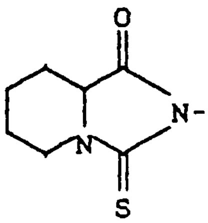 | Cl | $-O-CH(CH_3)-COOC_2H_5$ |
| 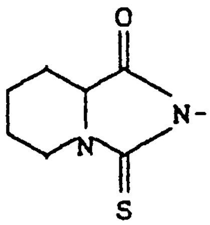 | Cl | $-O-CH_2-COOC_2H_5$ |
| 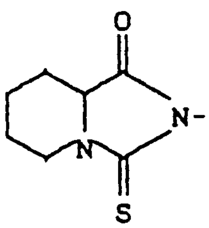 | Cl | $-O-CH_2-COO-CH_2-CH_2-OCH_3$ |
| 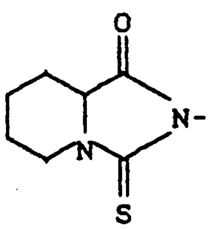 | Cl | $-O-CH_2-CH_2-O-CH_2-CH_2-OC_2H_5$ |

| Het | $R^1$ | $R^2$ |
|---|---|---|
| 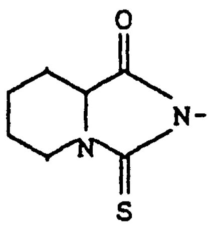 | Cl | $-S-CH(CH_3)-COOC_2H_5$ |
| 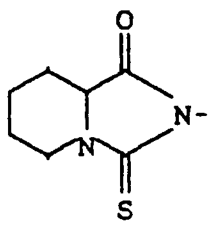 | Cl | $-O-CH(CH_2F)_2$ |
| 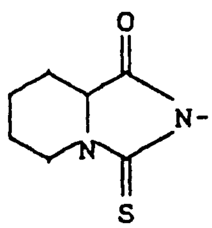 | Cl | $-O-CH_2-CN$ |
| 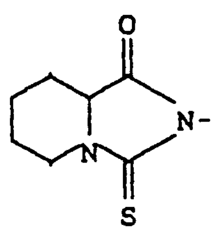 | Cl | $-S-CH_2-CH=CH_2$ |
| 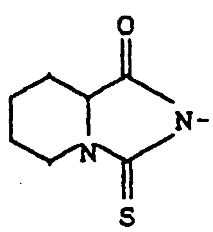 | Cl | $-S-CH_2-C\equiv CH$ |

| Het | $R^1$ | $R^2$ |
| --- | --- | --- |
| 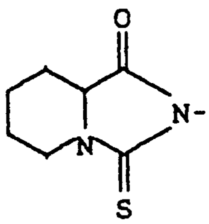 | Cl | $-O-CH_2-COO-(CH_2)_3-CH_3$ |
| 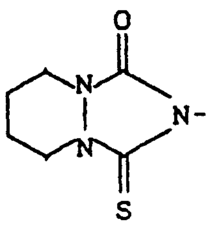 | H | $-O-CH_2-C\equiv CH$ |
| 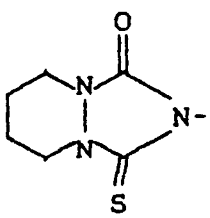 | H | $-O-CH_2-CH=CH_2$ |
| 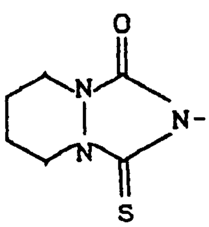 | H | $-O-CH_2-CH_2-OCH_3$ |
| 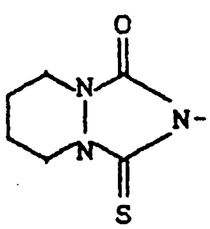 | H | $-S-CH_2-COO$ 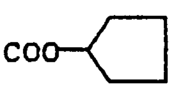 |

| Het | $R^1$ | $R^2$ |
|---|---|---|
| 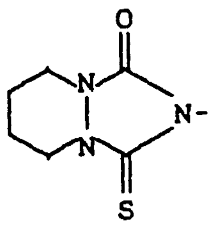 | H | $-O-\underset{CH_3}{CH}-COOC_2H_5$ |
| 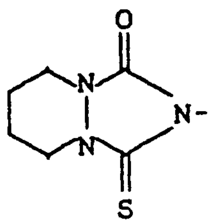 | H | $-O-CH_2-COOC_2H_5$ |
| 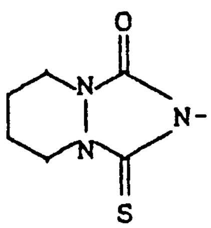 | H | $-O-CH_2-COO-CH_2-CH_2-OCH_3$ |
| 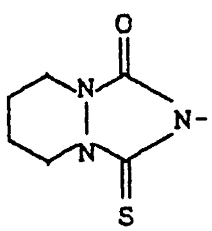 | H | $-O-CH_2-CH_2-O-CH_2-CH_2-OC_2H_5$ |
| 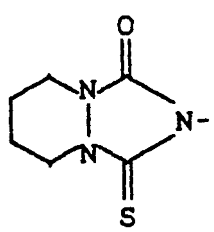 | H | $-S-\underset{CH_3}{CH}-COOC_2H_5$ |

| Het | $R^1$ | $R^2$ |
|---|---|---|
| 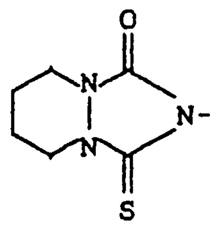 | H | $-O-CH(CH_2F)_2$ |
| 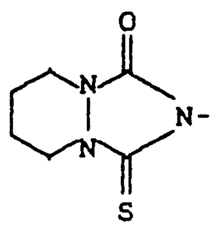 | H | $-O-CH_2-CN$ |
| 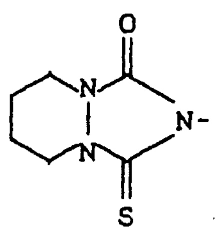 | H | $-S-CH_2-CH=CH_2$ |
| 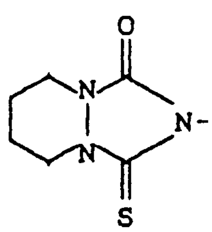 | H | $-S-CH_2-C\equiv CH$ |
| 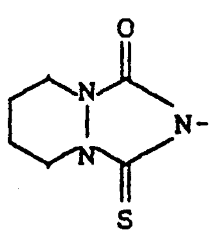 | H | $-O-CH_2-COO-(CH_2)_3-CH_3$ |

| Het | $R^1$ | $R^2$ |
|---|---|---|
| 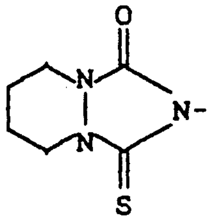 | F | $-O-CH_2-C{\equiv}CH$ |
| 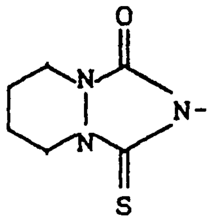 | F | $-O-CH_2-CH{=}CH_2$ |
| 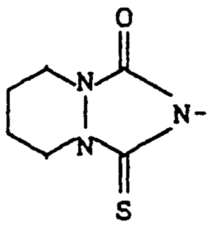 | F | $-O-CH_2-CH_2-OCH_3$ |
| 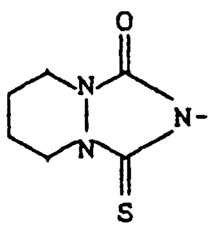 | F | $-S-CH_2-COO$ 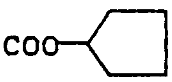 |
| 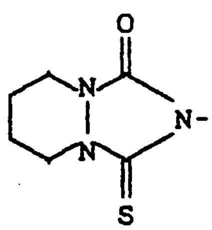 | F | $-O-\underset{CH_3}{\underset{\vert}{CH}}-COOC_2H_5$ |

| Het | $R^1$ | $R^2$ |
|---|---|---|
| 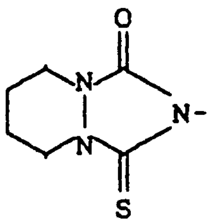 | F | $-O-CH_2-COOC_2H_5$ |
| 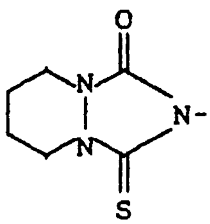 | F | $-O-CH_2-COO-CH_2-CH_2-OCH_3$ |
| 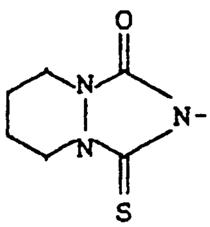 | F | $-O-CH_2-CH_2-O-CH_2-CH_2-OC_2H_5$ |
| 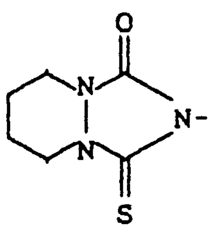 | F | $-S-\underset{CH_3}{\underset{\vert}{CH}}-COOC_2H_5$ |
| 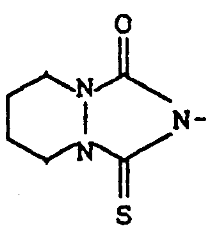 | F | $-O-CH(CH_2F)_2$ |

| Het | $R^1$ | $R^2$ |
|---|---|---|
| 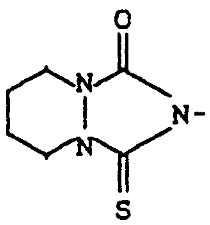 | F | $-O-CH_2-CN$ |
| 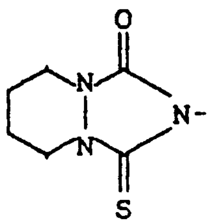 | F | $-S-CH_2-CH=CH_2$ |
| 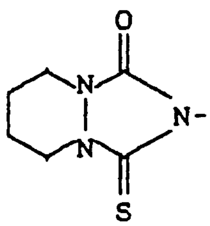 | F | $-S-CH_2-C\equiv CH$ |
| 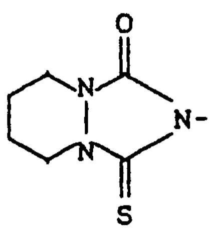 | F | $-O-CH_2-COO-(CH_2)_3-CH_3$ |

Verwendet man beispielsweise 3,4,5,6-Tetrahydrophthalsäureanhydrid und 2,5-Difluor-4-aminobenzonitril als Ausgangsstoffe, so läßt sich der Reaktionsablauf des erfindungsgemäßen Verfahrens (a) durch das folgende Formelschema darstellen:

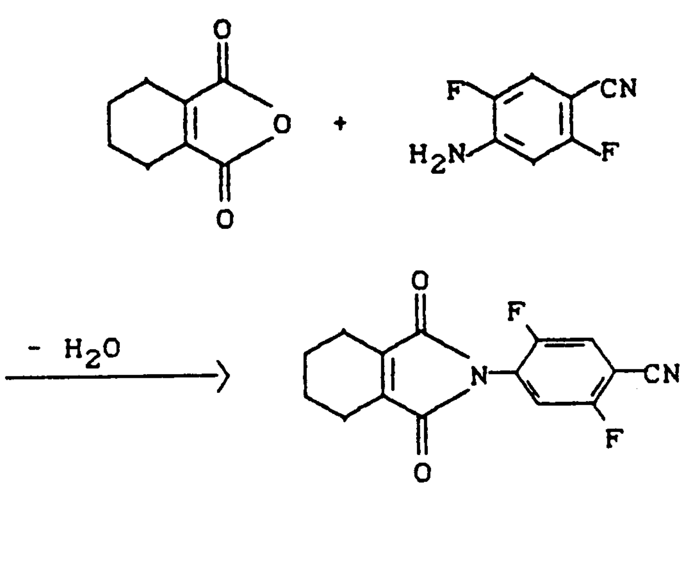

Verwendet man beispielsweise 2-Piperidinylcarbonsäureethylester und 4-Cyano-3-methoxyphenylisocyanat als Ausgangsstoffe, so läßt sich der Reaktionsablauf des erfindungsgemäßen Verfahrens (b) durch das folgende Formelschema darstellen:

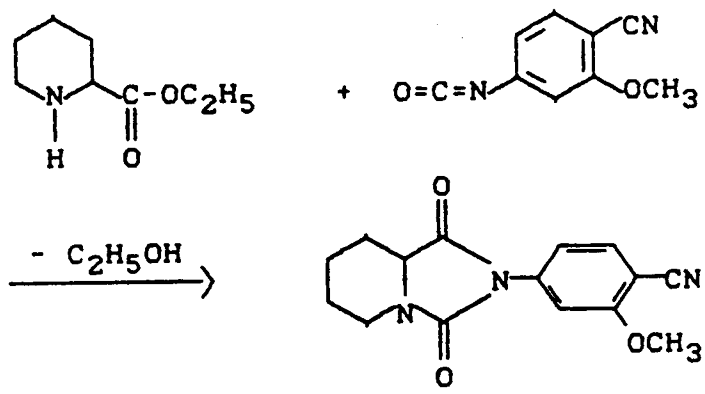


Verwendet man beispielsweise N-(2,5-Difluor-4-cyanophenyl)-3,4-dimethyl-$\Delta^3$-pyrrolin-2,5-dion und t-Butylmercaptan als Ausgangsstoffe, so läßt sich der Reaktionsablauf des erfindungsgemäßen Verfahrens (c) durch das folgende Formelschema darstellen:

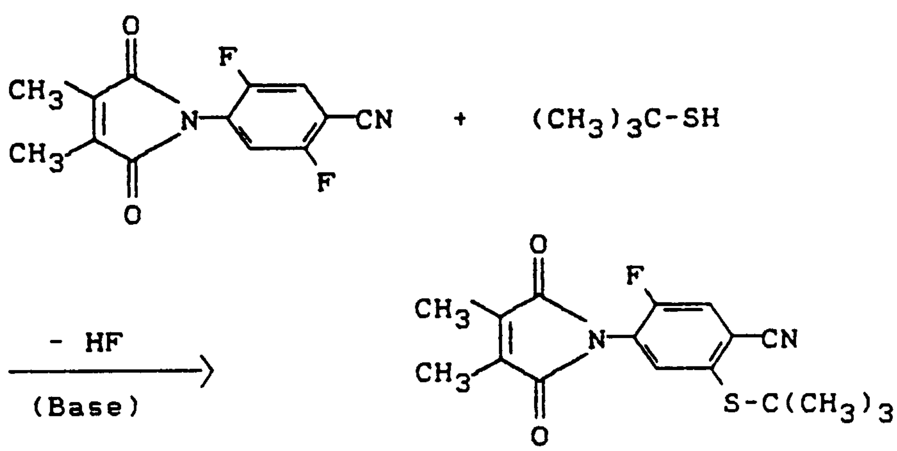


Verwendet man beispielsweise 2-(4-Cyano-2-fluor-5-hydroxyphenyl)-hexahydroimidazo[1,5-a]pyridin-1,3-dion und Allylbromid als Ausgangsstoffe, so läßt sich der Reaktionsablauf des erfindungsgemäßen Verfahrens (d) durch das folgende Formelschema darstellen:

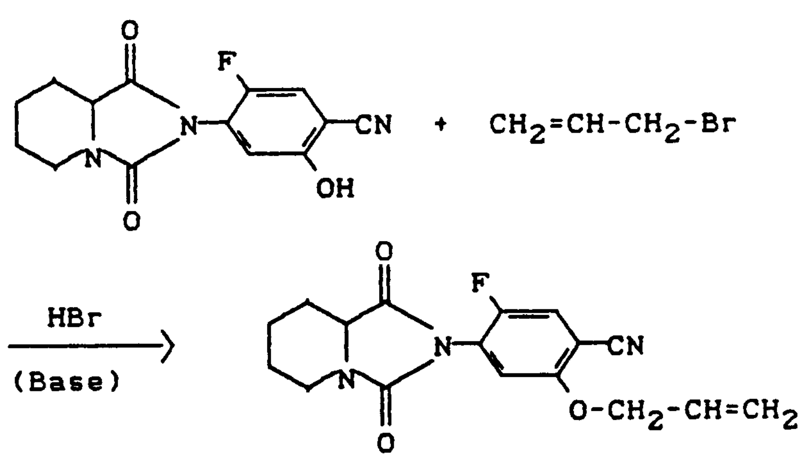

Verwendet man beispielsweise N-(2-Fluor-4-cyano-5-allyl-oxyphenyl)-3,4,5,6-tetrahydrophthalimid als Ausgangsstoff und Tris-triphenylphosphin-rhodiumchlorid als Reduktionsmittel, so läßt sich der Reaktionsablauf des erfindungsgemäßen Verfahrens (e-α) durch das folgende Formelschema darstellen:

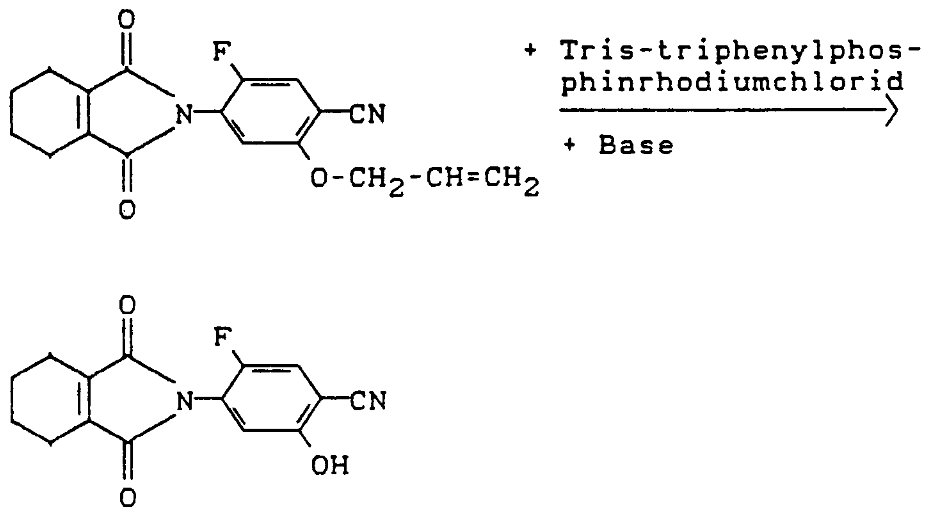


Verwendet man beispielsweise 2-Allyloxy-4-amino-5-fluorbenzonitril als Ausgangsstoff und Tris-triphenylphosphin-rhodiumchlorid als Reduktionsmittel in der 1. Stufe und Tetrahydrophthalsäureanhydrid als Ausgangsstoff in der 2. Stufe, so läßt sich der Reaktionsablauf des erfindungsgemäßen Verfahrens (e-β) durch das folgende Formelschema darstellen:

1. Stufe

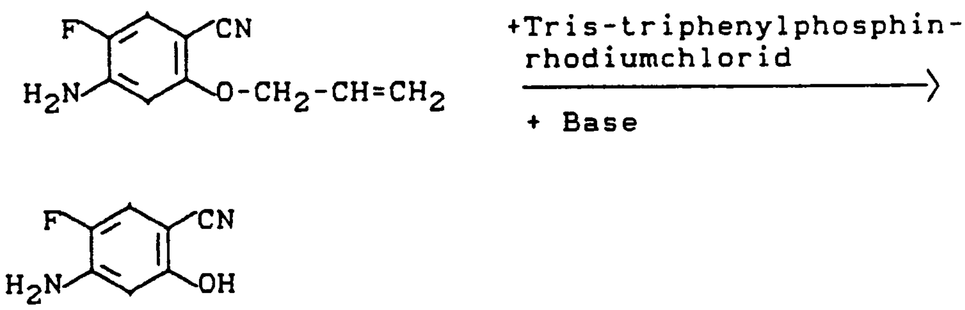


2. Stufe

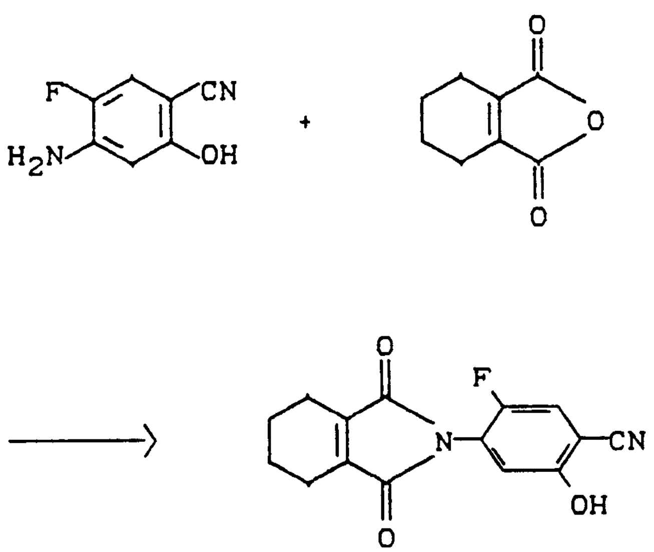

Die zur Durchführung des erfindungsgemäßen Verfahrens (a) als Ausgangsstoffe benötigten Anhydride sind durch die Formel (II) allgemein definiert. In dieser Formel (II) steht A vorzugsweise für einen Rest der Formel

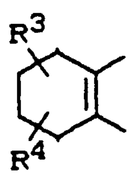 ; 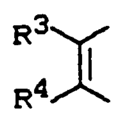 oder 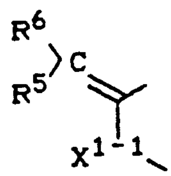

steht, wobei

$X^{1-1}$ für eine $-CH_2$-Gruppe oder für eine

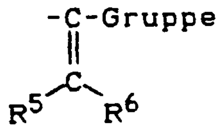

steht und

$R^3$, $R^4$, $R^5$ und $R^6$ vorzugsweise für diejenigen Reste stehen, die bereits in Zusammenhang mit der Beschreibung der erfindungsgemäßen Stoffe der Formel (I) als bevorzugt für diese Substituenten genannt wurden.

Die Anhydride der Formel (II) sind bekannt oder erhältlich in Analogie zu bekannten Verfahren (vgl. z.B. Gazz. chim. Ital. 57, 300-311 [1927]; DE-OS 3 644 222; J. org. Chem. 51, 4150-4158 [1986]; Tetrahedron Lett. 25, 6027-6030 [1984]; J. org. Chem. 42, 4162-4164 [1977]; Liebigs Ann. Chem. 1977, 772-790; Tetrahedron 25, 4099-4108 [1969]; JP 43/9046).

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind bekannt oder erhältlich in Analogie zu bekannten Verfahren (vgl. z.B. EP 224 001; US 4 424 371; Ind. Chim. Belge 39, 490-500 [1974]; Liebigs Ann. Chem. 716, 47-60 [1968]; EP 40 932; Jp 46/3368 sowie die Angaben zum erfindungsgemäßen Verfahren (c)).

Die zur Durchführung des erfindungsgemäßen Verfahrens (b) als Ausgangsstoffe benötigten Carbonsäureester sind durch die Formel (IV) allgemein definiert. In dieser Formel (IV) steht $R^9$ vorzugsweise für einen Rest der Formel

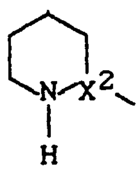 ; 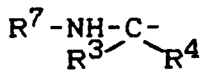 oder 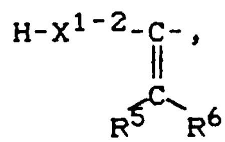

wobei

$X^{1-2}$ für Sauerstoff oder eine

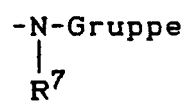

steht und

$X^2$, $R^3$, $R^4$, $R^5$, $R^6$ und $R^7$ vorzugsweise für diejenigen Reste stehen, die bereits im Zusammenhang mit der Beschreibung der erfindungsgemäßen Stoffe der Formel (I) als bevorzugt für diese Substituenten genannt wurden.

$R^{10}$ steht vorzugsweise für geradkettiges oder verzweigtes Alkyl mit 1 bis 4 Kohlenstoffatomen, insbesondere für Methyl oder Ethyl.

Die Carbonsäureester der Formel (IV) sind allgemein bekannte Verbindungen der organischen Chemie oder erhältlich in Analogie zu bekannten Verfahren (vgl. z.B. US 4 730 006. Pestic. Sci. 16, 277-286 [1985]; Chem. Pharm. Bull. 32, 3934-3944 [1984]; Liebigs Ann. Chem. 1983, 1133-1151; Synthesis 1981, 915-917; Tetrahedron Lett. 22, 2485-2486 [1981]; Chem. Ber. 111, 1058-1076 [1978]; Angew. Chem. 89, 344-345 [1977]; Chem. Ber. 110, 942-947 [1977]; Chem. Lett. 1976, 1095-1096; Angew. Chem. 88, 295-296 [1976]; DE 2 058 012; Bull. chem. Soc. Japan 44, 474-477 [1971]; J. chem. Soc. Perkin I, 1987, 877-884; DE 3 702 943; DE 2 331 549; J. Heterocycl. Chem. 6, 181-185, [1969]).

Die zur Durchführung des erfindungsgemäßen Verfahrens (b) weiterhin als Ausgangsstoffe benötigten Iso(thio)cyanate sind durch die Formel (V) allgemein definiert. In dieser Formel (V) stehen $R^1$, $R^2$ und $Z^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (V) sind bekannt oder erhältlich in Analogie zu bekannten Verfahren (vgl. z.B. FR 2 003 438; ZA 67/3761 bzw. CA70; 67 955d; EP 105 991; EP 67 689 oder GB 1 336 871 sowie die Herstellungsbeispiele), beispielsweise wenn man Amine der Formel (III),

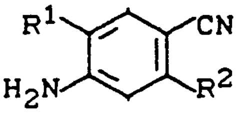

(III)

in welcher

$R^1$ und $R^2$ die oben angegebene Bedeutung haben,

mit Phosgen, Thiophosgen oder Diphosgen ($Cl_3C$-O-CO-Cl) gegebenenfalls in Gegenwart eines Verdünnungsmittels wie beispielsweise Toluol bei Temperaturen zwischen 20 °C und 120 °C umsetzt.

Die zur Durchführung des erfindungsgemäßen Verfahrens (c) als Ausgangsstoffe benötigten N-Aryl-Stickstoffheterocyclen sind durch die Formel (If) allgemein definiert. In dieser Formel (If) stehen Het und $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-1}$ steht vorzugsweise für Fluor, Chlor oder Brom, insbesondere für Fluor.

Die N-Aryl-Stickstoffheterocyclen der Formel (If) sind erfindungsgemäße Verbindungen und erhältlich mit Hilfe der erfindungsgemäßen Verfahren (a) oder (b).

Die zur Durchführung des erfindungsgemäßen Verfahrens (c) weiterhin als Ausgangsstoffe benötigten Alkohole oder Thiole sind durch die Formel (VI) allgemein definiert. In dieser Formel (VI) stehen $Z^2$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

Die Alkohole und Thiole der Formel (VI) sind allgemein bekannte Verbindungen der organischen Chemie.

Die zur Durchführung des erfindungsgemäßen Verfahrens (d) als Ausgangsstoffe benötigten N-Aryl-Stickstoffheterocyclen sind durch die Formel (Ie) allgemein definiert. In dieser Formel (Ie) stehen Het und $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (Ie) sind erfindungsgemäße Verbindungen und erhältlich mit Hilfe des erfindungsgemäßen Verfahrens (e).

Die zur Durchführung des erfindungsgemäßen Verfahrens (d) weiterhin als Ausgangsstoffe benötigten Alkylierungsmittel sind durch die Formel (VII) allgemein definiert. In dieser Formel (VII) steht $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

E steht für einen bei Alkylierungsmitteln üblichen Abgangsrest, vorzugsweise für Halogen, insbesondere für Chlor, Brom oder Iod,oder für jeweils gegebenenfalls substituiertes Alkylsulfonyloxy mit vorzugsweise 1 bis 4 Kohlenstoffatomen, Alkoxysulfonyloxy mit vorzugsweise 1 bis 4 Kohlenstoffatomen oder Arylsulfonyloxy mit vorzugsweise 6 bis 10 Kohlenstoffatomen, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (VII) sind allgemein bekannte Verbindungen der organischen Chemie.

Die zur Durchführung des erfindungsgemäßen Verfahrens (e-$\alpha$) als Ausgangsstoffe benötigten N-Aryl-Stickstoffheterocyclen der Formel (Ig) sind erfindungsgemäße Verbindungen und erhältlich mit Hilfe der erfindungsgemäßen Verfahren (a), (b) oder (c). In der Formel (Ig) stehen $R^1$ und Het vorzugsweise für diejenigen Reste, die bereits im Zusammenhang mit der Beschreibung der erfindungsgemäßen Stoffe der Formel (I) als bevorzugt für diese Substituenten genannt wurden, $R^{8-1}$ steht für Allyl oder Benzyl.

Die zu Durchführung des erfindungsgemäßen Verfahrens (e-$\beta$) als Ausgangsstoffe benötigten Anhydride der Formel (II) und Amine der Formel (IIIa) wurden bereits oben im Zusammenhang mit der Beschreibung der Verbindungen der Formel (II) und (III) beschrieben. Die bei der Durchführung des erfindungsgemäßen Verfahrens (e-$\beta$) anfallenden Aminophenole der Formel (VIII) sind teilweise bekannt (vgl. z.B. Ind. J. Chem. 1613, 297 -300 (1978); J. Org. Chem. 52, 144 - 149 (1987).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1.0 bis 1.5 Mol, vorzugsweise 1.0 bis 1.2 Mol an Amin der Formel (III) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahren (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole wie Methanol, Ethanol oder Propanol.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (b) setzt man pro Mol an Carbonsäureester der Formel (IV) oder eines entsprechenden Säureadditionssalzes im allgemeinen 0.5 bis 5.0 Mol, vorzugsweise 0.8 bis 1.5 Mol an Iso(thio)cyanat der Formel (V) und gegebenenfalls 1.0 bis 10.0 Mol, vorzugsweise 1.0 bis 5.0 Mol an Reaktionshilfsmittel ein. Dabei ist es auch möglich die Iso(thio)cyanate der Formel (V) in einer vorgelagerten Reaktion aus Aminen der Formel (III) und Phosgen, Thiophosgen oder Diphosgen ($Cl_3C$-O-CO-Cl) direkt im Reaktionsgefäß herstellen und ohne Isolierung im "Eintopfverfahren" mit den Carbonsäureestern der Formel (IV) weiter umzusetzen.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (c) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate oder -carbonate, wie beispielsweise Natriumhydrid, Natriumamid, Natriumhydroxid, Natriummethylat, Natriumethylat, Kalium-t-butylat oder Kaliumcarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN)

oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (c) setzt man pro Mol an N-Aryl-Stickstoffheterocyclus der Formel (If) im allgemeinen 1.0 bis 3.0 Mol, vorzugsweise 1.0 bis 1.5 Mol an Alkohol oder Thiol der Formel (VI) und 1.0 bis 3.0 Mol, vorzugsweise 1.0 bis 2.0 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (d) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylierungsmittel der Formel (VII) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (d) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (d) setzt man pro Mol N-Aryl-Stickstoffheterocyclus der Formel (Ie) im allgemeinen jeweils 1,0 bis 20,0 Mol, vorzugsweise jeweils 1,0 bis 15,0 Mol, an Alkylierungsmittel der Formel (VII) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (Id) erfolgt nach üblichen Methoden.

Als Reduktionsmittel zur Durchführung des erfindungsgemäßen Verfahrens (e-$\alpha$) und (e-$\beta$) ($R^{8-1}$ = Benzyl) kommen alle üblichen für derartige Reduktionsreaktionen verwendbaren Reduktionsmittel infrage. Vorzugsweise verwendet man Tris-Triphenylphosphin-Rhodiumchlorid, Tris-Triphenylphosphin-Palladiumchlorid oder molekularen Wasserstoff, welcher in Gegenwart eines üblichen Hydrierkatalysators, wie beispielsweise Raney-Nickel oder Palladium, verwendet wird.

Als Verdünnungsmittel zur Durchführung der erfindungsgemäßen Verfahren (e-$\alpha$) und (e-$\beta$) kommen in Abhängigkeit vom verwendeten Reduktionsmittel alle üblichen organischen oder anorganischen Lösungsmittel in Frage. Vorzugsweise verwendet man Ether wie Diethylether, Dioxan oder Tetrahydrofuran, Alkohole wie Methanol, Ethanol oder Propanol, Carbonsäuren wie Essigsäure oder Propionsäure oder Gemische der o.g. Lösungsmittel mit Carbonsäuren.

Als Reaktionshilfsmittel zur Durchführung des erfindungsgemäßen Verfahrens (e-$\alpha$) und (e-$\beta$) ($R^{8-1}$ = Benzyl) kommen alle für derartige Reaktionen üblichen Reaktionshilfsmittel in Frage. Vorzugsweise verwendet man tertiäre Amine wie beispielsweise Pyridin, Triethylamin, Diazabicyclooctan (DABCO), Diazabicyclononen (DBN) oder Diazabicycloundecen (DBU).

Als Säuren setzt man bei der Durchführung der erfindungsgemäßen Verfahren (e-$\alpha$) und (e-$\beta$) ($R^{8-1}$ = Allyl) alle üblichen für derartige Reaktionen verwendbaren Säuren ein. Vorzugsweise verwendet man anorganische oder organische Säuren wie beispielsweise Salzsäure, Schwefelsäure oder Salpetersäure oder aliphatische oder aromatische Carbonsäuren wie beispielsweise Essigsäure, Propionsäure oder Benzoesäure oder einen sauren Ionenaustauscher.

Die Reaktionstemperaturen können bei der Durchführung des erfindungsgemäßen Verfahrens (e-$\alpha$) und (e-$\beta$)/2. Stufe in Abhängigkeit vom verwendeten Reduktionsmittel in einem größeren Bereich variiert werden. Im allgemeinen arbeitet man bei Temperaturen zwischen 0 und 150 °C, vorzugsweise zwischen 20 und 120 °C.

Zur Durchführung der erfindungsgemäßen Verfahren (e-$\alpha$) und (e-$\beta$) ($R^{8-1}$ = Benzyl) setzt man pro Mol an Verbindung der Formel (I-g) bzw. (IIIa) im allgemeinen 1 Mol bis 5 Mol, vorzugsweise 1 Mol bis 3 Mol an

Reduktionsmittel und gegebenenfalls 0,001 Mol bis 0,5 Mol, vorzugsweise 0,05 Mol bis 0,3 Mol an Katalysator und gegebenenfalls katalytische Mengen bis 5 Mol, vorzugsweise katalytische Mengen bis 3 Mol an Reaktionshilfsmittel ein.

Zur Durchführung der erfindungsgemäßen Verfahren (e-α) und (e-β) ($R^{8-1}$ = Allyl) setzt man pro Mol an Verbindung der Formel (Ig) bzw. (IIIa) im allgemeinen 0,001 Mol bis 1 Mol, vorzugsweise 0,05 Mol bis 0,5 Mol, an Edelmetallkatalysator und 0,1 Mol bis 5 Mol, vorzugsweise 0,2 Mol bis 3 Mol an Säure ein.

Als Verdünnungsmittel und als Reaktionshilfsmittel kommen bei der 2. Stufe des erfindungsgemäßen Verfahrens (e-β) die bei Verfahren (a) bereits genannten Lösungsmittel und Reaktionshilfsmittel in Frage.

Die Reaktionstemperaturen können bei der Durchführung des erfindungsgemäßen Verfahrens (e-β) 2. Stufe in einem größeren Bereich variiert werden. Im allgemeinen arbeitet man bei Temperaturen zwischen 20 °C und 180 °C, vorzugsweise bei Temperaturen zwischen 50 °C und 150 °C.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung von mono- und dikotylen Unkräutern in mono- und dikotylen Kulturen, wie beispielsweise Soja, Getreide, Mais, Reis oder Baumwolle einsetzen.
Auch die Zwischenprodukte der Formel (VII) besitzen gute herbizide Wirksamkeit.

In geeigneten Aufwandmengen lassen sich die erfindungsgemäßen Wirkstoffe auch als Pflanzenwachstumsregulatoren, insbesondere als Defoliantien einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit Chloressigsäure-N-(methoxymethyl)-2,6-diethylanilid (ALACHLOR);
2-Ethyl-6-methyl-N-(1-methyl-2-methoxyethyl)-chloracetanilid (METOLACHLOR);
5-(2-Chlor-4-trifluormethyl-phenoxy)-2-nitro-benzoesäure (ACIFLUORFEN);
Methyl-5-(2,4-dichlorphenoxy)-2-nitrobenzoat (BIFENOX);
5-(2-Chlor-4-trifluormethyl-phenoxy)-N-methylsulfonyl-2-nitrobenzamid (FOMESAFEN);
(2-Ethoxy-1-methyl-2-oxo-ethyl)-5-[2-chlor-4-(trifluormethyl)-phenoxy]-2-nitrobenzoat (LACTOFEN);
(2-Chlor-4-trifluormethylphenyl)-(3-ethoxy-4-nitrophenyl)-ether (OXYFLUORFEN);
2-[4,5-Dihydro-4-methyl-4-isopropyl-5-oxo-(1H)-imidazol-2-yl]-5-ethyl-pyridin-3-carbonsäure (IMAZETHAPYR);
Methyl-2-[4,5-dihydro-4-methyl-4-(1-methylethyl)-5-oxo-1H-imidazol-2-yl]-4(5)-methylbenzoat (IMAZAMETHABENZ);
2-[5-Methyl-5-(1-methylethyl)-4-oxo-2-imidazolin-2-yl]-3-chinolincarbonsäure (IMAZAQUIN);
2-[[[[[(4,6-Dimethoxypyrimidin-2-yl)-amino]-carbonyl]-amino]-sulfonyl]-methyl]-benzoesäuremethylester, (BENSULFURON);
Ethyl-2-{[(4-chlor-6-methoxy-2-pyrimidinyl)-aminocarbonyl]-aminosulfonyl}-benzoat (CHLORIMURON);
2-Chlor-N-{[(4-methoxy-6-methyl-1,3,5-triazin-2-yl)-amino]-carbonyl}-benzolsulfonamid (CHLORSULFURON);
2-{[[((4-Methoxy-6-methyl-1,3,5-triazin-2-yl)-amino)-carbonyl]-amino]-sulfonyl}-benzoesäure oder deren Methylester (METSULFURON);
3-[[[[(4-Methoxy-6-methyl-1,3,5-triazin-2-yl)-amino]-carbonyl]-amino]-sulfonyl]-thiophen-2-carbonsäuremethylester (THIAMETHURON);
2-Chlor-4-ethylamino-6-isopropylamino-1,3,5-triazin (ATRAZIN);
2-Chlor-4-ethylamino-6-(3-cyanopropylamino)-1,3,5-triazin (CYANAZIN);
3-Isopropyl-2,1,3-benzothiadiazin-4-on-2,2-dioxid (BENTAZON);
4-Amino-6-t-butyl-3-ethylthio-1,2,4-triazin-5(4H)-on (ETHIOZIN);
3-Cyclohexyl-6-dimethylamino-1-methyl-1,3,5-triazin-2,4-dion (HEXAZINONE);
2-[(2-Chlorphenyl)-methyl]-4,4-dimethylisoxazolidin-3-on (DIMETHAZONE);
sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Herstellungsbeispiele

Beispiel 1

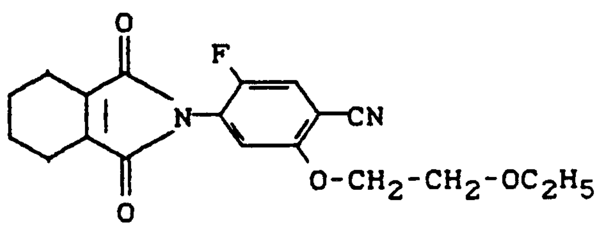

(Verfahren a)

4,8 g (0,0214 Mol) 2-(2-Ethoxyethoxy)-4-amino-5-fluorbenzonitril und 3,8 g (0,025 Mol) 3,4,5,6-Tetrahydrophthalsäureanhydrid werden in Gegenwart von 0,57 g (0,003 Mol) p-Toluolsulfonsäure 2 Stunden auf 100 °C erhitzt, das abgekühlte Reaktionsgemisch in 40 ml Tetrahydrofuran aufgenommen in wässrige Natriumhydrogencarbonatlösung eingerührt, mehrfach mit Dichlormethan extrahiert, über Natriumsulfat getrocknet, im Vakuum eingeengt und durch Chromatographie an Kieselgel (Laufmittel: Cyclohexan/Essigester 2:1) gereinigt. Das so erhältliche Produkt läßt sich aus Ether/n-Hexan umkristallisieren.

Man erhält 3,04 g (40 % der Theorie) an N-[4-Cyano-5-(2-ethoxyethoxy)-2-fluorphenyl]-3,4,5,6-tetrahydrophthalimid vom Schmelzpunkt 90 °C.

Beispiel 2

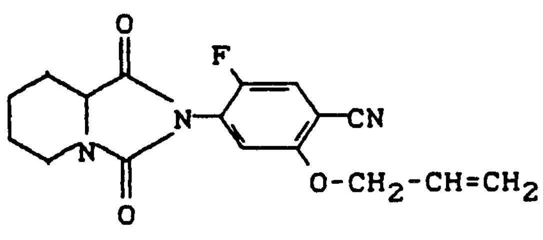

(Verfahren b-Eintopfvariante)

Zu 2,5 g (0,013 Mol) 2-Allyloxy-4-amino-5-fluorbenzonitril in 25 ml absolutem Toluol gibt man bei 80 °C tropfenweise unter Rühren 1,58 ml (0,013 Mol) Diphosgen, erhitzt anschließend 4 Stunden auf Rückflußtemperatur, destilliert dann das Toluol ab, versetzt den Rückstand erneut mit 15 ml absolutem Toluol und 2,2 ml (0,014 Mol) Piperidin-2-carbonsäureethylester, gibt 1 Tropfen Diazabicycloundecen (DBU) zu und erhitzt für 3 Stunden auf Rückflußtemperatur. Die erkaltete Reaktionsmischung wird filtriert, das Filtrat im Vakuum eingeengt, der Rückstand über Kieselgel (Laufmittel: Cyclohexan/Essigester 2:3) chromatographiert und anschließend aus Ether/n-Hexan umkristallisiert.

Man erhält 2,27 g (53 % der Theorie) an 2-(5-Allyloxy-4-cyano-2-fluorphenyl)-hexahydroimidazo[1,5-a]-pyridin-1,3-dion vom Schmelzpunkt 132 °C.

Beispiel 3

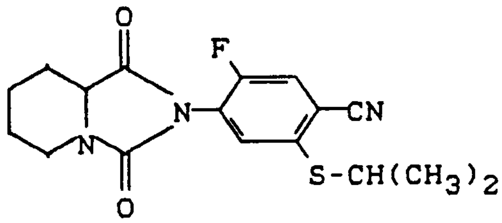

(Verfahren c)

Zu 1,3 ml (0,014 Mol) Isopropylmercaptan in 30 ml absolutem Acetonitril gibt man 1,12 g (0,02 Mol) gepulvertes Kaliumhydroxid und anschließend 2-(4-Cyano-2,5-difluorphenyl)-hexahydroimidazo[1,5-a]-pyridin-1,3-dion) rührt 15 Minuten bei 40 °C, filtriert, engt das Filtrat ein und reinigt den Rückstand durch Chromatographie an Kieselgel (Laufmittel: Dichlormethan/Essigester 40:1) und Umkristallisation aus Ether/n-Hexan.

Man erhält 1,72 g (38 % der Theorie) an 2-(4-Cyano-2-fluor-5-isopropylthiophenyl)-hexahydroimidazo-[1,5-a]pyridin-1,3-dion vom Schmelzpunkt 61 °C.

Beispiel 4

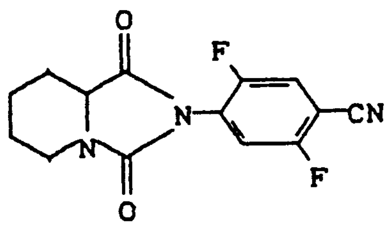

(Verfahren b-Eintopfvariante)

Zu 7,7 g (0,05 Mol) 4-Amino-2,5-difluorbenzonitril in 100 ml absolutem Toluol tropft man innerhalb von 30 Minuten bei 80 °C 6,05 ml (0,05 Mol) Diphosgen und erhitzt anschließend für 5 Stunden auf Rückflußtemperatur. Anschließend wird das Lösungsmittel abdestilliert, der Rückstand mit 8,2 ml (0,052 Mol) Piperidin-2-carbonsäureethylester in 50 ml absolutem Toluol und 1 Tropfen Diazabicycloundecen (DBU) versetzt und 3 Stunden auf Rückflußtemperatur erhitzt. Nach Erkalten der Lösung wird der Rückstand abgesaugt, das Filtrat eingeengt und über Kieselgel (Laufmittel: Cyclohexan/Essigester 2:1) chromatographiert.

Durch Umkristallisieren aus Toluol/n-Hexan erhält man 9,55 g (65,6% der Theorie) an 2-(4-Cyano-2,5-difluorphenyl)-hexahydroimidazo[1,5a]pyridin-1,3-dion vom Schmelzpunkt 95 °C.

Beispiel 5

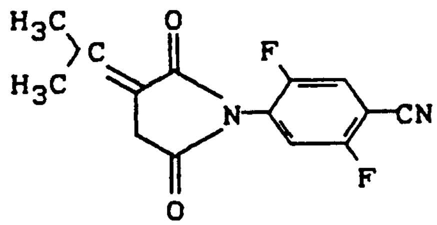

(Verfahren a)

3,68 g (0,0263 Mol) 2-Isopropylidenbernsteinsäureanhydrid und 3,85 g (0,025 Mol) 2,5-Difluor-4-aminobenzonitril (vgl. EP-A 224 001) in 70 ml Eisessig werden 5 Stunden auf 135 °C erhitzt, anschließend eingeengt, der Rückstand in 30 ml Acetanhydrid aufgenommen, mit 1,5 g (0,018 Mol) Natriumacetat versetzt und weitere 2 Stunden auf 90 °C erhitzt. Zur Aufarbeitung wird eingeengt, der Rückstand zwischen Dichlormethan und Wasser verteilt, die organische Phase mit Wasser gewaschen, über Natriumsulfat getrocknet, im Vakuum eingeengt und durch Chromatographie an Kieselgel gereinigt.

Man erhält 2,54 g (36 % der Theorie) an 1-(2,5-Difluor-4-cyanophenyl)-4-isopropyliden-pyrrolidin-2,5-dion vom Schmelzpunkt 135 °C.

Beispiel 6

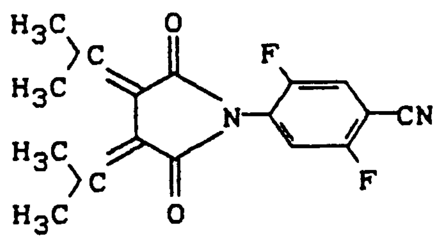

(Verfahren a)

2,85 g (0,0158 Mol) 3,4-Diisopropylidenbernsteinsäureanhydrid und 2,31 g (0,015 Mol) 2,5-Difluor-4-aminobenzonitril (vgl. EP-A 224 001) in 60 ml Eisessig werden 5 Stunden auf 135 °C erhitzt, anschließend eingeengt, der Rückstand in 18 ml Acetanhydrid aufgenommen, mit 0,9 g (0,011 Mol) Natriumacetat versetzt und für weitere 2 Stunden auf 90 °C erhitzt. Zur Aufarbeitung wird eingeengt, der Rückstand zwischen Dichlormethan und Wasser verteilt, die organische Phase mit Wasser gewaschen, über Natriumsulfat getrocknet, im Vakuum eingeengt und durch Chromatographie an Kieselgel gereinigt.

Man erhält 0,73 g (15,4 % der Theorie) an 1-(2,5-Difluor-4-cyanophenyl)-3,4-diisopropyliden-pyrrolidin-2,5-dion vom Schmelzpunkt 144 °C.

In entsprechender Weise und gemäß den allgemeinen Angaben zur Herstellung erhält man die folgenden N-Aryl-Stickstoffheterocyclen der allgemeinen Formel (I):

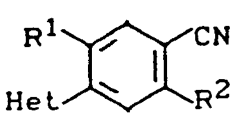

(I)

Tabelle 1

| Bsp. Nr. | Het | $R^1$ | $R^2$ | physikalische Eigenschaften |
|---|---|---|---|---|
| 7 | 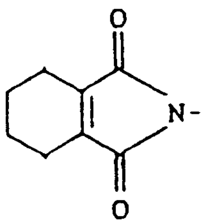 | F | $-OCF_3$ | $^1H$-NMR*): 1,85; 2,47; 7,39; 7,58; |
| 9 | 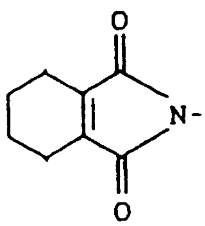 | F | $-O-CH_2-\overset{CH_3}{\overset{\vert}{C}}=CH_2$ | $^1H$-NMR*): 1,85; 2,45; 6,88; 7,44 |

Tabelle 1 - Fortsetzung

| Bsp. Nr. | Het | $R^1$ | $R^2$ | physikalische Eigenschaften |
|---|---|---|---|---|
| 10 | 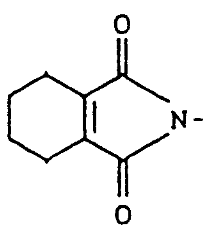 | F | $-O-CH_2-CH=CH-CH_3$ | Fp 118-121° C |
| 12 | 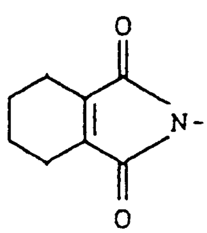 | F | $-O-CH_2-\overset{CH_3}{\overset{\vert}{CH}}-OC_2H_5$ | Fp 118-121° C |
| 14 | 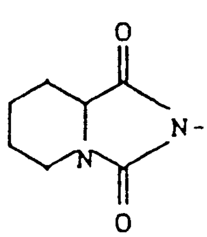 | F | $-S-C(CH_3)_3$ | Fp 55° C |

Tabelle 1 - Fortsetzung

| Bsp. Nr. | Het | $R^1$ | $R^2$ | physikalische Eigenschaften |
|---|---|---|---|---|
| 15 | 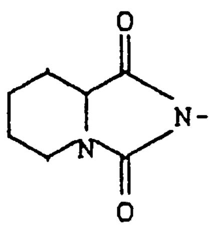 | F | $-O-CH_2-CH_2-OC_2H_5$ | Fp $112^{\circ}$ C |
| 16 | 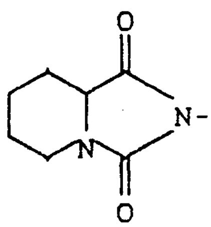 | F | $-S-CH_2-$ 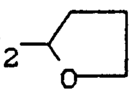 | Fp $105^{\circ}$ C |
| 17 | 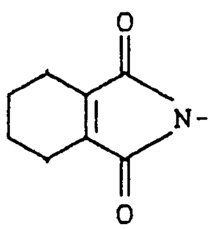 | F | $-O-(CH_2)_2-O-(CH_2)_2-OC_2H_5$ | Fp $86^{\circ}$ C |
| 18 | 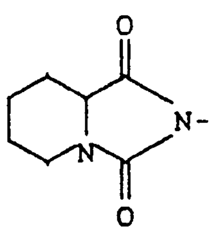 | F | $-O-(CH_2)_2-O-(CH_2)_2-O-C_2H_5$ | Fp $80^{\circ}$ C |
| 19 | 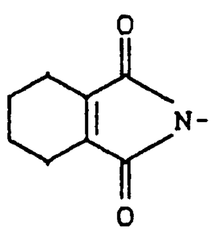 | H | $-O-CH_2-CH_2-OC_2H_5$ | Fp $108-115^{\circ}$ C |

Tabelle 1 - Fortsetzung

| Bsp. Nr. | Het | $R^1$ | $R^2$ | physikalische Eigenschaften |
|---|---|---|---|---|
| 20 | 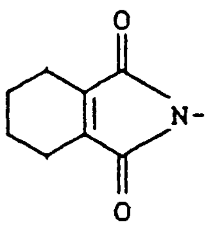 | F | F | Fp 115° C |
| 22 | 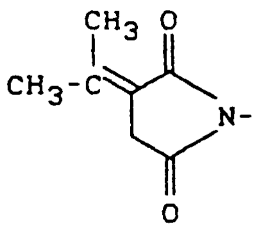 | F | $-O-(CH_2)_2-O-(CH_2)_2-OC_2H_5$ | Fp.: 80-85° C |
| 23 | 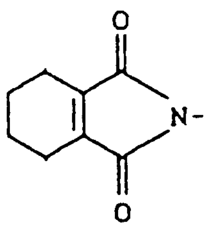 | F | $-S-CH_2-COOC_2H_5$ | |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Tabelle 1 - Fortsetzung

| Bsp. Nr. | Het | $R^1$ | $R^2$ | physikalische Eigenschaften |
|---|---|---|---|---|
| 24 | 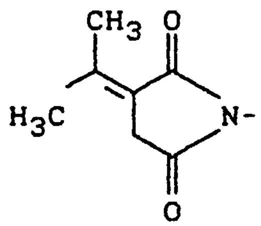 | F | $-O-CH_2-CH=CH_2$ | Fp.: 114° C° C |
| 25 | 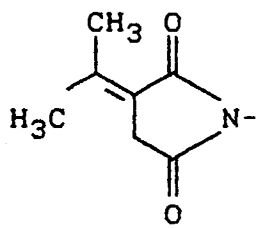 | F | $-OCH_2CH_2OC_2H_5$ | |

Beispiel 26

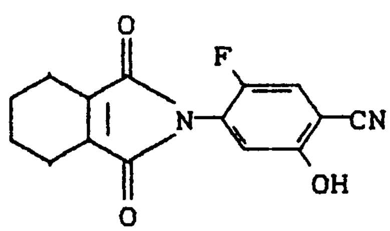

Zu 1 g (0,003 Mol) N-(2-Fluor-4-cyano-5-allyloxyphenyl)-3,4,5,6-tetrahydrophthalimid in 10 ml 10prozentigem wässrigem Ethanol gibt man 0,2 g (0,0002 Mol) Tris-triphenylphosphin-rhodiumchlorid und 70 mg (0,0006 Mol) 1,4-Diazabicyclo[2,2,2]octan (DABCO), erhitzt 3 Stunden auf Rückflußtemperatur, verteilt anschließend die abgekühlte Reaktionsmischung zwischen 30 ml Ether und 10 ml 1 normaler Salzsäure, trennt die organische Phase ab, trocknet über Natriumsulfat engt im Vakuum ein und erhält dann 0,56 g (65 % der Theorie) an N-(4-Cyano-2-fluor-5-hydroxyphenyl)-3,4,5,6-tetrahydrophthalimid als wachsartigen Feststoff.

MS: m/e (% rel. Intensität) = 287 (16); 286 (61) [$M^+$]; 108 (81); 80 (73); 79 (100).

Herstellung der Ausgangsverbindungen

Beispiel VIII-1

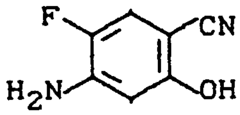

Zu 3 g (0,0156 Mol) 2-Allyloxy-4-amino-5-fluorbenzonitril in 50 ml 10prozentigem wässrigem Ethanol gibt man 0,46 g (0,005 Mol) Tris-triphenylphosphin-rhodiumchlorid und 0,36 g (0,0032 Mol) 1,4-Diazabicyclo-[2,2,2]octan (DABCO), erhitzt 3 Stunden auf Rückflußtemperatur, kühlt ab, gibt 100 ml Ether zu, wäscht mit 30 ml Wasser, trocknet über Natriumsulfat und entfernt das Lösungsmittel im Vakuum.

Man erhält 0,4 g (17 % der Theorie) an 4-Amino-5-fluor-2-hydroxybenzonitril.
IR (rein): $\gamma$ = 3050-3530; 2260; 1635 $cm^{-1}$
$^1$H-NMR ($CD_3OD$) : $\delta$ = 6,28 (d;1H; J = 7Hz) 7,04 (d;1H; J = 10,5 Hz)

Beispiel III-1

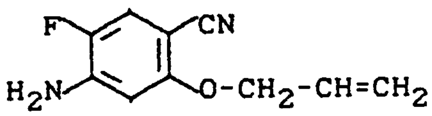

Zu 1,8 g (0,06 Mol) Natriumhydrid in 30 ml N-Methylpyrrolidon gibt man bei Raumtemperatur tropfenweise unter Rühren 4,07 ml (0,06 Mol) Allylalkohol, rührt anschließend weitere 15 Minuten bei Raumtemperatur, gibt dann 4,62 g (0,03 Mol) 4-Amino-2,5-difluorbenzonitril zu, erwärmt für eine Stunde auf 100 °C, rührt dann die erkaltete Mischung in Eiswasser, extrahiert mehrfach mit Toluol, trocknet über Natriumsulfat und engt im Vakuum ein. Der Rückstand wird duch Chromatographie an Kieselgel (Laufmittel: Cyclohexan/Essigester 3:1) gereinigt.

Man erhält 2,5 g (43 % der Theorie) an 2-Allyloxy-4-amino-5-fluorbenzonitril als Öl.
$^1$H-NMR ($CDCl_3$/Tetramethylsilan):
$\delta$ = 4,55 (m,2H); 5,3 (dd,1H), 5,44 (dd,1H); 6,0 (m,1H); 6,27 (d,1H); 7,1 (d,1H) ppm.

Beispiel III-2

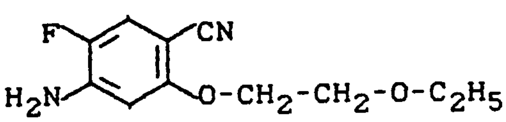

Zu 1,8 g (0,06 Mol) Natriumhydrid in 30 ml N-Methylpyrrolidon gibt man bei Raumtemperatur tropfenweise unter Rühren 5,82 ml (0,06 Mol) 2-Ethoxyethanol, rührt anschließend weitere 15 Minuten bei Raumtemperatur, gibt dann 4,62 g (0,03 Mol) 4-Amino-2,5-difluorbenzonitril zu, erwärmt für eine Stunde auf 100 °C, rührt dann die erkaltete Mischung in Eiswasser, extrahiert mehrfach mit Toluol, trocknet über Natriumsulfat und engt im Vakuum ein. Der Rückstand wird durch Chromatographie an Kieselgel (Laufmittel: Cyclohexan/Essigester 1:1) gereinigt.

Man erhält 3,5 g (52 % der Theorie) an 2-(2-Ethoxyethoxy)-4-amino-5-fluorbenzonitril als Öl.
$^1$H-NMR ($CDCl_3$/Tetramethylsilan):
$\delta$ = 1,22 (t,3H); 3,62 (q,2H); 3,8 (dd,2H); 4,1 (dd,4H); 6,32 (d,1H); 7,1 (d,1H) ppm.

Anwendungsbeispiele

In den folgenden Anwendungsbeispielen wurde die nachstehend aufgeführte Verbindung als Vergleichsubstanz eingesetzt:

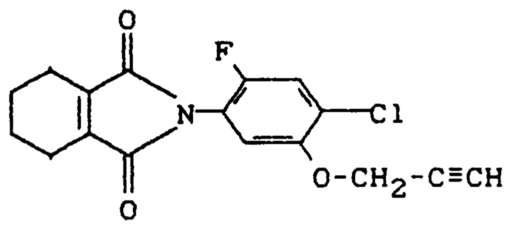

(A)

(bekannt aus EP-A 61 741 / Verbindung Nr. 10)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität gegenüber dem Stand der Technik zeigen in diesem Test z.B. die Verbindungen gemäß folgender Herstellungsbeispiele: 1, 16 und 17.

**Patentansprüche**

**1.** N-Aryl-Stickstoffheterocyclen der Formel (I),

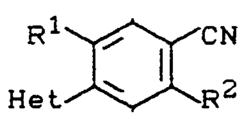

(I)

in welcher
Het für einen Heterocyclus der Formel

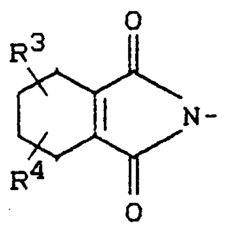 ; 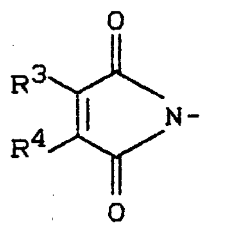 ; 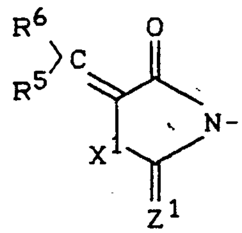

oder 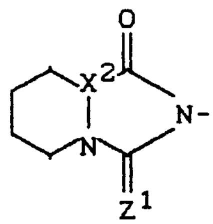

steht,

| | |
|---|---|
| $R^1$ | für Wasserstoff oder Halogen steht und |
| R2 | für Halogen, Hydroxy oder für einen Rest $-Z^2-R^8$ steht, wobei |
| $X^1$ | für eine $-CH_2$-Gruppe, für eine |

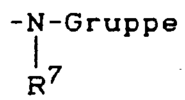

oder für eine

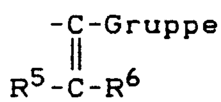

steht,

| | |
|---|---|
| $X^2$ | für Stickstoff oder eine CH-Gruppe steht, |
| $Z^1$ | für Sauerstoff oder Schwefel steht, |
| $Z^2$ | für Sauerstoff oder Schwefel steht, |
| $R^3$ und $R^4$ | unabhängig voneinander jeweils für Wasserstoff oder Alkyl stehen, |
| $R^5$ und $R^6$ | entweder unabhängig voneinander jeweils für Wasserstoff oder Alkyl stehen oder gemeinsam für einen zweifach verknüpften Alkandiylrest stehen, |
| $R^7$ | für Wasserstoff, Alkyl oder für gegebenenfalls substituiertes Aryl steht und |
| $R^8$ | für jeweils gegebenenfalls substituiertes Alkyl, Alkenyl, Alkinyl oder Cycloalkyl steht, |

ausgenommen die Verbindungen in denen Het für einen Heterocyclus der Formel

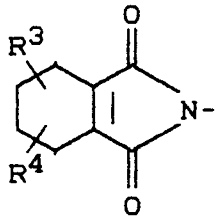

oder

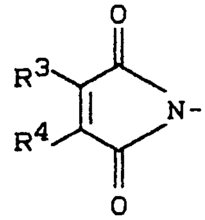

steht

R[1] für Wasserstoff oder Halogen steht,

R[2] für einen Rest $-Z^2-R^8$ steht, wobei

Z[2] für Sauerstoff steht und

R[8] für Wasserstoff oder für jeweils gegebenenfalls substituiertes Alkyl, Alkenyl, Alkinyl oder Cycloalkyl steht und

$R^3$ und $R^4$ unabhängig voneinander jeweils für Wasserstoff oder Alkyl stehen,

wobei die folgenden Verbindungen vom Disclaimer ausgenommen sind:

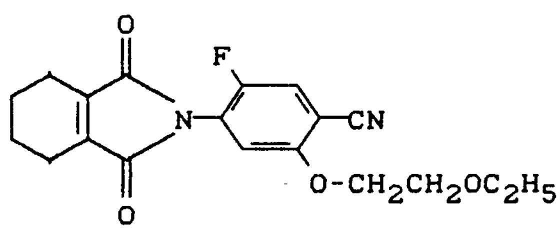

,

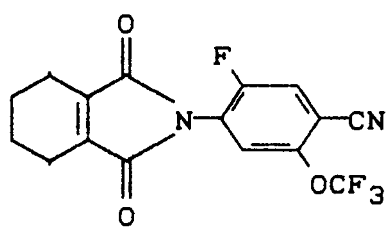

,

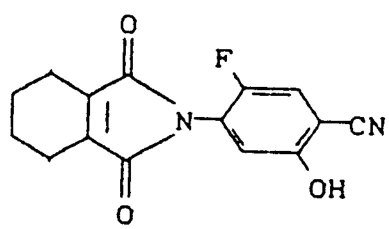

,

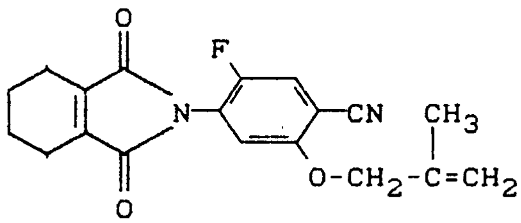

,

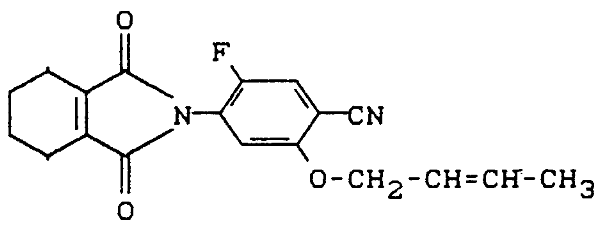

,

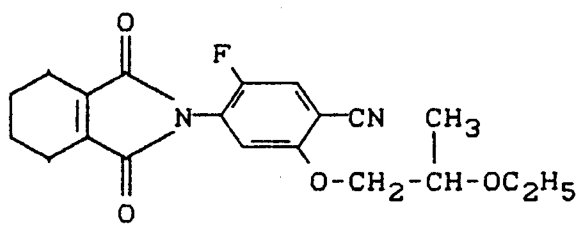

,

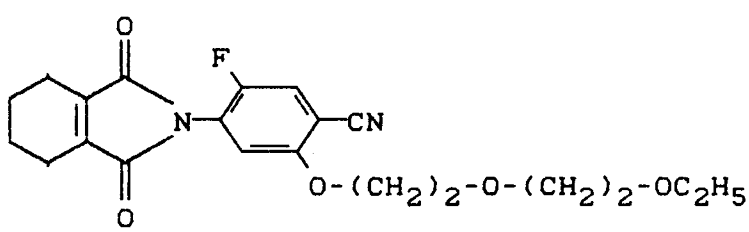

und

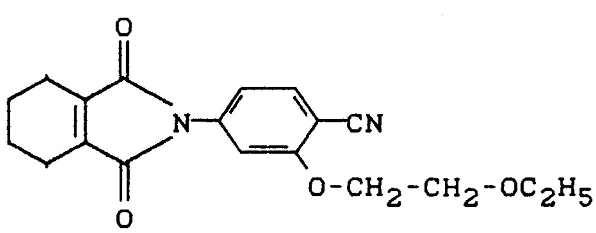

.

2. N-Aryl-Stickstoffheterocyclen der Formel (I) gemäß Anspruch 1, in welcher

Het für einen Heterocyclus der Formel

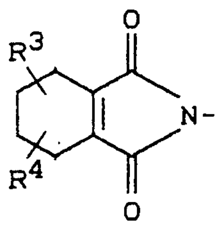 ; 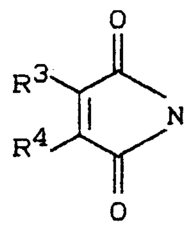 ; 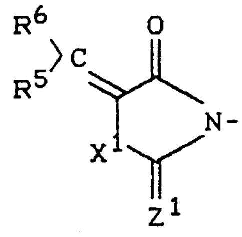

oder 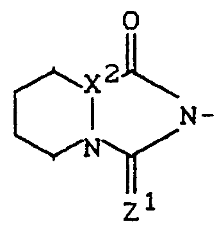

steht

R¹ für Wasserstoff, Fluor, Chlor oder Brom steht und

$R^2$ für Fluor, Chlor, Brom, Hydroxy oder für einen Rest $-Z^2-R^8$ steht, wobei

$X^1$ für eine $-CH_2$-Gruppe, für eine

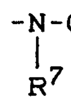-Gruppe

oder für eine

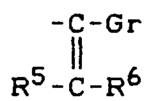-Gruppe

steht,

$X^2$ für Stickstoff oder eine CH-Gruppe steht,

$Z^1$ für Sauerstoff oder Schwefel steht,

$Z^2$ für Sauerstoff oder Schwefel steht,

$R^3$ und $R^4$ unabhängig voneinander jeweils für Wasserstoff oder für geradkettiges oder verzweigtes Alkyl mit 1 bis 4 Kohlenstoffatomen stehen,

$R^5$ und $R^6$ entweder unabhängig voneinander jeweils für Wasserstoff oder Für geradkettiges oder verzweigtes Alkyl mit 1 bis 4 Kohlenstoffatomen stehen oder gemeinsam für einen zweifachverknüpften Alkandiylrest mit 2 bis 7 Kohlenstoffatomen stehen

$R^7$ für Wasserstoff, für geradkettiges oder verzweigtes Alkyl mit 1 bis 4 Kohlenstoffatomen oder für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

$R^8$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl,

Alkylthioalkyl, Halogenalkoxyalkyl, Alkoxyalkoxyalkyl, Bis-(Alkoxy)alkyl, Bis-(Alkylthio)-alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils infrage kommen: Halogen sowie jeweils geradkettiges oder verzweigtes Alkyl oder Alkoxy mit jeweils 1 bis 4 Kohlenstoffatomen, $R^8$ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen Alkylteilen steht und für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

ausgenommen die Verbindungen in denen Het für einen Heterocyclus der Formel

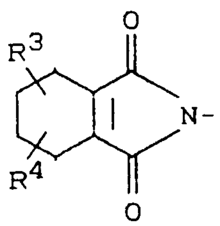

oder

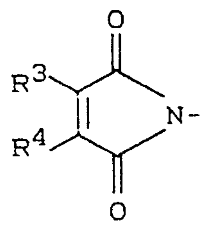

steht

- $R^1$ für Wasserstoff oder Halogen steht,
- $R^2$ für einen Rest $-Z^2-R^8$ steht, wobei
- $Z^2$ für Sauerstoff steht und
- $R^8$ für Wasserstoff oder für jeweils gegebenenfalls substituiertes Alkyl, Alkenyl, Alkinyl oder Cycloalkyl steht und
  $R^3$ und $R^4$ unabhängig voneinander jeweils für Wasserstoff oder Alkyl stehen,

wobei die folgenden Verbindungen vom Disclaimer ausgenommen sind:

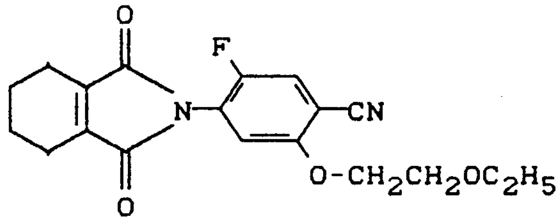

,

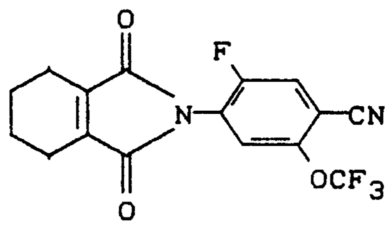

,

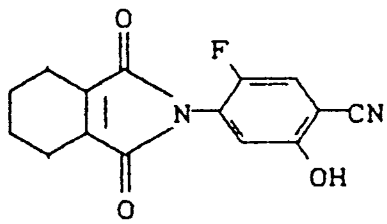

,

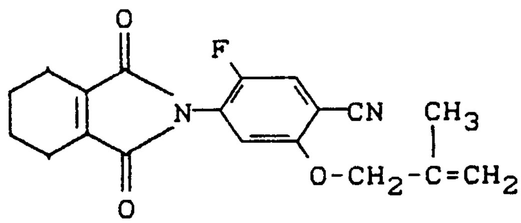 ,

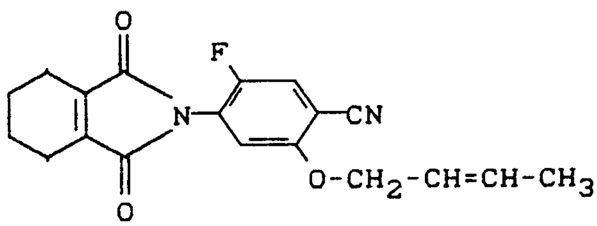 ,

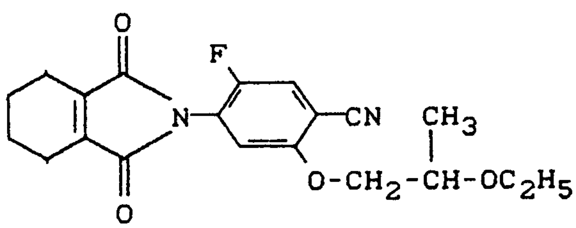 ,

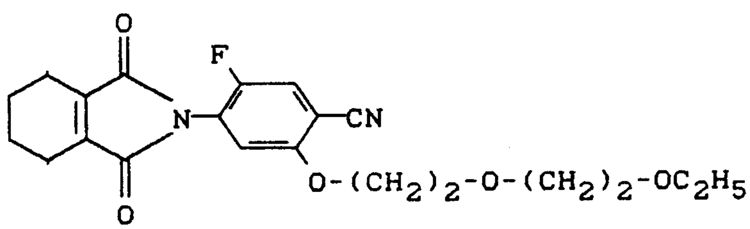

und

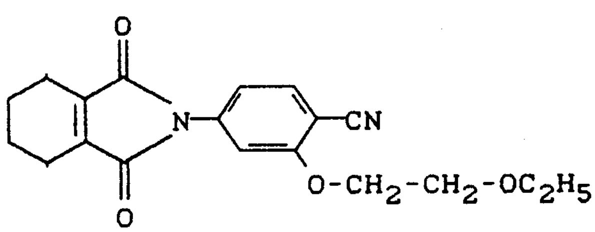 .

**3.** N-Aryl-Stickstoffheterocyclen der Formel (I) gemäß Anspruch 1, in welcher

Het für einen Heterocyclus der Formel

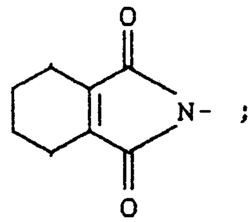 ; 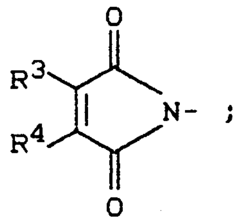 ; 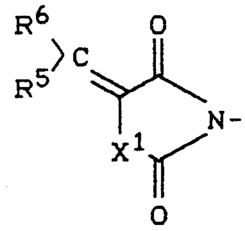

oder 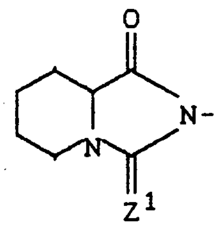

steht,

| | |
|---|---|
| $R^1$ | für Wasserstoff, Fluor oder Chlor steht und |
| $R^2$ | für Fluor, Chlor oder für einen Rest $-Z^2-R^8$ steht, |
| $X^1$ | für eine $-CH_2$-Gruppe, für eine |

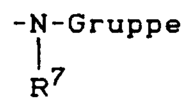

oder für eine

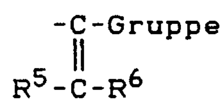

steht,

| | |
|---|---|
| $Z^1$ | für Sauerstoff oder Schwefel steht, |
| $Z^2$ | für Sauerstoff oder Schwefel steht, |
| $R^3$ und $R^4$ | unabhängig voneinander jeweils für Wasserstoff, Methyl, Ethyl, n- oder i-Propyl stehen, |
| $R^5$ und $R^6$ | entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen, |
| $R^7$ | für Wasserstoff, Methyl, Ethyl, n- oder i-Propyl oder für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio und |
| $R^8$ | für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopen- |

tyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

ausgenommen die Verbindungen in denen Het für einen Heterocyclus der Formel

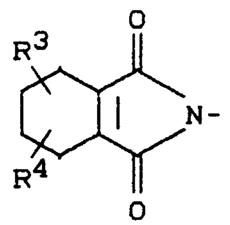

oder

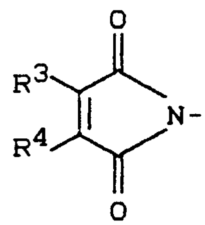

steht

- $R^1$ für Wasserstoff oder Halogen steht,
- $R^2$ für einen Rest $-Z^2-R^8$ steht, wobei
- $Z^2$ für Sauerstoff steht und
- $R^8$ für Wasserstoff oder für jeweils gegebenenfalls substituiertes Alkyl, Alkenyl, Alkinyl oder Cycloalkyl steht und

$R^3$ und $R^4$ unabhängig voneinander jeweils für Wasserstoff oder Alkyl stehen,

wobei die folgenden Verbindungen vom Disclaimer ausgenommen sind:

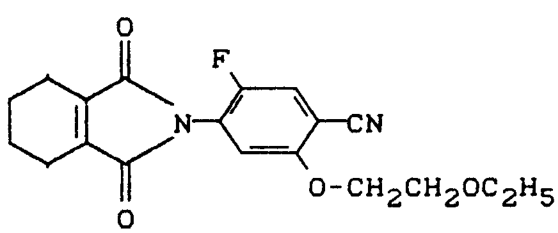

,

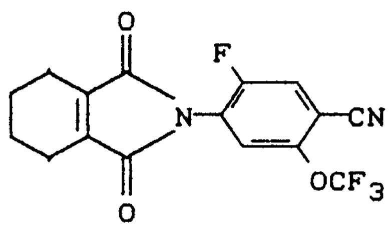

,

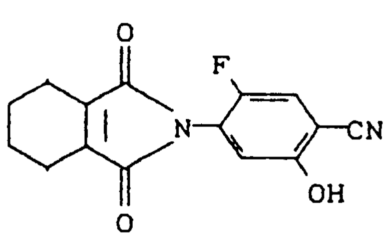

,

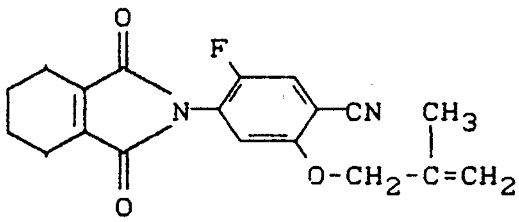

,

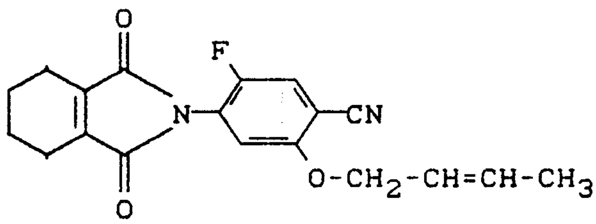

,

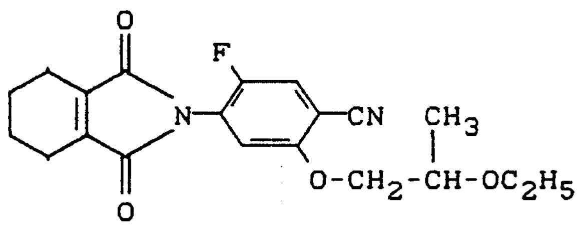

,

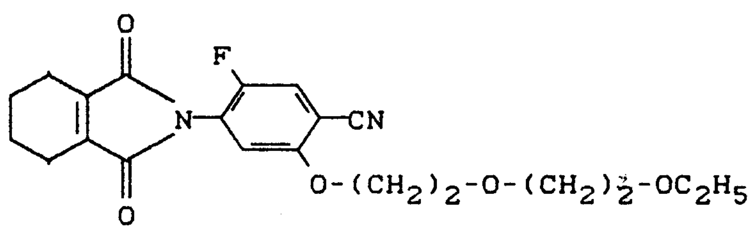

und

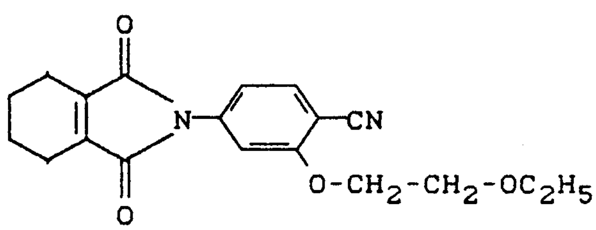

.

**4.** Verfahren zur Herstellung von N-Aryl-Stickstoffheterocyclen der Formel (I),

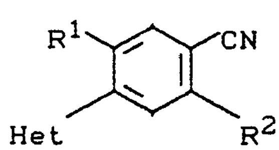

(I)

in welcher

Het für einen Heterocyclus der Formel

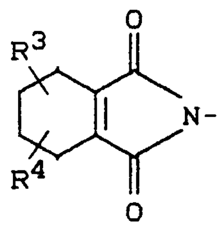 ; 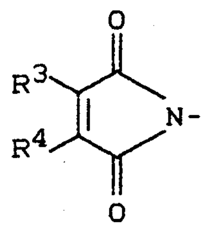 ; 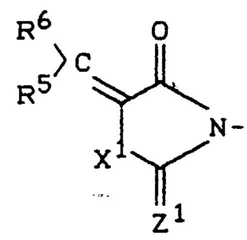

oder 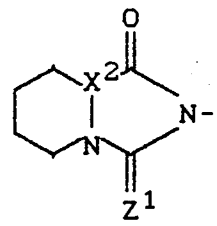

steht,

R¹ für Wasserstoff oder Halogen steht und

$R^2$ für Halogen, Hydroxy oder für einen Rest $-Z^2-R^8$ steht, wobei

$X^1$ für eine $-CH_2$-Gruppe, für

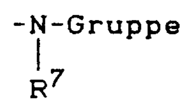

oder für eine

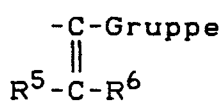

steht,

$X^2$ für Stickstoff oder eine CH-Gruppe steht,

$Z^1$ für Sauerstoff oder Schwefel steht,

$Z^2$ für Sauerstoff oder Schwefel steht,

$R^3$ und $R^4$ unabhängig voneinander jeweils für Wasserstoff oder Alkyl stehen,

$R^5$ und $R^6$ entweder unabhängig voneinander jeweils für Wasserstoff oder Alkyl stehen oder gemeinsam für einen zweifach verknüpften Alkandiylrest stehen,

$R^7$ für Wasserstoff, Alkyl oder für gegebenenfalls substituiertes Aryl steht und

$R^8$ für jeweils gegebenenfalls substituiertes Alkyl, Alkenyl, Alkinyl oder Cycloalkyl steht,

ausgenommen die Verbindungen in denen Het für einen Heterocyclus der Formel

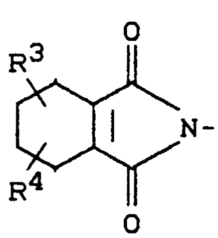 oder 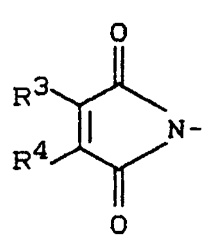

steht

$R^1$ für Wasserstoff oder Halogen steht,

$R^2$ für einen Rest $-Z^2-R^8$ steht, wobei

$Z^2$ für Sauerstoff steht und

$R^8$ für Wasserstoff oder für jeweils gegebenenfalls substituiertes Alkyl, Alkenyl, Alkinyl oder Cycloalkyl steht und

$R^3$ und $R^4$ jeweils für Alkyl stehen, wobei die folgenden Verbindungen vom Disclaimer ausgenommen sind,

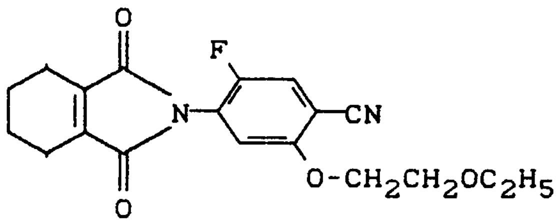

,

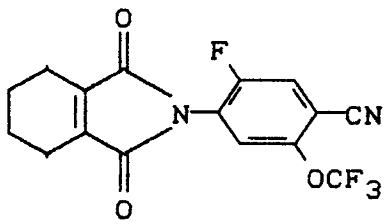

,

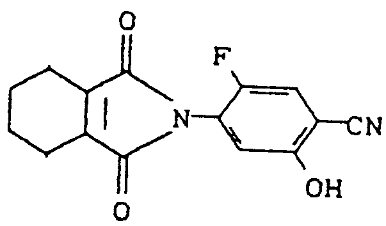

,

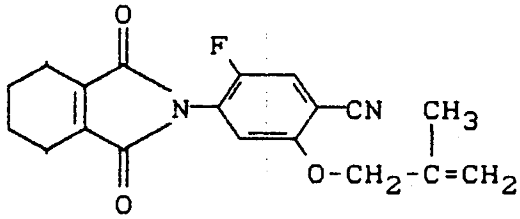,

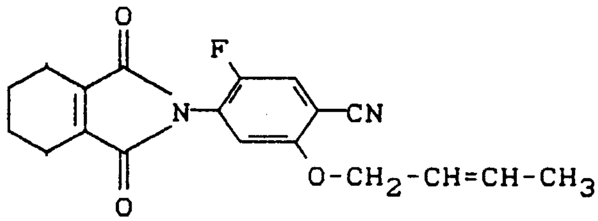,

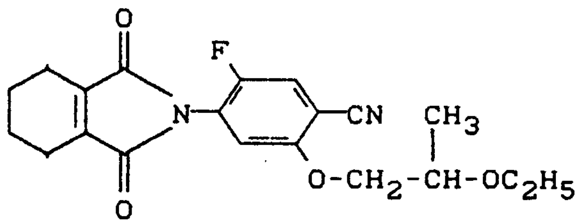,

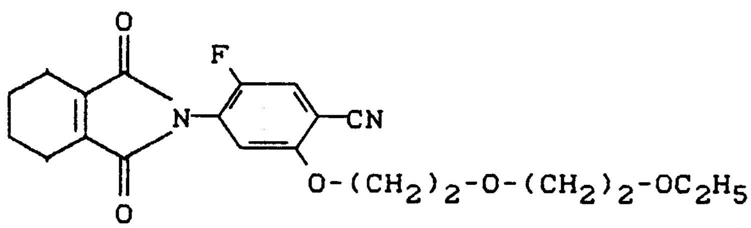

und

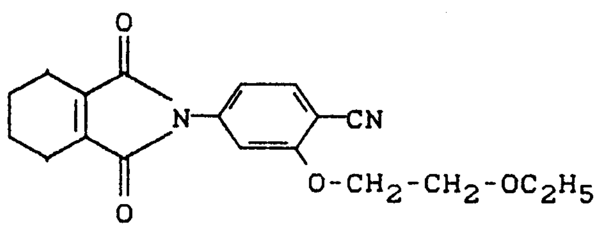,

dadurch gekennzeichnet, daß man

(a) N-Aryl-Stickstoffheterocyclen der Formel (Ia),

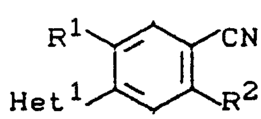

(Ia)

in welcher

Het[1] für einen Heterocyclus der Formel

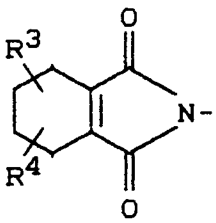 ; 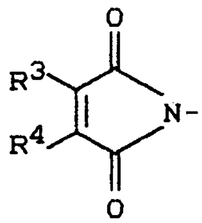 ; 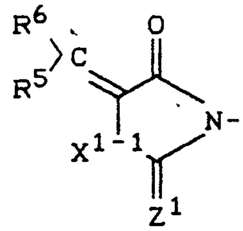 ;

steht, wobei

$X^{1-1}$ für eine $-CH_2$-Gruppe oder für eine

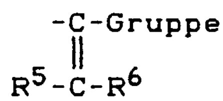

steht und

$R^1$, $R^2$, $R^3$, $R^4$, $R^5$ und $R^6$ die oben angegebene Bedeutung haben,

erhält, wenn man Anhydride der Formel (II),

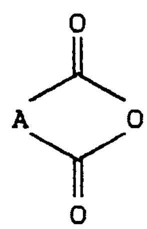 (II)

in welcher

A für einen Rest der Formel

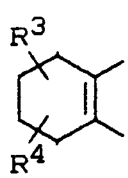 ; 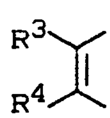 oder 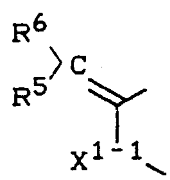

steht,

wobei

$X^{1-1}$, $R^3$, $R^4$, $R^5$ und $R^6$ die oben angegebene Bedeutung haben,

mit Aminen der Formel (III),

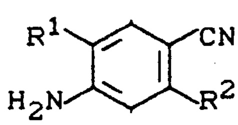 (III)

in welcher

$R^1$ und $R^2$ die oben angegebene Bedeutung hat,

gegebenenfalls in Gegenwart eines Verdünnungsmittels und gegebenenfalls in Gegenwart eines Reaktionshilfsmittels umsetzt; oder daß man

(b) N-Aryl-Stickstoffheterocyclen der Formel (Ib),

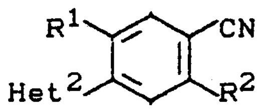

(Ib)

in welcher

Het² für einen Heterocyclus der Formel

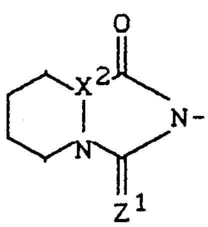

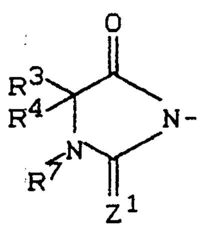

oder

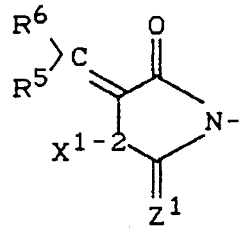

steht, wobei

$X^{1-2}$ für eine

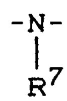 Gruppe

steht und

$R^1$, $R^2$, $R^3$, $R^4$, $R^5$, $R^6$, $R^7$, $X^2$ und $Z^1$ die oben angegebene Bedeutung haben,

erhält, wenn man Carbonsäureester der Formel (IV),

$R^9$-COOR$^{10}$ (IV)

in welcher

$R^9$ für einen Rest der Formel

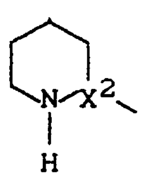

;

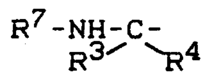

oder

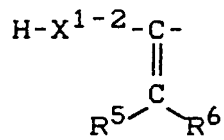

steht,

und

$R^{10}$ für Alkyl steht, wobei

$X^{1-2}$, $X^2$, $R^3$, $R^4$, $R^5$, $R^6$ und $R^7$ die oben angegebene Bedeutung haben,

oder deren Säureadditionssalze mit Iso(thio)cyanaten der Formel (V),

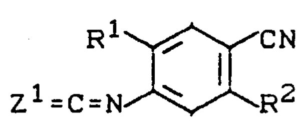

(V)

in welcher

$R^1$, $R^2$ und $Z^1$ die oben angegebene Bedeutung haben,

gegebenenfalls in Gegenwart eines Verdünnungsmittels und gegebenenfalls in Gegenwart eines Reaktionshilfsmittels umsetzt; oder daß man

(c) N-Aryl-Stickstoffheterocyclen der Formel (Ic),

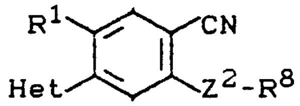

(Ic)

in welcher

$R^1$, $R^8$, $Z^2$ und Het die oben angegebene Bedeutung haben,

erhält, wenn man die nach Verfahren (a) und (b) erhältliche N-Arylstickstoffheterocyclen der Formel (If)

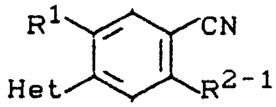

(If)

in welcher

$R^{2-1}$ für Halogen steht und

Het und $R^1$ die oben angegebene Bedeutung haben,

mit Alkoholen oder Thiolen der Formel (VI),

$R^8$-$Z^2$H (VI)

in welcher

$R^8$ und $Z^2$ die oben angegebene Bedeutung haben,

gegebenenfalls in Gegenwart eines Verdünnungsmittels sowie in Gegenwart eines Reaktionshilfsmittels umsetzt; oder daß man

(d) N-Aryl-Stickstoffheterocyclen der Formel (Id),

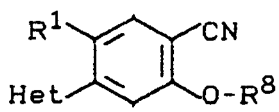

(Id)

in welcher

$R^1$, $R^8$ und Het die oben angegebene Bedeutung haben,

erhält, wenn man N-Aryl-Stickstoffheterocyclen der Formel (Ie),

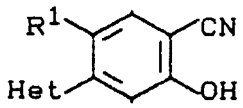

(Ie)

in welcher

$R^1$ und Het die oben angegebene Bedeutung haben,

mit Alkylierungsmitteln der Formel (VII),

$R^8$-E (VII)

in welcher

E für eine elektronenanziehende Abgangsgruppe steht und

$R^8$ die oben angegebene Bedeutung hat,

gegebenenfalls in Gegenwart eines Verdünnungsmittels und gegebenenfalls in Gegenwart eines Reaktionshilfsmittels umsetzt; oder daß man

e) N-Aryl-Stickstoffheterocyclen der Formel (Ie)

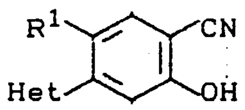

(Ie)

in welcher

$R^1$ und Het die oben angegebene Bedeutung haben, erhält, wenn man

e-α) N-Aryl-Stickstoffheterocyclen der Formel (Ig)

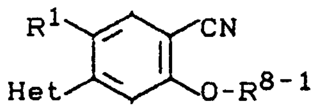

(Ig)

in welcher

$R^{8-1}$ für Allyl oder Benzyl steht und

$R^1$ und Het die oben angegebene Bedeutung haben,

mit Reduktionsmitteln, gegebenenfalls in Gegenwart eines Katalysators und gegebenenfalls in Gegenwart eines Verdünnungsmittels umsetzt, oder daß man

e-β) Amine der Formel (IIIa),

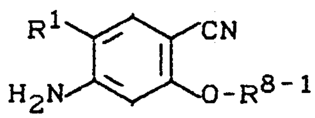

(IIIa)

in welcher

$R^1$ und $R^{8-1}$ die oben angegebene Bedeutung haben,

mit Reduktionsmitteln, gegebenenfalls in Gegenwart eines Katalysators und gegebenenfalls in Gegenwart eines Verdünnungsmittels und gegebenenfalls in Gegenwart eines Reaktionshilfsmittels umsetzt und die so erhältlichen Aminophenole der Formel (VIII),

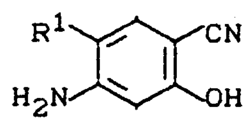

(VIII)

in welcher

$R^1$ die oben angegebene Bedeutung hat,

mit Anhydriden der Formel (II),

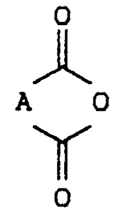

(II)

in welcher

A für einen Rest der Formel

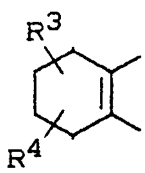

;

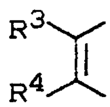

oder

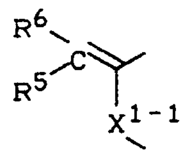

steht, wobei

$X^{1-1}$ für eine $-CH_2$-Gruppe oder für eine

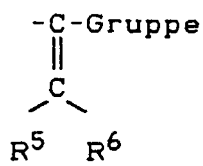

steht und

$R^3$, $R^4$, $R^5$ und $R^6$ die oben angegebene Bedeutung haben,

gegebenenfalls in Gegenwart eines Verdünnungsmittels und gegebenenfalls in Gegenwart eines Reaktionshilfsmittels umsetzt.

**5.** Herbizide Mittel, gekennzeichnet durch einen Gehalt an mindestens einem N-Aryl-Stickstoffheterocyclus der Formel (I) gemäß den Ansprüchen 1 bis 4.

**6.** Verfahren zur Bekämpfung von Unkräutern, dadurch gekennzeichnet, daß man N-Aryl-Stickstoffheterocyclen der Formel (I) gemäß den Ansprüchen 1 bis 4 auf die Unkräuter und/oder ihren Lebensraum einwirken läßt.

**7.** Verwendung von N-Aryl-Stickstoffheterocyclen der Formel (I) gemäß den Ansprüchen 1 bis 4 zur Bekämpfung von Unkräutern.

**8.** Verfahren zur Herstellung von herbiziden Mitteln, dadurch gekennzeichnet, daß man N-Aryl-Stickstoffheterocyclen der Formel (I) gemäß den Ansprüchen 1 bis 4 mit Streckmitteln und/oder oberflächenaktiven Substanzen vermischt.

**9.** Die Verbindungen der Formel:

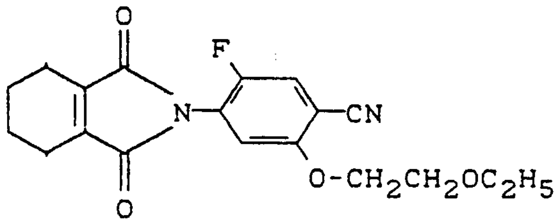

,

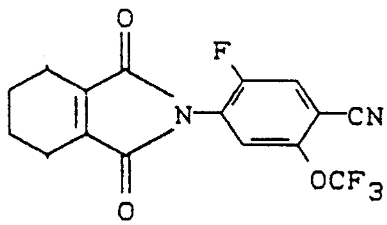

,

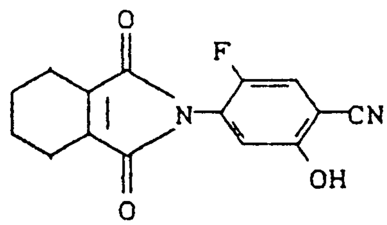

,

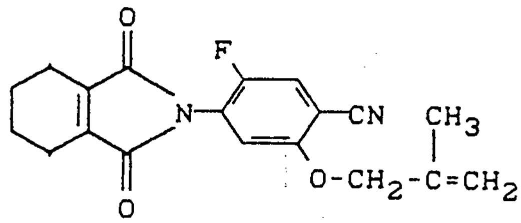

,

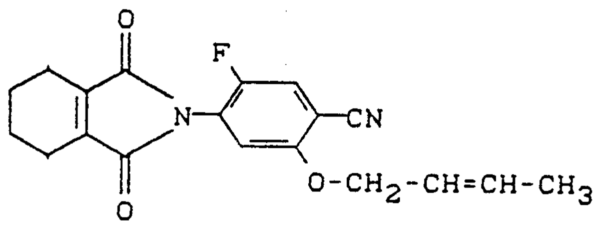

,

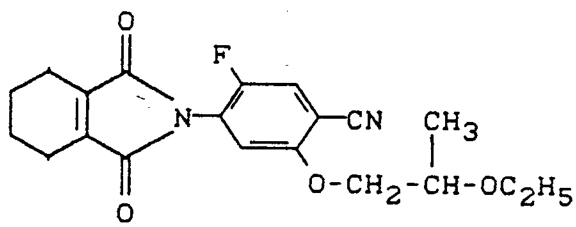

,

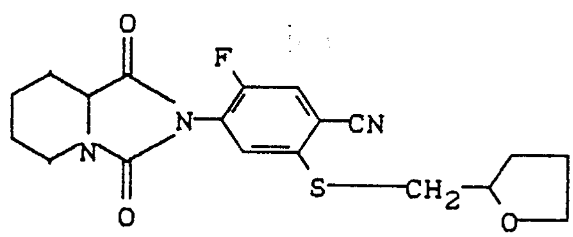

,

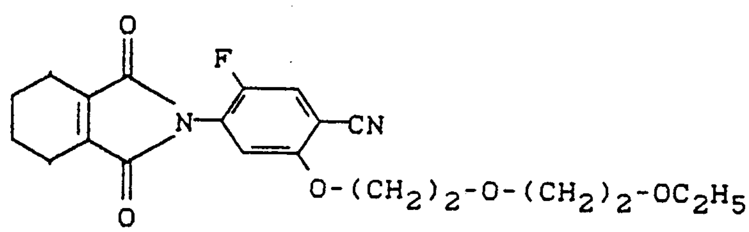

und

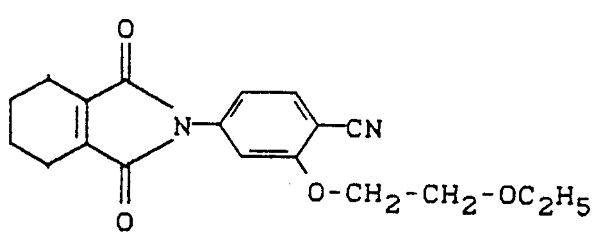

.

## Claims

**1.** N-Aryl nitrogen heterocycles of the formula (I)

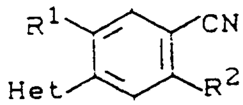

(I)

in which

Het represents a heterocycle of the formula

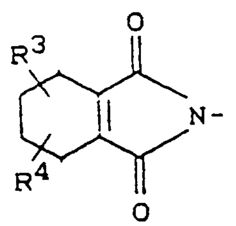 ; 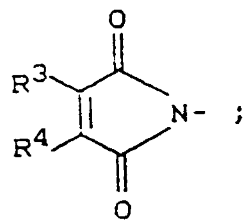 ; 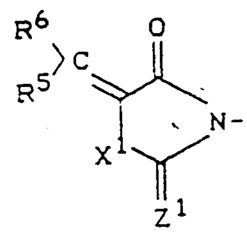

or

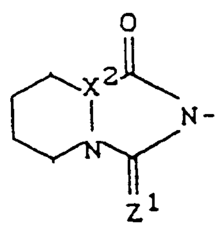 ,

$R^1$ represents hydrogen or halogen and

$R^2$ represents halogen, hydroxyl or represents a radical $-Z^2-R^8$, where

$X^1$ represents a $-CH_2$-group, represents an

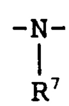

group or represents a

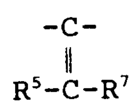

group,

$X^2$ represents nitrogen or a CH group,

$Z^1$ represents oxygen or sulphur,

$Z^2$ represents oxygen or sulphur,

$R^3$ and $R^4$ independently of one another in each case represent hydrogen or alkyl,

$R^5$ and $R^6$ either independently of one another in each case represent hydrogen or alkyl or together represent a double-linked alkanediyl radical,

$R^7$ represents hydrogen, alkyl or represents optionally substituted aryl and

$R^8$ represents in each case optionally substituted alkyl, alkenyl, alkinyl or cycloalkyl,

except for the compounds in which Het represents a heterocycle of the formula

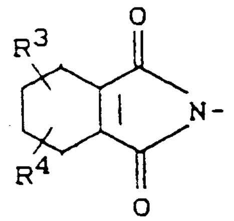

or

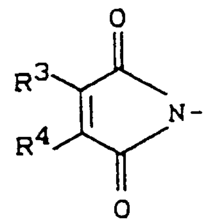

in which

| | |
|---|---|
| $R^1$ | represents hydrogen or halogen, |
| $R^2$ | represents a radical $-Z^2-R^8$, where |
| $Z^2$ | represents oxygen and |
| $R^8$ | represents hydrogen or represents in each case optionally substituted alkyl, alkenyl, alkinyl or cycloalkyl and |
| $R^3$ and $R^4$ | independently of one another in each case represent hydrogen or alkyl, |

the following compounds of the disclaimer being excluded:

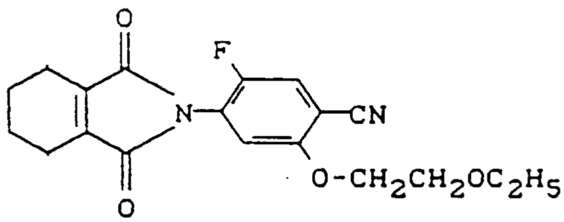

,

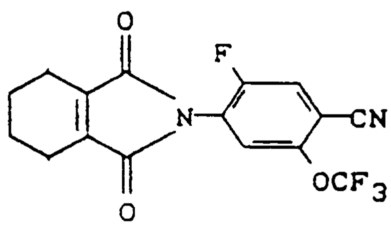

,

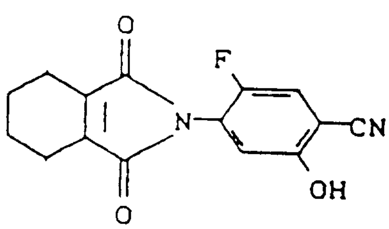

,

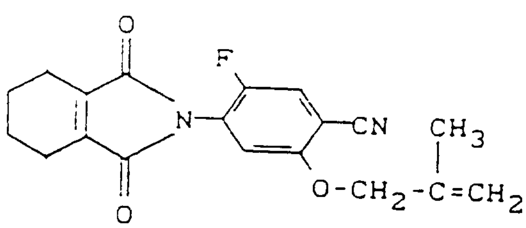

,

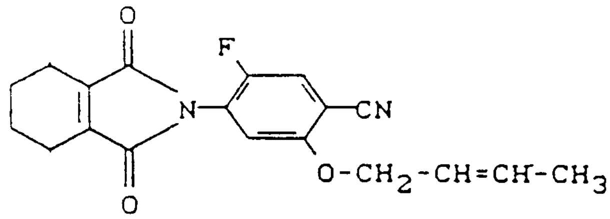

,

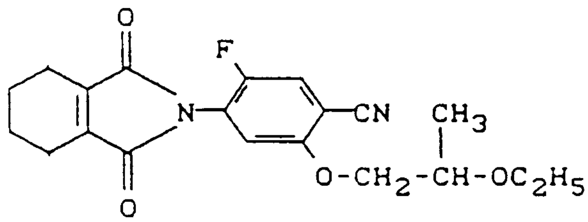

,

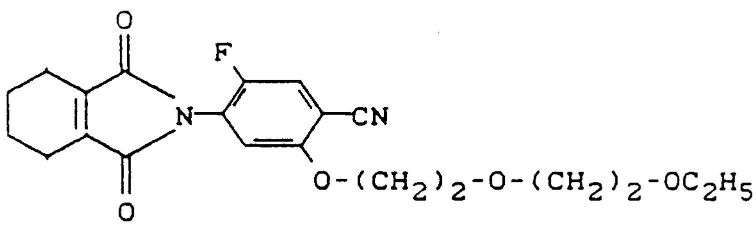

and

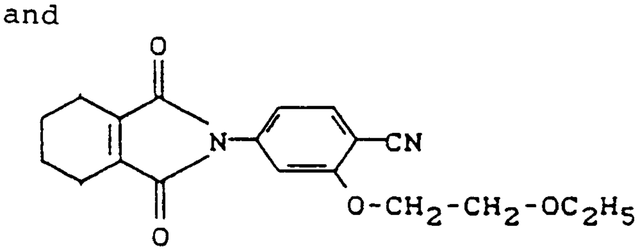

.

**2.** N-Aryl nitrogen heterocycles of the formula (I) according to Claim 1, in which
Het represents a heterocycle of the formula

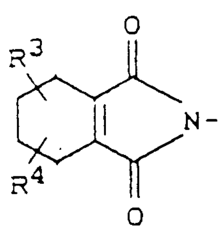 ; 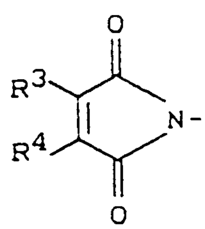 ; 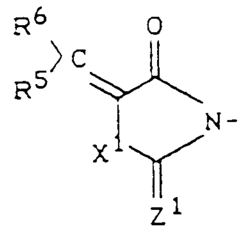

or 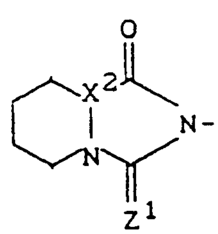 ,

| | |
|---|---|
| $R^1$ | represents hydrogen, fluorine, chlorine or bromine and |
| $R^2$ | represents fluorine, chlorine, bromine, hydroxyl or represents a radical $-Z^2-R^8$, where |
| $X^1$ | represents a $-CH_2-$ group, represents an 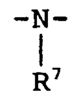 group or represents a 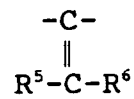 group, |
| $X^2$ | represents nitrogen or a CH group, |
| $Z^1$ | represents oxygen or sulphur, |
| $Z^2$ | represents oxygen or sulphur, |
| $R^3$ and $R^4$ | independently of one another in each case represent hydrogen or represent straight-chain or branched alkyl having 1 to 4 carbon atoms, |
| $R^5$ and $R^6$ | either independently of one another in each case represent hydrogen or represent straight-chain or branched alkyl having 1 to 4 carbon atoms, or together represent a double-linked alkanediyl radical having 2 to 7 carbon atoms, |
| $R^7$ | represents hydrogen, represents straight-chain or branched alkyl having 1 to 4 carbon atoms or represents phenyl which is optionally monosubstituted or polysubstituted by identical or different substituents, suitable substituents in each case being: halogen, cyano, nitro, in each case straight-chain or branched alkyl, alkoxy or alkylthio, each having 1 to 4 carbon atoms, and also in each case straight-chain or branched halogenoalkyl, halogenoalkoxy or halogenoalkylthio, each having 1 to 4 carbon atoms and 1 to 9 identical or different halogen atoms and |
| $R^8$ | represents in each case straight-chain or branched alkyl having 1 to 8 carbon atoms, alkenyl having 2 to 8 carbon atoms, alkinyl having 3 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 3 to 8 carbon atoms and 1 to 13 identical or different halogen atoms, cyanoalkyl, alkoxyalkyl, alkylthioalkyl, halogenoalkoxyalkyl, alkoxyalkoxyalkyl, bis-(alkoxy)alkyl, bis-(alkylthio)alkyl, alkylcarbonylalkyl, alkoxycarbonylalkyl or alkoxyalkoxycarbonylalkyl, each having 1 to 8 carbon atoms in the individual alkyl moieties and if appropriate 1 to 9 identical or different halogen atoms, or represents cycloalkyl, cycloalkyloxycarbonylalkyl or cycloalkylalkyl, each of which has 3 to 7 carbon atoms in the cycloalkyl moiety and if appropriate 1 to 4 carbon atoms in the straight-chain or branched alkyl moiety and each of which is optionally monosubstituted or polysubstituted by identical or different substituents, suitable substituents in the cycloalkyl moiety in each case being: halogen as well as in each case straight-chain or branched alkyl or alkoxy, each having 1 to 4 carbon atoms, $R^8$ furthermore also represents oxetanylalkyl, tetrahydrofuranylalkyl or tetrahydropyranylalkyl, each of which has 1 to 3 carbon atoms in the respective alkyl moieties and each of which is optionally substituted by alkyl having 1 to 4 carbon atoms, and represents aralkyl which has 6 to 10 carbon atoms in the aryl moiety and 1 to 4 carbon atoms in the straight-chain or branched alkyl moiety and is optionally monosubstituted or polysubstituted by identical or different substituents, possible aryl substituents being: halogen, cyano, nitro, in each case straight-chain or branched alkyl, alkoxy, alkylthio or alkoxycarbonyl, each having 1 to 4 carbon atoms, or in each case straight-chain or branched halogenoalkyl, halogenoalkoxy or halogenoalkylthio, each having 1 to 4 carbon atoms and 1 to 9 identical or different halogen atoms, |

except for the compounds in which Het represents a heterocycle of the formula

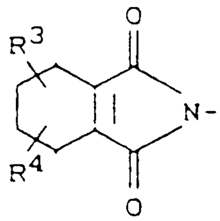

or

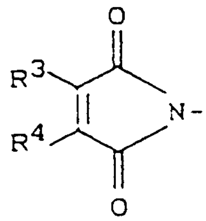

in which

| | |
|---|---|
| $R^1$ | represents hydrogen or halogen, |
| $R^2$ | represents a radical $-Z^2-R^8$, where |
| $Z^2$ | represents oxygen and |
| $R^8$ | represents hydrogen or represents in each case optionally substituted alkyl, alkenyl, alkinyl or cycloalkyl and |
| $R^3$ and $R^4$ | independently of one another in each case represent hydrogen or alkyl, |

the following compounds of the disclaimer being excluded:

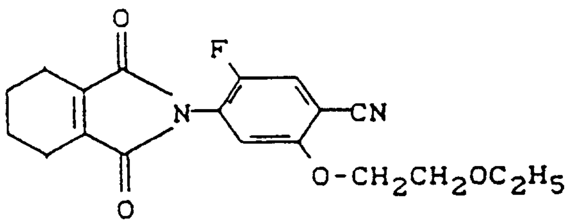

,

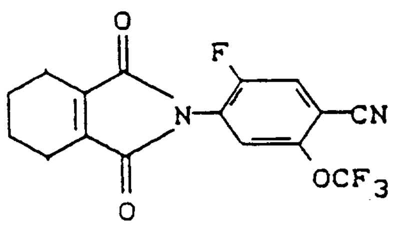

,

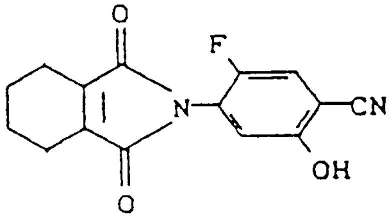

,

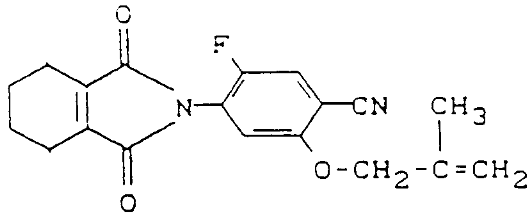

,

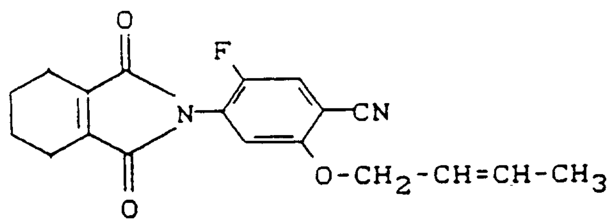

,

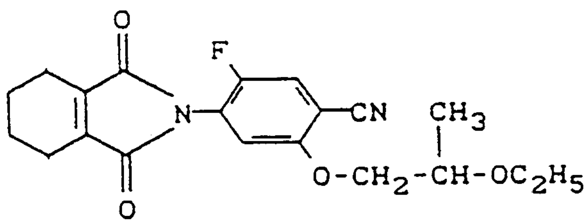

,

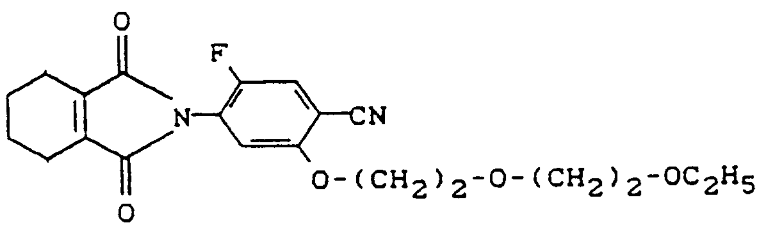

and

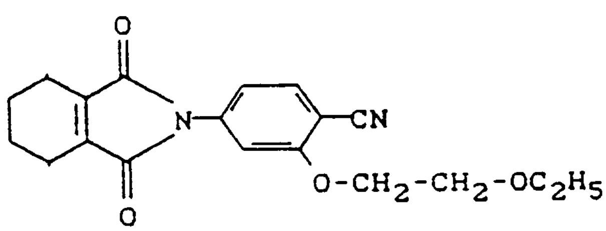

.

**3.** N-Aryl nitrogen heterocycles of the formula (I) according to Claim 1, in which

Het represents a heterocycle of the formula

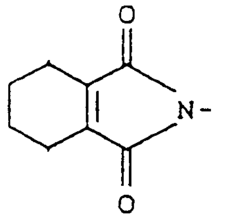 ; 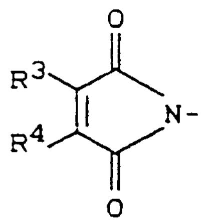 ; 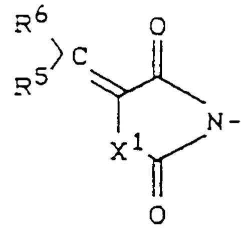

or 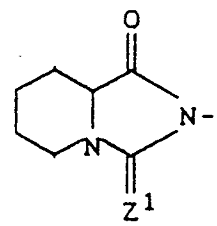 ,

R¹ represents hydrogen, fluorine or chlorine and

$R^2$ represents fluorine, chlorine or represents a radical $-Z^2-R^8$,

$X^1$ represents a $-CH_2-$ group, represents an

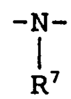

group or represents a

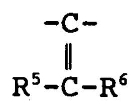

group,

$Z^1$ represents oxygen or sulphur,

$Z^2$ represents oxygen or sulphur,

$R^3$ and $R^4$ independently of one another in each case represent hydrogen, methyl, ethyl, n- or i-propyl,

$R^5$ and $R^6$ either independently of one another in each case represent hydrogen, methyl, ethyl, n- or i-propyl, or together represent a double-linked alkanediyl radical having 2 to 5 carbon atoms,

$R^7$ represents hydrogen, methyl, ethyl, n- or i-propyl, or represents phenyl which is optionally monosubstituted to trisubstituted by identical or different substituents, suitable substituents being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy or trifluoromethylthio and

$R^8$ represents methyl, ethyl, n- or i-propyl, n-, i-, s- or t-butyl, represents allyl, propargyl, represents in each case straight-chain or branched pentyl, hexyl, butenyl, pentenyl, hexenyl, butinyl, pentinyl or hexinyl, furthermore represents in each case straight-chain or branched halogenoalkyl having 1 to 4 carbon atoms or halogenoalkenyl having 3 to 5 carbon atoms and in each case 1 to 8 identical or different halogen atoms, or represents in each case straight-chain or branched cyanoalkyl, alkoxyalkyl, alkylthioalkyl, halogenoalkoxyalkyl, alkoxyalkoxyalkyl, alkylcarbonylalkyl, alkoxycarbonylalkyl or alkoxyalkoxycarbonylalkyl, each having 1 to 5 carbon atoms in the individual alkyl moieties, furthermore represents cyclopropylmethyl, cyclopentylmethyl, cyclohexylmethyl, cyclopropyloxycarbonylmethyl, cyclopentyloxycarbonylmethyl, cyclohexyloxycarbonylmethyl, cyclopropyl, cyclopentyl or cyclohexyl, in each case optionally monosubstituted to trisubstituted by identical or different substituents

from the series comprising methyl, methoxy, fluorine and chlorine, or represents oxetanylmethyl, oxetanylethyl, tetrahydrofuranylmethyl, tetrahydrofuranylethyl, tetrahydropyranylmethyl or tetrahydropyranylethyl, in each case optionally substituted by methyl or ethyl, or represents benzyl or phenylethyl, in each case optionally monosubstituted to trisubstituted by identical or different substituents, suitable substituents in each case being: fluorine, chlorine, bromine, cyano, nitro, methyl, ethyl, n- or i-propyl, n-, i-, s- or t-butyl, methoxy, ethoxy, n- or i-propoxy, methylthio, ethylthio, methoxycarbonyl, ethoxycarbonyl, trifluoromethyl, trifluoromethoxy or trifluoromethylthio,

except for the compounds in which Het represents a heterocycle of the formula

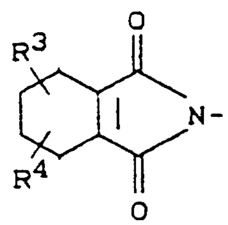

or

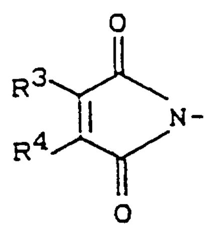

in which

- $R^1$ represents hydrogen or halogen,
- $R^2$ represents a radical $-Z^2-R^8$, where
- $Z^2$ represents oxygen and
- $R^8$ represents hydrogen or represents in each case optionally substituted alkyl, alkenyl, alkinyl or cycloalkyl and
- $R^3$ and $R^4$ independently of one another in each case represent hydrogen or alkyl,

the following compounds of the disclaimer being excluded:

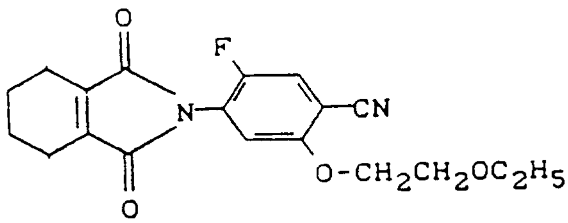

,

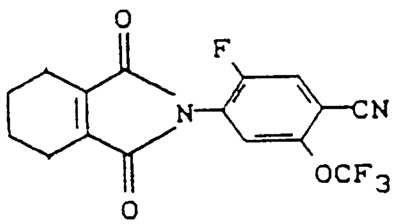

,

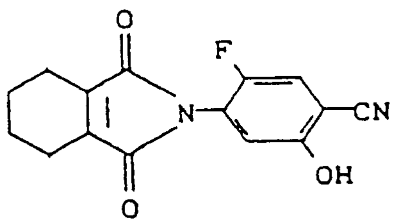

,

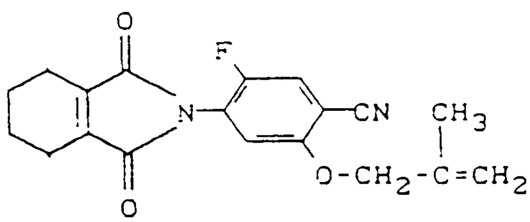

,

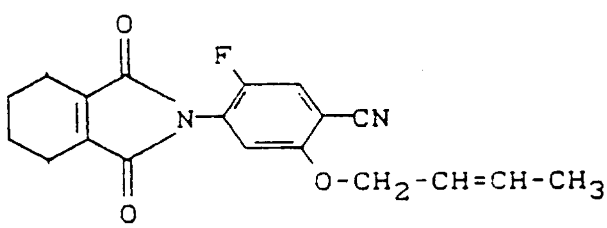

,

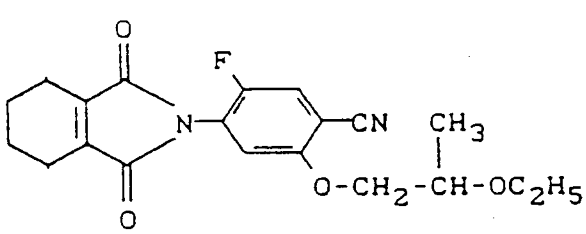

,

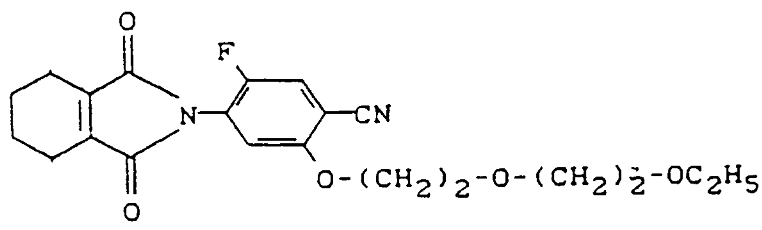

and

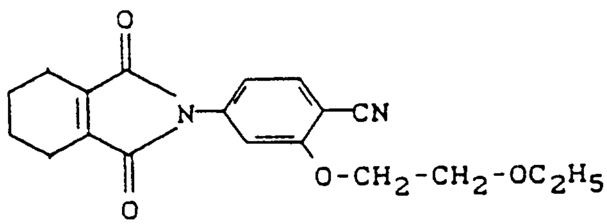

.

4. Process for the preparation of N-aryl nitrogen heterocycles of the formula (I)

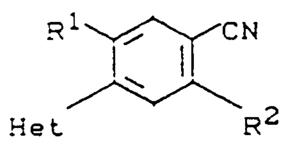

(I)

in which

Het represents a heterocycle of the formula

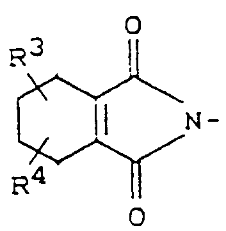

;

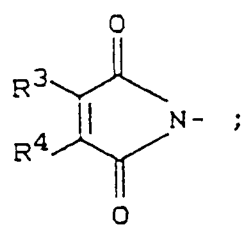

;

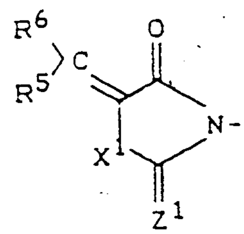

or

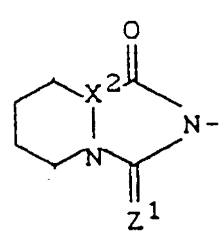

,

$R^1$ represents hydrogen or halogen and

$R^2$ represents halogen, hydroxyl or represents a radical $-Z^2-R^8$, where

$X^1$ represents a $-CH_2-$ group, represents an

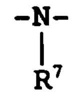

group or represents a

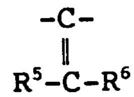

group,

- $X^2$ represents nitrogen or a CH group,
- $Z^1$ represents oxygen or sulphur,
- $Z^2$ represents oxygen or sulphur,
- $R^3$ and $R^4$ independently of one another in each case represent hydrogen or alkyl,
- $R^5$ and $R^6$ either independently of one another in each case represent hydrogen or alkyl or together represent a double-linked alkanediyl radical,
- $R^7$ represents hydrogen, alkyl or represents optionally substituted aryl and
- $R^8$ represents in each case optionally substituted alkyl, alkenyl, alkinyl or cycloalkyl,

except for the compounds in which Het represents a heterocycle of the formula

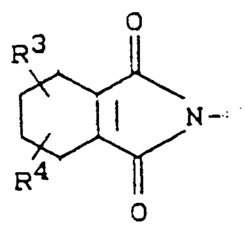
or
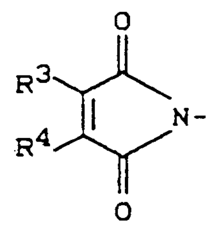

in which

- $R^1$ represents hydrogen or halogen,
- $R^2$ represents a radical $-Z^2-R^8$, where
- $Z^2$ represents oxygen and
- $R^8$ represents hydrogen or represents in each case optionally substituted alkyl, alkentyl, alkinyl or cycloalkyl and

$R^3$ and $R^4$ independently of one another in each case represent hydrogen or alkyl, the following compounds of the disclaimer being excluded;

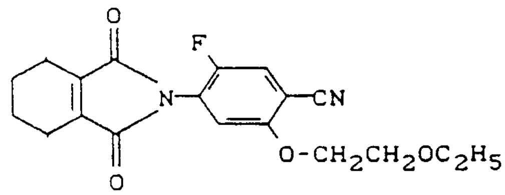

,

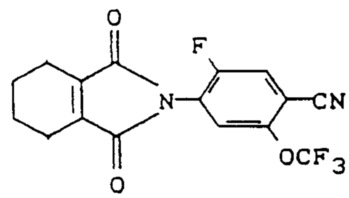

,

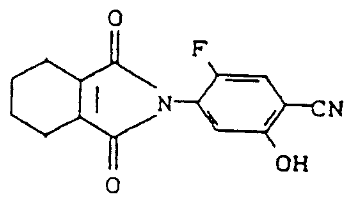

,

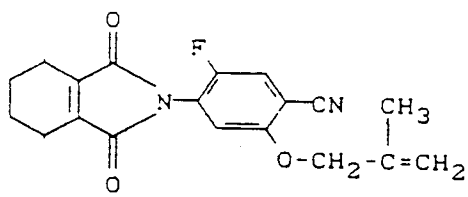

,

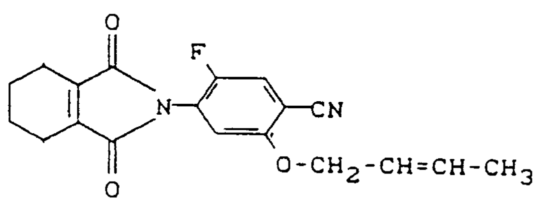

,

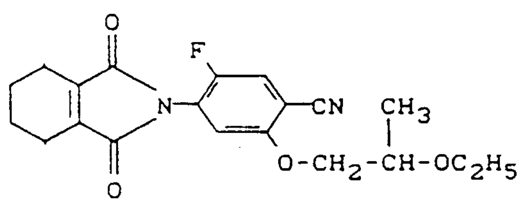

,

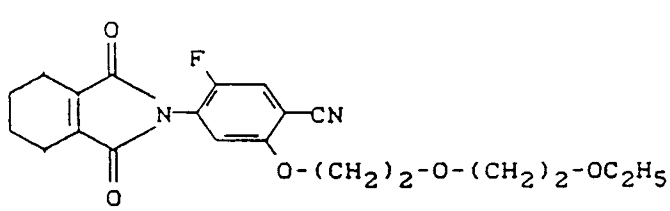

and 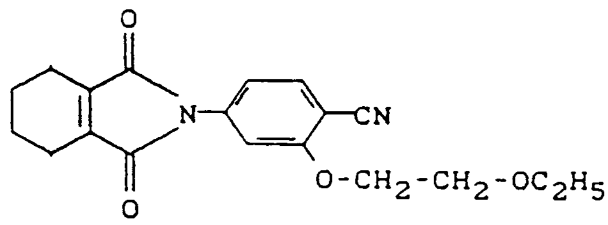 ,

characterized in that

(a) N-aryl nitrogen heterocycles of the formula (Ia)

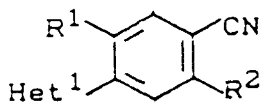 (Ia)

in which

Het[1] represents a heterocycle of the formula

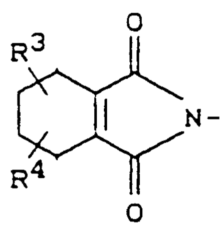 ; 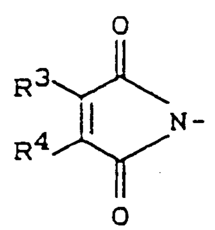 ; 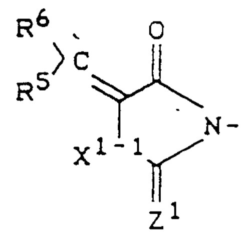 ;

where

$X^{1-1}$ represents a $-CH_2-$ group or represents a

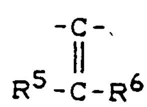

group and

$R^1$, $R^2$, $R^3$, $R^4$, $R^5$ and $R^6$ have the abovementioned meaning,

are obtained when anhydrides of the formula (II)

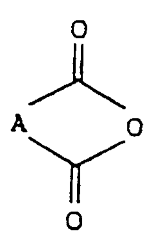 (II)

in which

A represents a radical of the formula

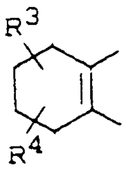 ; 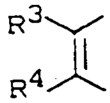 or 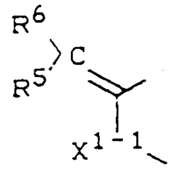

where

$X^{1-1}$, $R^3$, $R^4$, $R^5$ and $R^6$ have the abovementioned meaning,

are reacted with amines of the formula (III)

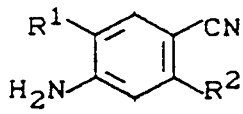 (III)

in which

$R^1$ and $R^2$ have the abovementioned meaning,

if appropriate in the presence of a diluent and if appropriate in the presence of a reaction auxiliary; or in that

(b) N-aryl nitrogen heterocycles of the formula (Ib)

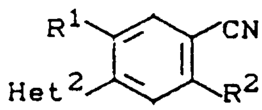 (Ib)

in which

$Het^2$ represents a heterocycle of the formula

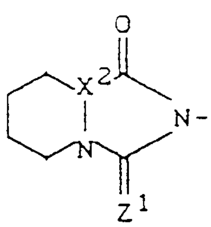 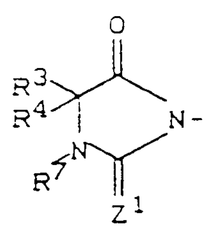 or 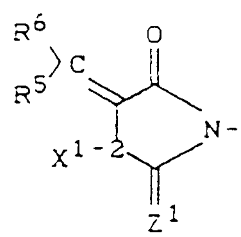 ,

where

$X^{1-2}$ represents an

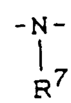

group and

$R^1$, $R^2$, $R^3$, $R^4$, $R^5$, $R^6$, $R^7$, $X^2$ and $Z^1$ have the abovementioned meaning,

are obtained when carboxylic acid esters of the formula (IV)

$R^9$-$COOR^{10}$ (IV)

in which

$R^9$ represents a radical of the formula

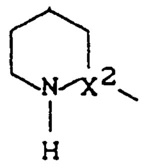 ; 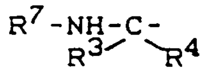 or 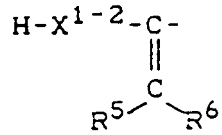 ,

and

$R^{10}$ represents alkyl, where

$X^{1-2}$, $X^2$, $R^3$, $R^4$, $R^5$, $R^6$ and $R^7$ have the abovementioned meaning,

or the acid addition salts thereof are reacted with iso(thio)cyanates of the formula (V)

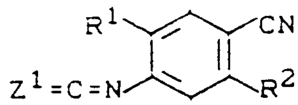

(V)

in which

$R^1$, $R^2$ and $Z^1$ have the abovementioned meaning,

if appropriate in the presence of a diluent and if appropriate in the presence of a reaction auxiliary; or in that

(c) N-aryl nitrogen heterocycles of the formula (Ic)

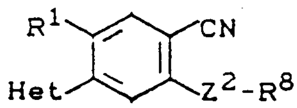

(Ic)

in which

$R^1$, $R^8$, $Z^2$ and Het have the abovementioned meaning,

are obtained when the N-aryl nitrogen heterocycles, which are obtainable by processes (a) and (b), of the formula (If)

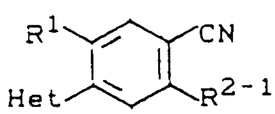

(If)

in which

$R^{2-1}$ represents halogen and

Het and $R^1$ have the abovementioned meaning,

are reacted with alcohols or thiols of the formula (VI)

$R^8$-$Z^2$H (VI)

in which

$R^8$ and $Z^2$ have the abovementioned meaning,

if appropriate in the presence of a diluent and in the presence of a reaction auxiliary; or in that

(d) N-aryl nitrogen heterocycles of the formula (Id)

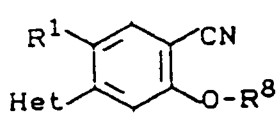

(Id)

in which

$R^1$, $R^8$ and Het have the abovementioned meaning,

are obtained when N-aryl nitrogen heterocycles of the formula (Ie)

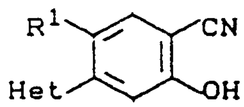

(Ie)

in which

$R^1$ and Het have the abovementioned meaning,

are reacted with alkylating agents of the formula (VII)

$R^8$-E (VII)

in which

E represents an electron-withdrawing leaving group and

$R^8$ has the abovementioned meaning,

if appropriate in the presence of a diluent and if appropriate in the presence of a reaction auxiliary; or in that

e) N-aryl nitrogen heterocycles of the formula (Ie)

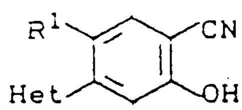

(Ie)

in which

$R^1$ and Het have the abovementioned meaning,

are obtained when

e-α) N-aryl nitrogen heterocycles of the formula (Ig)

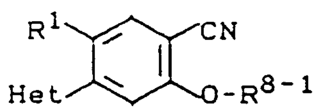

(Ig)

in which

$R^{8-1}$ represents allyl or benzyl and

$R^1$ and Het have the abovementioned meaning,

are reacted with reducing agents, if appropriate in the presence of a catalyst and if appropriate in the presence of a diluent, or in that

e-β) amines of the formula (IIIa)

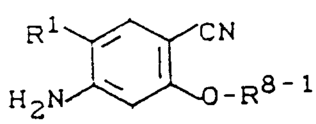

(IIIa)

in which

$R^1$ and $R^{8-1}$ have the abovementioned meaning,

are reacted with reducing agents, if appropriate in the presence of a catalyst and if appropriate in the presence of a diluent and if appropriate in the presence of a reaction auxiliary, and the resulting aminophenols of the formula (VIII)

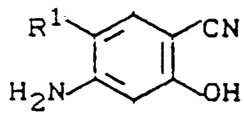

(VIII)

in which

$R^1$ has the abovementioned meaning,

are reacted with anhydrides of the formula (II)

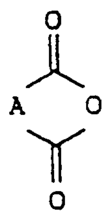

(II)

in which

A represents a radical of the formula

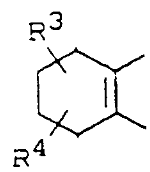 ; 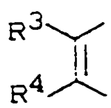 or 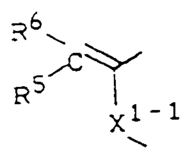

where

$X^{1-1}$ represents a $-CH_2-$ group or represents a group and

$R^3$, $R^4$, $R^5$ and $R^6$ have the abovementioned meaning,

if appropriate in the presence of a diluent and if appropriate in the presence of a reaction auxiliary.

5. Herbicidal agents, characterized in that they contain at least one N-aryl nitrogen heterocycle of the formula (I) according to Claims 1 to 4.

6. Method of combating weeds, characterized in that N-aryl nitrogen heterocycles of the formula (I) according to Claims 1 to 4 are allowed to act on the weeds and/or their environment.

7. Use of N-aryl nitrogen heterocycles of the formula (I) according to Claims 1 to 4 for combating weeds.

8. Process for the preparation of herbicidal agents, characterized in that N-aryl nitrogen heterocycles of the formula (I) according to Claims 1 to 4 are mixed with extenders and/or surface-active substances.

**9.** The compounds of the formula:

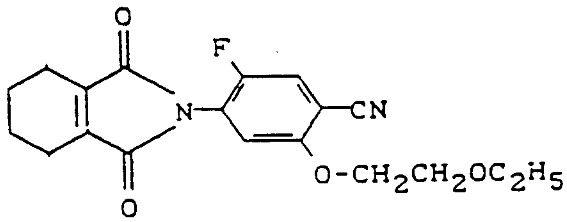

,

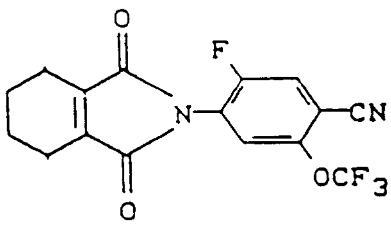

,

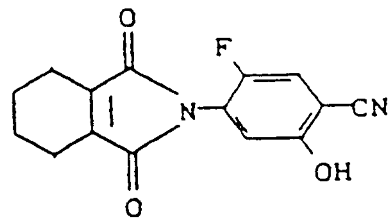

,

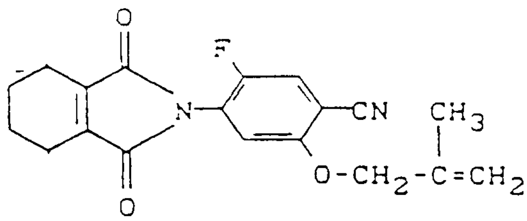

,

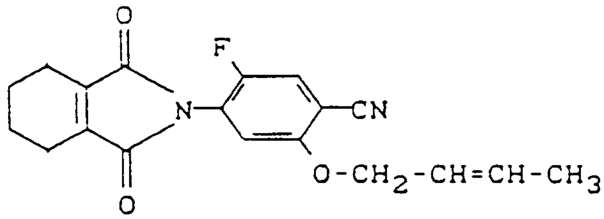

,

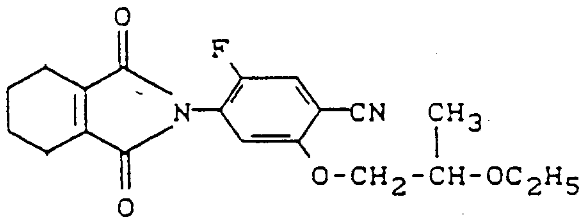

,

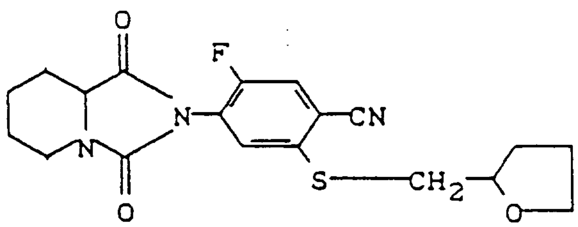

,

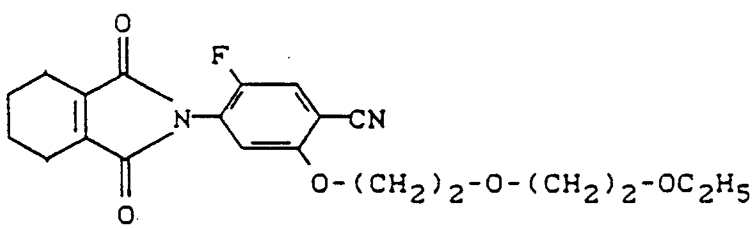

and

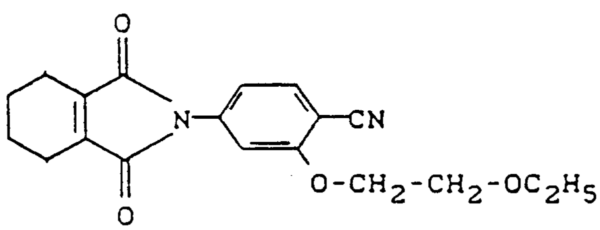

.

## Revendications

**1.** Composés hétérocycliques azotés N-arylés de formule (I)

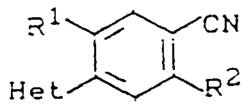

(I)

dans laquelle

Het représente un radical hétérocyclique de formules

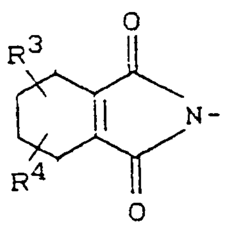

;

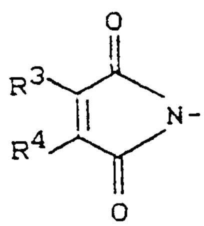

;

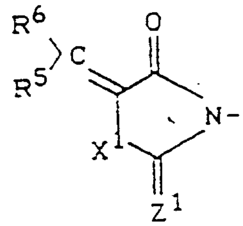

ou

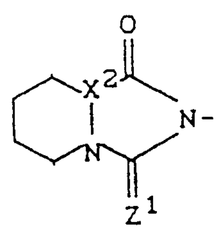

$R^1$ représente un atome d'hydrogène ou un atome d'halogène, et

$R^2$ représente un atome d'halogène, un groupe hydroxyle ou représente un radical $-Z^2-R^8$, et

$X^1$ représente un groupe $-CH_2-$, un groupe

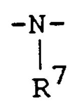

ou encore un groupe

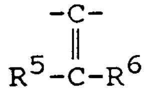

$X^2$ représente un atome d'azote ou un groupe CH,

$Z^1$ représente un atome d'oxygène ou un atome de soufre,

$Z^2$ représente un atome d'oxygène ou un atome de soufre,

$R^3$ et $R^4$ représentent chacun, indépendamment l'un de l'autre, un atome d'hydrogène ou un groupe alkyle,

$R^5$ et $R^6$ représentent, soit chacun indépendamment l'un de l'autre, un atome d'hydrogène ou un groupe alkyle, soit ensemble, un radical alcanediyle deux fois lié,

$R^7$ représente un atome d'hydrogène, un groupe alkyle; ou un groupe aryle éventuellement substitué, et

$R^8$ représente un groupe alkyle, un groupe alcényle, un groupe alcynyle ou un groupe cycloalkyle, chacun de ces groupes étant éventuellement substitué,

à l'exception des composés dans lesquels

Het représente un radical hétérocyclique de formules

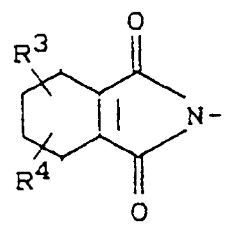

ou

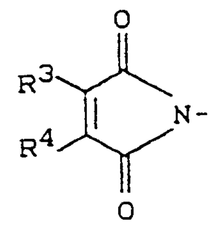

$R^1$ représente un atome d'hydrogène ou un atome d'halogène,

$R^2$ représente un radical $-Z^2-R^8$ où $Z^2$ représente un atome d'oxygène, et

$R^8$ représente un atome d'hydrogène ou représente un groupe alkyle, un groupe alcényle, un groupe alcynyle ou un groupe cycloalkyle, chacun de ces groupes étant éventuellement substitué, et

$R^3$ et $R^4$ représentent chacun, indépendamment l'un de l'autre, un atome d'hydrogène ou un groupe alkyle,

les composés ci-après étant exclus de la renonciation :

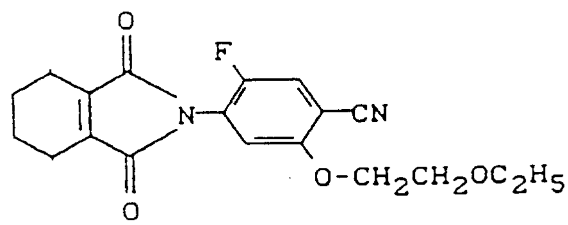

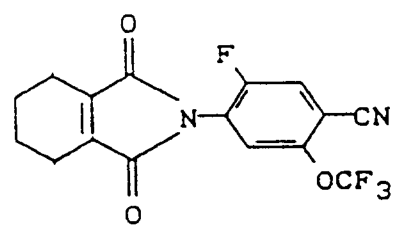

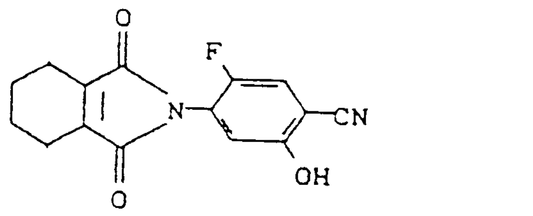

,

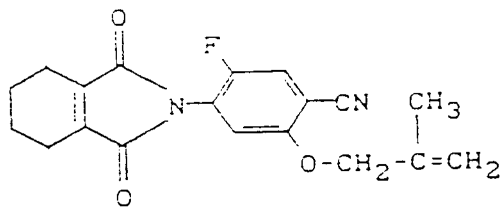

,

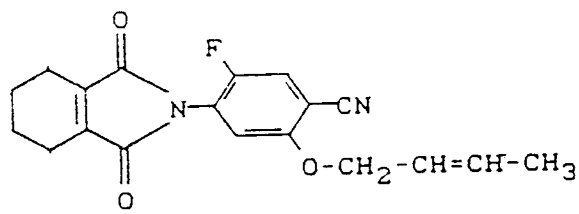

,

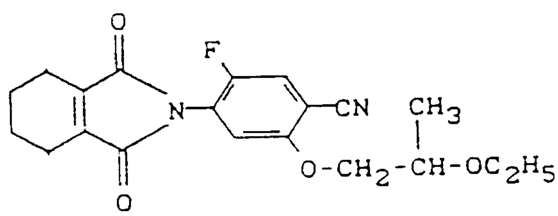

,

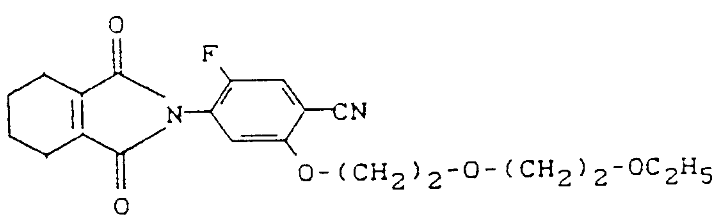

et

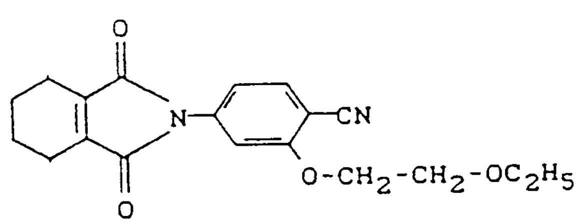

.

**2.** Composés hétérocycliques azotés N-arylés de formule (I) selon la revendication 1, dans lesquels

Het représente un radical hétérocyclique de formules

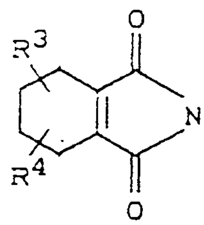 ; 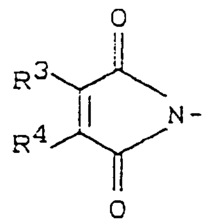 ; 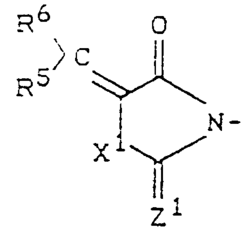

ou 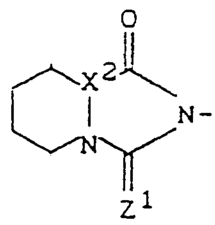

$R^1$ représente un atome d'hydrogène, un atome de fluor, un atome de chlore ou un atome de brome, et

$R^2$ représente un atome de fluor, un atome de chlore, un atome de brome, un groupe hydroxyle ou encore un radical $-Z^2-R^8$, et

$X^1$ représente un groupe $-CH_2-$, un groupe

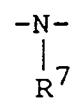

ou encore un groupe

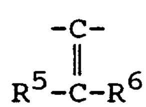

$X^2$ représente un atome d'azote ou un groupe CH,

$Z^1$ représente un atome d'oxygène ou un atome de soufre,

$Z^2$ représente un atome d'oxygène ou un atome de soufre,

$R^3$ et $R^4$ représentent chacun, indépendamment l'un de l'autre, un atome d'hydrogène ou un groupe alkyle à chaîne droite ou ramifiée contenant de 1 à 4 atomes de carbone,

$R^5$ et $R^6$ représentent, soit chacun indépendamment l'un de l'autre, un atome d'hydrogène ou un groupe alkyle à chaîne droite ou ramifiée contenant de 1 à 4 atomes de carbone, soit ensemble, un radical alcanediyle deux fois lié contenant de 2 à 7 atomes de carbone,

$R^7$ représente un atome d'hydrogène; un groupe alkyle à chaîne droite ou ramifiée contenant de 1 à 4 atomes de carbone: ou encore un groupe phényle éventuellement substitué une ou plusieurs fois de manière identique ou différente, dans lequel, comme substituants, on envisage : un atome d'halogène, un groupe cyano, un groupe nitro; un groupe alkyle, un groupe alcoxy ou un groupe alkylthio contenant chacun de 1 à 4 atomes de carbone, chacun de ces groupes étant à chaîne droite ou ramifiée; ainsi qu'un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée, et

$R^8$ représente un groupe alkyle contenant de 1 à 8 atomes de carbone, un groupe alcényle contenant de 2 à 8 atomes de carbone, un groupe alcynyle contenant de 3 à 8 atomes de carbone, un groupe halogénalkyle contenant de 1 à 8 atomes de carbone et de 1 à 17 atomes d'halogène identiques ou différents, un groupe halogénalcényle

contenant de 2 à 8 atomes de carbone et de 1 à 15 atomes d'halogène identiques ou différents, un groupe halogénalcynyle contenant de 3 à 8 atomes de carbone et de 1 à 13 atomes d'halogène identiques ou différents, un groupe cyanalkyle un groupe alcoxyalkyle, un groupe alkylthioalkyle, un groupe halogénalcoxyalkyle, un groupe alcoxyalcoxyalkyle, un groupe bis-(alcoxy)alkyle, bis-(alkylthio)alkyle, un groupe alkylcarbonylalkyle, un groupe alcoxycarbonylalkyle ou un groupe alcoxyalcoxycarbonylalkyle contenant chacun de 1 à 8 atomes de carbone dans les fractions alkyle individuelles et éventuellement de 1 à 9 atomes d'halogène identiques ou différents, chacun de ces groupes étant à chaîne droite ou ramifiée; ou encore un groupe cycloalkyle, un groupe cycloalcoxycarbonylalkyle ou un groupe cycloalkylalkyle contenant chacun de 3 à 7 atomes de carbone dans la fraction cycloalkyle et éventuellement de 1 à 4 atomes de carbone dans la fraction alkyle à chaîne droite ou ramifiée, chacun de ces groupes étant éventuellement substitué une ou plusieurs fois de manière identique ou différente, dans lesquels, comme substituants dans la fraction cycloalkyle, on envisage respectivement : un atome d'halogène; ainsi qu'un groupe alkyle ou un groupe alcoxy à chaîne droite ou ramifiée contenant chacun de 1 à 4 atomes de carbone; $R^8$ représente, en outre, un groupe oxétanylalkyle, un groupe tétrahydrofuranylalkyle ou un groupe tétrahydropyranylalkyle contenant chacun de 1 à 3 atomes de carbone dans les fractions alkyle individuelles, chacun de ces groupes étant éventuellement substitué par un groupe alkyle contenant de 1 à 4 atomes de carbone; et représente un groupe aralkyle contenant de 6 à 10 atomes de carbone dans la fraction aryle et de 1 à 4 atomes de carbone dans la fraction alkyle à chaîne droite ou ramifiée, éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio ou un groupe alcoxycarbonyle contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée,

à l'exception des composés dans lesquels

Het représente un radical hétérocyclique de formules

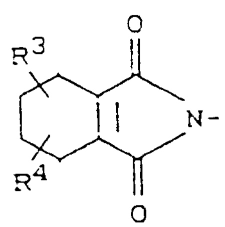

ou

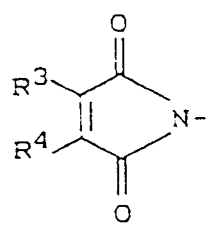

$R^1$ représente un atome d'hydrogène ou un atome d'halogène,

$R^2$ représente un radical $-Z^2-R^8$ où $Z^2$ représente un atome d'oxygène, et

$R^8$ représente un atome d'hydrogène ou représente un groupe alkyle, un groupe alcényle, un groupe alcynyle ou un groupe cycloalkyle, chacun de ces groupes étant éventuellement substitué, et

$R^3$ et $R^4$ représentent chacun, indépendamment l'un de l'autre, un atome d'hydrogène ou un groupe alkyle,

les composés ci-après étant exclus de la renonciation :

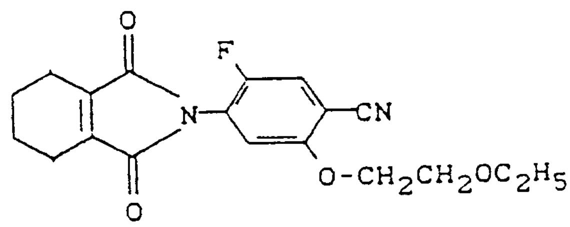

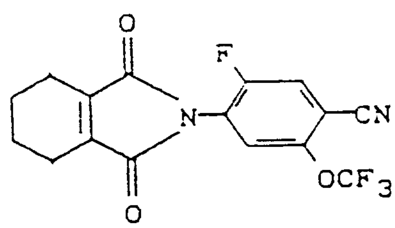

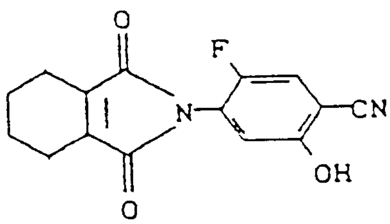

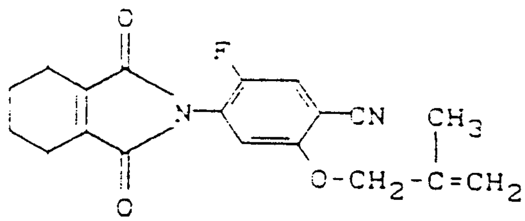

,

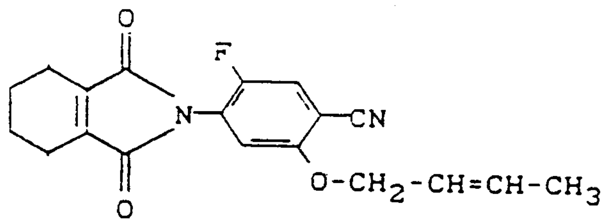

,

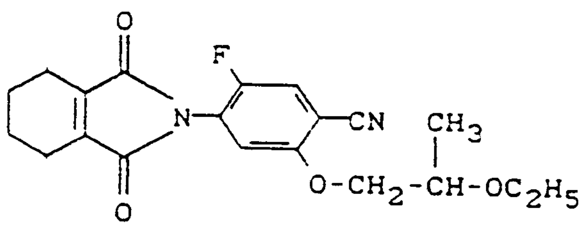

,

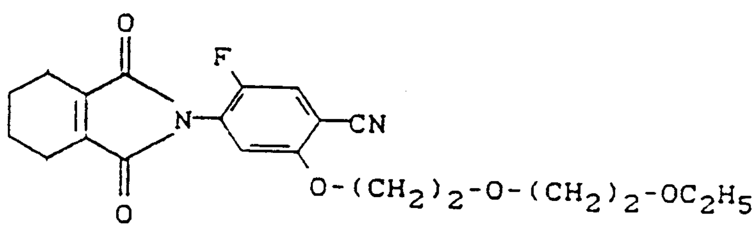

et

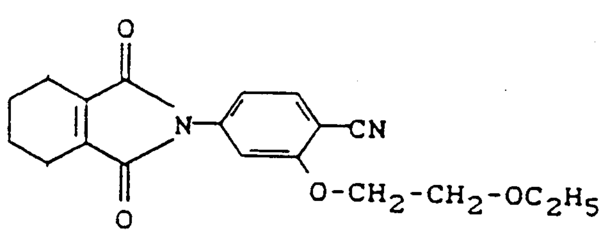

.

**3.** Composés hétérocycliques azotés N-arylés de formule (I) selon la revendication 1, dans lesquels
Het représente un radical hétérocyclique de formules

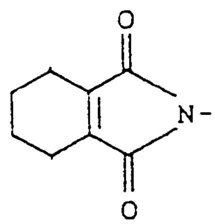 ; 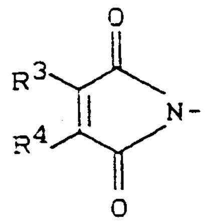 ; 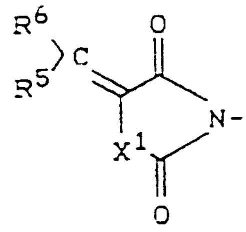

ou 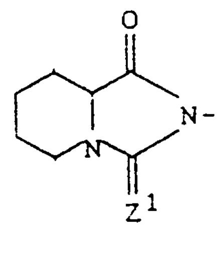

$R^1$ représente un atome d'hydrogène, un atome de fluor ou un atome de chlore, et

$R^2$ représente un atome de fluor, un atome de chlore ou représente un radical $-Z^2-R^8$,

$X^1$ représente un groupe $-CH_2-$, un groupe

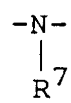

ou encore un groupe

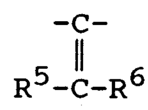

$Z^1$ représente un atome d'oxygène ou un atome de soufre,

$Z^2$ représente un atome d'oxygène ou un atome de soufre,

$R^3$ et $R^4$ représentent chacun, indépendamment l'un de l'autre, un atome d'hydrogène, un groupe méthyle, un groupe éthyle, un groupe n- ou i-propyle,

$R^5$ et $R^6$ représentent, soit chacun indépendamment l'un de l'autre, un atome d'hydrogène, un groupe méthyle, un groupe éthyle, un groupe n- ou i-propyle, soit ensemble, un radical alcanediyle deux fois lié contenant de 2 à 5 atomes de carbone,

$R^7$ représente un atome d'hydrogène, un groupe méthyle, un groupe éthyle, un groupe n- ou i-propyle; ou encore un groupe phényle éventuellement substitué de une à trois fois de manière identique ou différente, dans lequel, comme substituants, on envisage : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy ou un groupe trifluorométhylthio, et

$R^8$ représente un groupe méthyle, un groupe éthyle, un groupe n- ou i-propyle, un groupe n-, i-, s- ou t-butyle: un groupe allyle, un groupe propargyle; un groupe pentyle, un groupe hexyle, un groupe butényle, un groupe pentényle, un groupe hexényle, un groupe butynyle, un groupe pentynyle ou un groupe hexynyle, chacun de ces groupes étant à chaîne droite ou ramifiée: représente, en outre, un groupe halogénalkyle contenant de 1 à 4 atomes de carbone ou un groupe halogénalcényle contenant de 3 à 5 atomes de carbone, chacun de ces groupes contenant de 1 à 8 atomes d'halogène identiques ou différents et étant chacun à chaîne droite ou ramifiée; un groupe cyanalkyle, un groupe alcoxyalkyle, un groupe alkylthioalkyle, un groupe halogénalcoxyalkyle, un groupe alcoxyalcoxyalkyle, un groupe alkylcarbonylalkyle, un groupe alcoxycarbonylalkyle ou un groupe alcoxyalcoxycarbonylalkyle contenant chacun de 1 à 5 atomes de carbone dans les fractions alkyle individuelles, chacun de ces groupes étant à chaîne droite ou ramifiée: en outre, représente un groupe cyclopropylméthyle, un groupe cyclopentylméthyle, un groupe cyclohexylméthyle, un groupe cyclopropyloxycarbonylméthyle, un groupe cyclopentyloxycarbonylméthyle, un groupe cyclohexyloxycarbonylméthyle, un groupe cyclopropyle, un groupe cyclopentyle ou un groupe cyclohexyle, chacun de ces groupes étant éventuellement substitué de une à trois fois de manière identique ou différente par un groupe méthyle, un groupe méthoxy, par un atome de fluor et par un atome de chlore; représente un groupe oxétanylméthyle, un groupe oxétanyléthyle, un groupe tétrahydrofuranylméthyle, un groupe tétrahydrofuranyléthyle, un groupe tétrahydropyranylméthyle ou un groupe tétrahydropyranyléthyle, chacun de ces groupes étant éventuellement substitué par un groupe méthyle ou par un groupe éthyle; ou représente un groupe benzyle ou un groupe phényléthyle, chacun étant éventuellement substitué de une à trois fois de manière identique ou différente, dans lesquels, comme substituants, on envisage respectivement : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe éthylthio, un groupe méthoxycarbonyle, un groupe éthoxycarbonyle, un groupe trifluorométhyle, un groupe trifluorométhoxy ou un groupe trifluorométhylthio,

à l'exception des composés dans lesquels

Het représente un radical hétérocyclique de formules

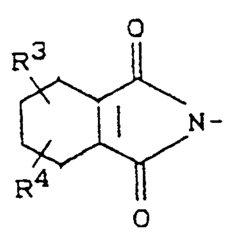

ou

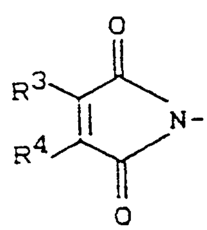

$R^1$ représente un atome d'hydrogène ou un atome d'halogène,

$R^2$ représente un radical $-Z^2-R^8$ où $Z^2$ représente un atome d'oxygène, et

$R^8$ représente un atome d'hydrogène ou représente un groupe alkyle, un groupe alcényle, un groupe alcynyle ou un groupe cycloalkyle, chacun de ces groupes étant éventuellement substitué, et

$R^3$ et $R^4$ représentent chacun, indépendamment l'un de l'autre, un atome d'hydrogène ou un groupe alkyle,

les composés ci-après étant exclus de la renonciation :

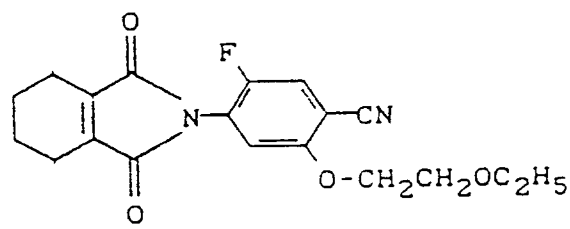

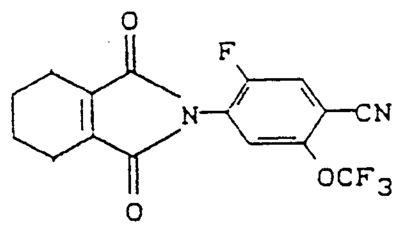

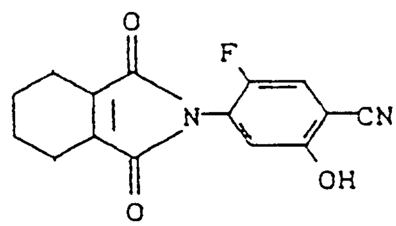

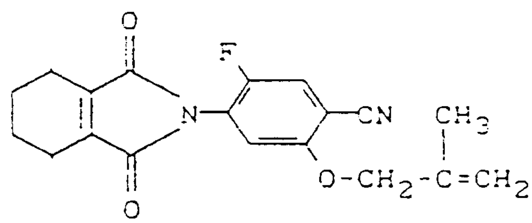

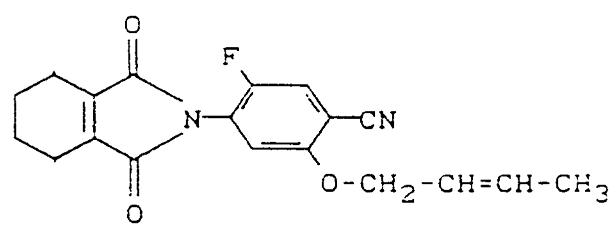

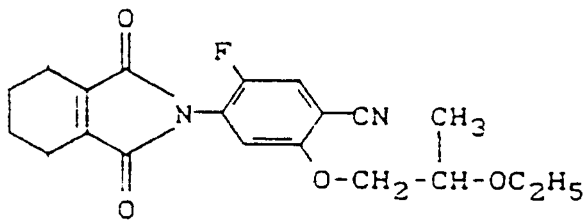 ,

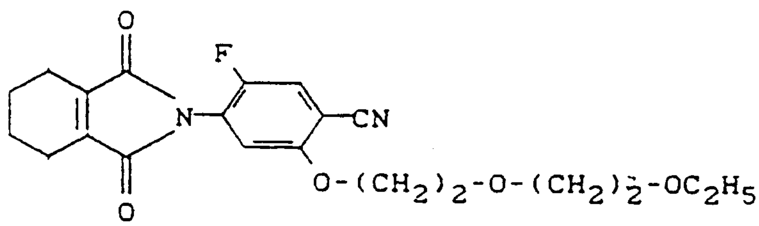

et

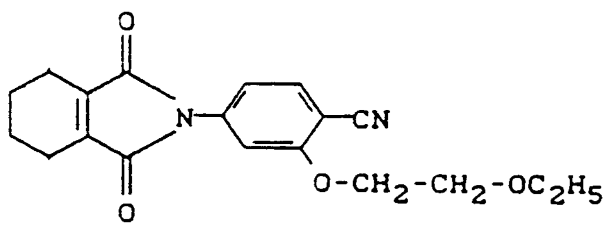 .

**4.** Procédé pour la préparation de composés hétérocycliques azotés N-arylés de formule (I)

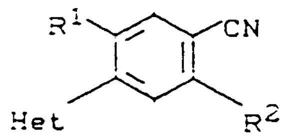 (I)

dans laquelle

Het représente un radical hétérocyclique de formules

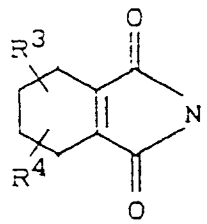 ; 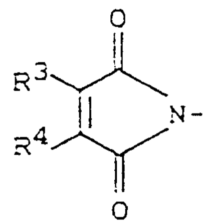 ; 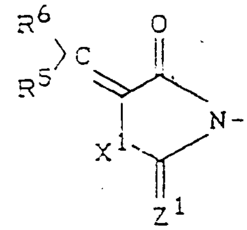

ou 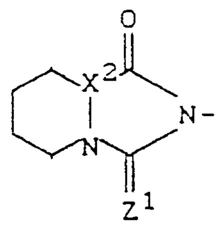

$R^1$ représente un atome d'hydrogène ou un atome d'halogène, et

$R^2$ représente un atome d'halogène, un groupe hydroxyle ou représente un radical $-Z^2-R^8$, et

$X^1$ représente un groupe $-CH_2-$, un groupe

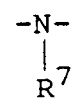

ou encore un groupe

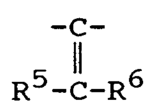

$X^2$ représente un atome d'azote ou un groupe CH,

$Z^1$ représente un atome d'oxygène ou un atome de soufre,

$Z^2$ représente un atome d'oxygène ou un atome de soufre,

$R^3$ et $R^4$ représentent chacun, indépendamment l'un de l'autre, un atome d'hydrogène ou un groupe alkyle,

$R^5$ et $R^6$ représentent, soit chacun indépendamment l'un de l'autre, un atome d'hydrogène ou un groupe alkyle, soit ensemble, un radical alcanediyle deux fois lié,

$R^7$ représente un atome d'hydrogène, un groupe alkyle; ou un groupe aryle éventuellement substitué, et

$R^8$ représente un groupe alkyle, un groupe alcényle, un groupe alcynyle ou un groupe cycloalkyle, chacun de ces groupes étant éventuellement substitué,

à l'exception des composés dans lesquels

Het représente un radical hétérocyclique de formules

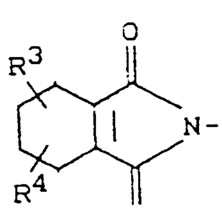 ou 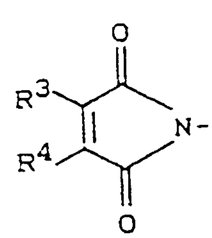

$R^1$ représente un atome d'hydrogène ou un atome d'halogène,

$R^2$ représente un radical $-Z^2-R^8$ où $Z^2$ représente un atome d'oxygène, et

$R^8$ représente un atome d'hydrogène ou représente un groupe alkyle, un groupe alcényle, un groupe alcynyle ou un groupe cycloalkyle, chacun de ces groupes étant éventuellement substitué, et

$R^3$ et $R^4$ représentent chacun un groupe alkyle,

les composés ci-après étant exclus de la renonciation :

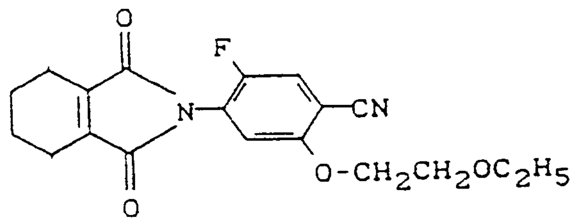

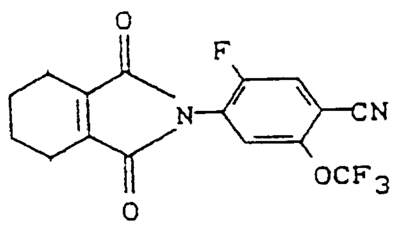

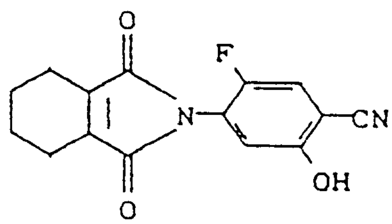

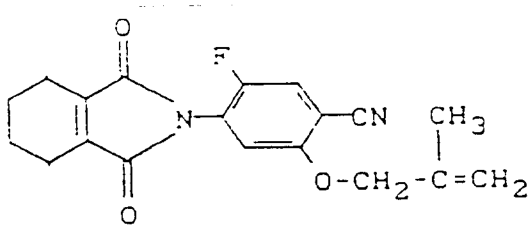

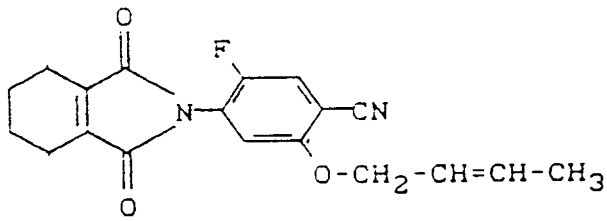 ,

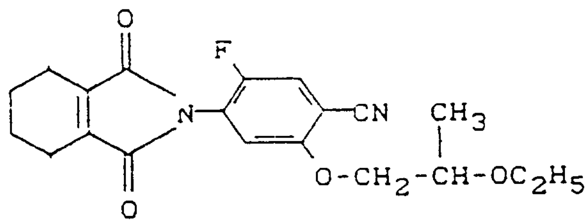 ,

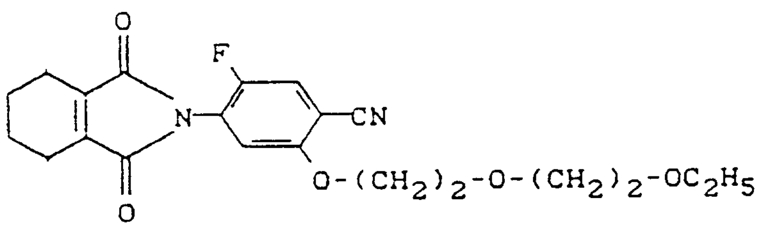

et

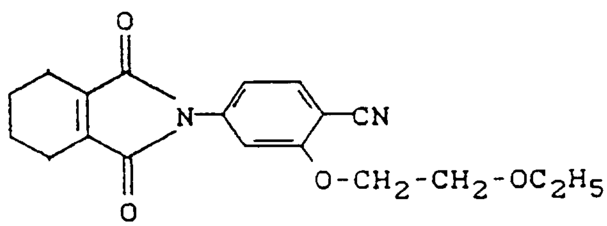 ,

caractérisé en ce qu'on obtient

(a) des composés hétérocycliques azotés N-arylés de formule (Ia)

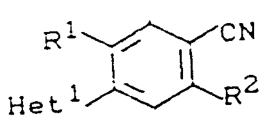 (Ia)

dans laquelle

Het[1] représente un radical hétérocyclique de formules

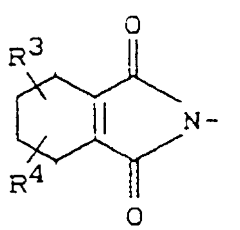 ; 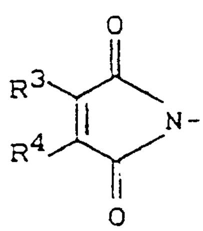 ; 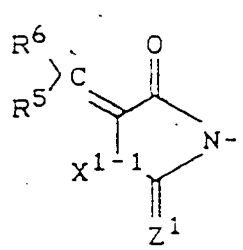 ;

dans lesquelles

$X^{1-1}$ représente un groupe $-CH_2-$ ou un groupe

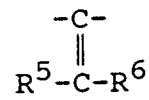

$R^1$, $R^2$, $R^3$, $R^4$, $R^5$ et $R^6$ ont la signification indiquée ci-dessus,

lorsqu'on fait réagir des anhydrides de formule (II)

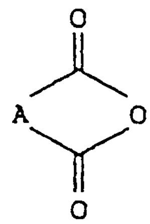

(II)

dans laquelle

A représente un radical de formules

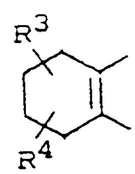

;

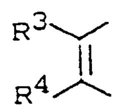

ou

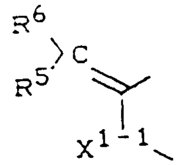

dans lesquelles

$X^{1-1}$, $R^3$, $R^4$, $R^5$ et $R^6$ ont la signification indiquée ci-dessus,

avec des amines de formule (III)

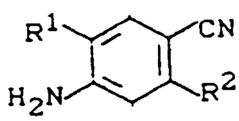

(III)

dans laquelle

$R^4$ et $R^2$ ont la signification indiquée ci-dessus,

éventuellement en présence d'un diluant et éventuellement en présence d'un adjuvant réactionnel; ou bien en ce qu'on obtient

(b) des composés hétérocycliques azotés N-arylés de formule (Ib)

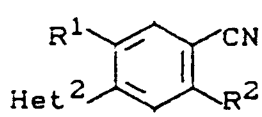

(Ib)

dans laquelle

$Het^2$ représente un radical hétérocyclique de formules

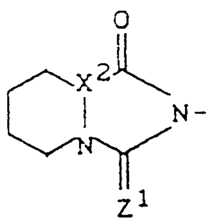 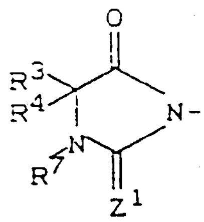 ou 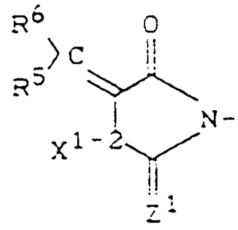

dans lesquelles

$X^{1-2}$ représente un groupe

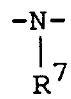

et

$R^1$, $R^2$, $R^3$, $R^4$, $R^5$, $R^6$, $R^7$, $X^2$ et $Z^1$ ont la signification indiquée ci-dessus, lorsqu'on fait réagir des esters d'acides carboxyliques de formule (IV)

$R^9$-COOR10 (IV)

dans laquelle

$R^9$ représente un radical de formules

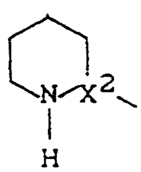 ; 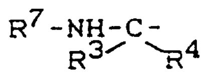 ou 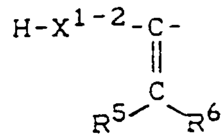

et

$R^{10}$ représente un groupe alkyle,

$X^{1-2}$, $X^2$, $R^3$, $R^4$, $R^5$, $R^6$ et $R^7$ ayant la signification indiquée ci-dessus,

ou leurs sels d'addition d'acides avec des iso(thio)cyanates de formule (V)

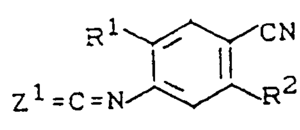 (V)

dans laquelle

$R^1$, $R^2$ et $Z^1$ ont la signification indiquée ci-dessus,

éventuellement en présence d'un diluant et éventuellement en présence d'un adjuvant réactionnel; ou bien en ce qu'on obtient

(c) des composés hétérocycliques azotés N-arylés de formule (Ic)

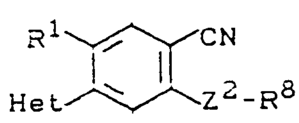 (Ic)

dans laquelle

$R^1$, $R^8$, $Z^2$ et Het ont la signification indiquée ci-dessus,

lorsqu'on fait réagir les composés hétérocycliques azotés N-arylés que l'on obtient conformément aux procédés (a) et (b), répondant à la formule (If)

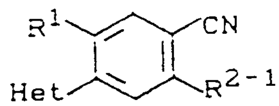

(If)

dans laquelle

$R^{2-1}$ représente un atome d'halogène, et

Het et $R^1$ ont la signification indiquée ci-dessus,

avec des alcools ou des thiols de formule (VI),

$R^8$-$Z^2$H (VI)

dans laquelle

$R^8$ et $Z^2$ ont la signification indiquée ci-dessus,

éventuellement en présence d'un diluant, ainsi qu'en présence d'un adjuvant réactionnel; ou bien en ce qu'on obtient

(d) des composés hétérocycliques azotés N-arylés de formule (Id)

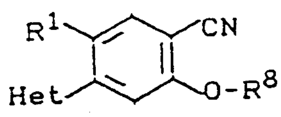

(Id)

dans laquelle

$R^1$, $R^8$ et Het ont la signification indiquée ci-dessus,

lorsqu'on fait réagir des composés hétérocycliques azotés N-arylés de formule (Ie)

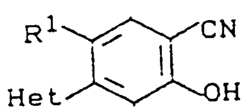

(Ie)

dans laquelle

$R^1$ et Het ont la signification indiquée ci-dessus, avec des agents d'alkylation de formule (VII)

$R^8$-E (VII)

dans laquelle

E représente un groupe qui se sépare, attirant les électrons, et

$R^8$ a la signification indiquée ci-dessus,

éventuellement en présence d'un diluant et éventuellement en présence d'un adjuvant réactionnel; ou bien en ce qu'on obtient

(e) des composés hétérocycliques azotés N-arylés de formule (Ie)

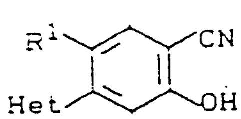

(Ie)

dans laquelle

$R^1$ et Het ont la signification indiquée ci-dessus, lorsqu'on fait réagir
e-α) des composés hétérocycliques azotés N-arylés de formule (Ig)

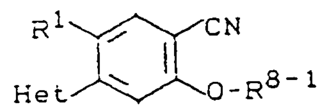

(Ig)

dans laquelle
$R^{8-1}$ représente un groupe allyle ou un groupe benzyle, et
$R^1$ et Het ont la signification indiquée ci-dessus,
avec des agents de réduction, éventuellement en présence d'un catalyseur et éventuellement en présence d'un diluant, ou bien en ce qu'on fait réagir
e-β) des amines de formule (IIIa)

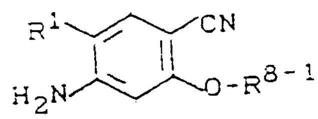

(IIIa)

dans laquelle
$R^1$ et $R^{8-1}$ ont la signification indiquée ci-dessus,
avec des agents de réduction, éventuellement en présence d'un catalyseur et éventuellement en présence d'un diluant et éventuellement en présence d'un adjuvant réactionnel, et on fait réagir les aminophénols ainsi obtenus répondant à la formule (VIII)

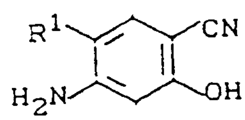

(VIII)

dans laquelle
$R^1$ a la signification indiquée ci-dessus,
avec des anhydrides de formule (II)

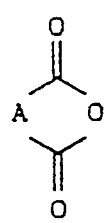

(II)

dans laquelle
A représente un radical de formules

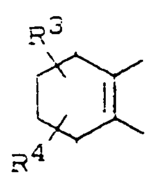

;

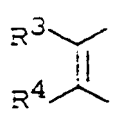

ou

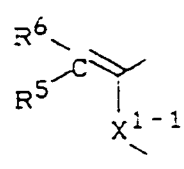

dans lesquelles

$X^{1-1}$ représente un groupe $CH_2$ ou un groupe

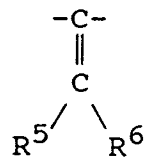

et

$R^3$, $R^4$, $R^5$ et $R^6$ ont la signification indiquée ci-dessus,

éventuellement en présence d'un diluant et éventuellement en présence d'un adjuvant réactionnel.

5. Agents herbicides caractérisés par une teneur en au moins un composé hétérocyclique azoté N-arylé de formule (I) selon les revendications 1 à 4.

6. Procédé pour lutter contre des mauvaises herbes, caractérisé en ce qu'on laisse agir des composés hétérocycliques azotés N-arylés de formule (I) selon les revendications 1 à 4, sur les mauvaises herbes et/ou sur leur biotope.

7. Utilisation de composés hétérocycliques azotés N-arylés de formule (I) selon les revendications 1 à 4, pour lutter contre les mauvaises herbes.

8. Procédé pour la préparation d'agents herbicides, caractérisé en ce qu'on mélange des composés hétérocycliques azotés N-arylés de formule (I) selon les revendications 1 à 4, avec des diluants et/ou avec des substances tensioactives.

9. Les composés répondant aux formules :

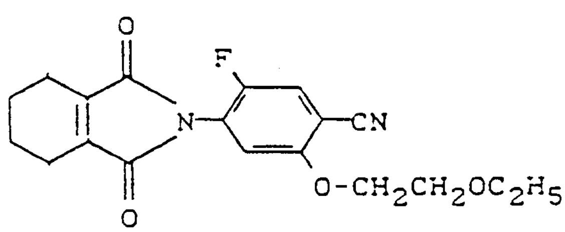

,

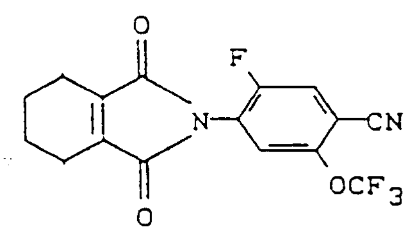

,

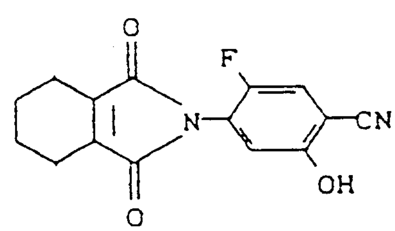

,

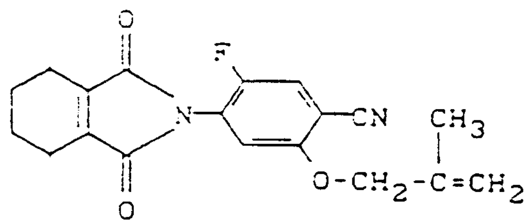

,

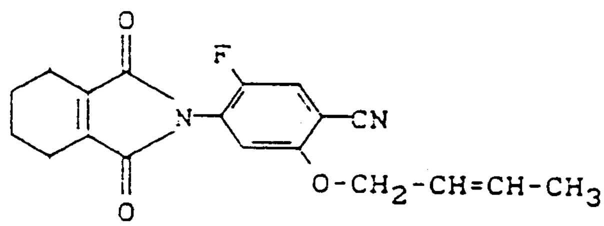

,

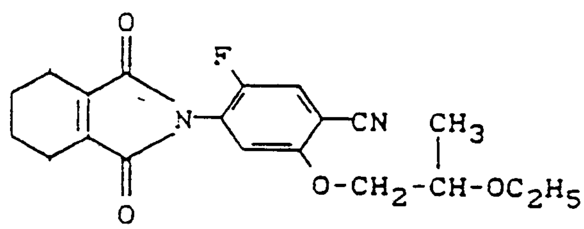

,

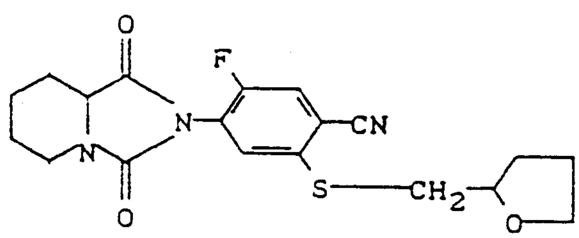

,

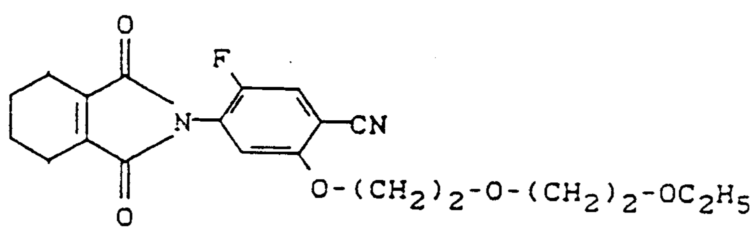

et

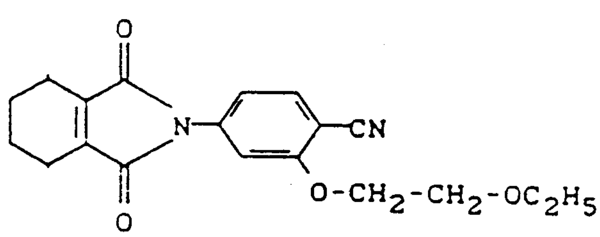

.